# EUROPEAN PATENT APPLICATION

(11) **EP 4 796 181 A1**
(43) Date of publication of application: **26.08.2026**
(21) Application number: 25382158.1
(22) Date of filing: 20.02.2025
(51) Int. Cl.: A61P 35/00, C07K 16/28

(54) **NEUTRALIZING ANTIBODIES AGAINST GLYCOPROTEIN 130, METHODS AND USES THEREOF**

(71) Applicant: Fundació Privada Institut d'Investigació Oncològica de Vall Hebron, 08035 Barcelona (ES); Institució Catalana de Recerca i Estudis Avançats, 08010 Barcelona (ES)
(72) Inventor: SEOANE SUAREZ, Joan, E-08035 Barcelona (ES); ESPINOSA GIL, Sergio, E-08035 Barcelona (ES); GALERA MARTINEZ, Cayetano Jesús, E-08035 Barcelona (ES); IURLARO, Raffaella, E-08035 Barcelona (ES)
(74) Representative: ABG Intellectual Property Law, S.L.

(57) **Abstract**

The present invention relates to neutralizing antibodies, antigen binding-fragment thereof or antibody constructs comprising an antigen-binding fragment thereof that specifically bind to glycoprotein 130, as well as to conjugates comprising a therapeutic agent or a detectable label and said neutralizing antibodies. The invention also relates to a method for detecting glycoprotein 130, to pharmaceutical compositions comprising said antibodies and to uses of said antibodies or conjugates in the treatment of cancer and/or inflammatory diseases. The invention also relates to combination therapies comprising a neutralizing antibody specific against glycoprotein 130 and to methods comprising said combination therapies.

## Description

### FIELD OF THE INVENTION

The present invention is comprised within the field of biotechnology and biomedicine. It specifically relates to neutralizing antibodies specific against glycoprotein 130, as well as to antibody conjugates, methods, compositions comprising said antibodies and to uses of said antibodies in the treatment of cancer and inflammatory diseases. The present invention also relates to combination therapies comprising a neutralizing antibody specific against glycoprotein 130.

### BACKGROUND OF THE INVENTION

Cancer is one of the leading causes of morbidity and mortality worldwide. It is responsible for almost one in six deaths globally and the number of new cases is expected to rise by about 70% over the next 2 decades.

Many therapies are now available to be used in the treatment of cancer. During the last 20 years, immunotherapies have revolutionized the treatment of some tumor types. However, many tumors do not respond to this type of therapies. Among immunotherapies, adoptive T cell therapies (ACTs), such as CAR-T cells and T-cell engagers (TCEs); immune checkpoint inhibitors (ICls) and antibody-drug conjugates (ADCs) have shown impressive responses in the context of hematologic tumors. However, solid tumors are still recalcitrant to these immunotherapies. This discrepancy is mainly due to the tumor microenvironment (TME).

Work over many years has shown that the TME is crucial for tumors to initiate and grow. The TME is composed by a diverse set of cell types, including immune cells, fibroblasts, and endothelial cells. Within the TME, the myeloid cells and specifically the tumor associated macrophages (TAMs) have been shown to play a fundamental role in anti-tumor immunity leading to immunosuppression and enabling tumor immune evasion. This is crucial in the context of resistance to immunotherapies such as immune checkpoint inhibitors, T cell engagers, CAR-T cells and antibody-drug conjugates.

Accordingly, given the lack of success of immunotherapies in the treatment of many cancers, especially in the treatment of solid tumors, there is an urgent need to identify and develop novel therapies to treat cancer patients. Provided herein are embodiments that meet such needs.

### SUMMARY OF THE INVENTION

The authors of the present invention have identified glycoprotein 130 (gp130) as a factor that promotes the oncogenic and immunosuppressive features of TAMs, and have developed neutralizing antibodies targeting gp130 which are capable of reversing the TAM phenotype thereby generating a strong anti-tumor response, optionally in combination with immunotherapies in solid tumors. This novel neutralizing antibody could offer a new cancer therapy for solid tumors characterized by the presence of TAMs with increased gp130 signaling, as well as a new therapy for inflammatory diseases characterized by increased gp130 signaling. Without wishing to be bound by any theory, the authors of the invention have found anti-gp130 specific antibodies which are capable of neutralizing the signaling mediated by different cytokines of the IL-6 family (OSM, IL-6, IL-11, CT- and LIF) but which is not capable of neutralizing the signaling mediated by IL-27, which is another member of the IL-6 family of cytokines.

Therefore, a first aspect of the present invention relates to a neutralizing antibody, an antigen-binding fragment thereof or an antibody construct comprising an antigen-binding fragment thereof capable of specifically binding to and neutralizing gp130, wherein said antibody, antigen-binding fragment thereof or antibody construct comprises:
(a) within the heavy chain of the variable region, a CDR comprising the amino acid sequence set forth in SEQ ID NO: 1 [CDR-H1], a CDR comprising the amino acid sequence set forth in SEQ ID NO: 2 [CDR-H2], and a CDR comprising the amino acid sequence set forth in SEQ ID NO: 3 [CDR-H3], or a functionally equivalent variant of said CDRs; and
(b) within the light chain of the variable region, a CDR comprising the amino acid sequence set forth in SEQ ID NO: 4 [CDR-L1], a CDR comprising the amino acid sequence set forth in SEQ ID NO: 5 [CDR-L2] (SAN), and a CDR comprising the amino acid sequence set forth in SEQ ID NO: 6 [CDR-L3], or a functionally equivalent variant of said CDRs.

In an alternative first aspect, the invention relates to an antibody which is capable of specifically binding to gp130 and of substantially neutralizing one or more of the cytokines of the IL-6 family but which is not capable of substantially neutralizing IL-27.

A second aspect of the present invention relates to a nucleic acid encoding the antibody, antigen-binding fragment thereof or antibody construct according to the first aspect of the invention.

A third aspect of the present invention relates to a gene construct, an expression cassette or a vector comprising the nucleic acid according to the second aspect of the invention.

A fourth aspect of the present invention relates to a cell comprising the nucleic acid according to the second aspect of the invention, or the gene construct, the expression cassette, or the vector according to the third aspect of the invention.

A fifth aspect of the present invention relates to a conjugate selected from the group consisting of:
(i) an antibody-drug conjugate (ADC) comprising a therapeutic agent and the antibody, antigen-binding fragment thereof or antibody construct according to the first aspect of the invention; and
(ii) a detectable label and the antibody, antigen-binding fragment thereof or antibody construct according to the first aspect of the invention.

A sixth aspect of the present invention relates to an *in vitro* method for detecting and/or quantifying the presence of gp130 in a sample, comprising:
(a) contacting the antibody, antigen-binding fragment thereof or antibody construct according to the first aspect of the invention, or the conjugate according to the fifth aspect of the invention (ii) with the sample; and
(b) detecting and/or quantifying the level of gp130 in the sample by measuring the formation of immune complexes formed between the gp130 in the sample and the antibody, antigen-binding fragment thereof, antibody construct, or the conjugate.

A seventh aspect of the present invention relates to a pharmaceutical composition comprising the antibody, antigen-binding fragment thereof or antibody construct according to the first aspect of the invention, or the ADC according to the fifth aspect of the invention (i), and a pharmaceutically acceptable excipient and/or carrier.

An eighth aspect of the present invention relates to the antibody, antigen-binding fragment thereof or antibody construct according to the first aspect of the invention, the ADC according to the fifth aspect of the invention (i), or the pharmaceutical composition according to the seventh aspect of the invention, for use in medicine.

A ninth aspect of the present invention relates to the antibody, antigen-binding fragment thereof or antibody construct according to the first aspect of the invention, the ADC according to the fifth aspect of the invention (i), or the pharmaceutical composition according to the seventh aspect of the invention, for use in the treatment and/or prevention of cancer and/or an inflammatory disease.

A tenth aspect of the present invention relates to the use of the antibody, antigen-binding fragment thereof or antibody construct according to the first aspect of the invention, the ADC according to the fifth aspect of the invention (i), the conjugate according to the fifth aspect of the invention (ii), or the pharmaceutical composition according to the seventh aspect of the invention:
- for targeting a therapeutic agent to a specific site wherein said specific site is characterized by the presence of tumor-associated macrophages (TAMs) with increased gp130 signaling or said specific site is characterized by increased gp130 signaling; and/or
- for detecting and/or quantifying the presence of gp130 in a sample.

An eleventh aspect of the present invention relates to a combination therapy comprising a neutralizing antibody, antigen-binding fragment thereof or antibody construct comprising an antigen-binding fragment thereof specifically binding to gp130 and at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC).

A twelfth aspect of the present invention relates to a method for increasing the therapeutic efficacy of at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC),
the method comprising the administration of a combination of said therapy and a neutralizing antibody, antigen-binding fragment thereof or antibody construct comprising an antigen-binding fragment thereof specifically binding to gp130, to a subject in need thereof.

A thirteenth aspect of the present invention relates to a method for reducing the immunosuppressive effect of tumor-associated macrophages (TAMs) in the tumor of a subject suffering from said tumor, the method comprising the administration of a neutralizing antibody, antigen-binding fragment thereof or antibody construct comprising an antigen-binding fragment thereof specifically binding to gp130, to a subject in need thereof.

### BRIEF DESCRIPTION OF THE FIGURES

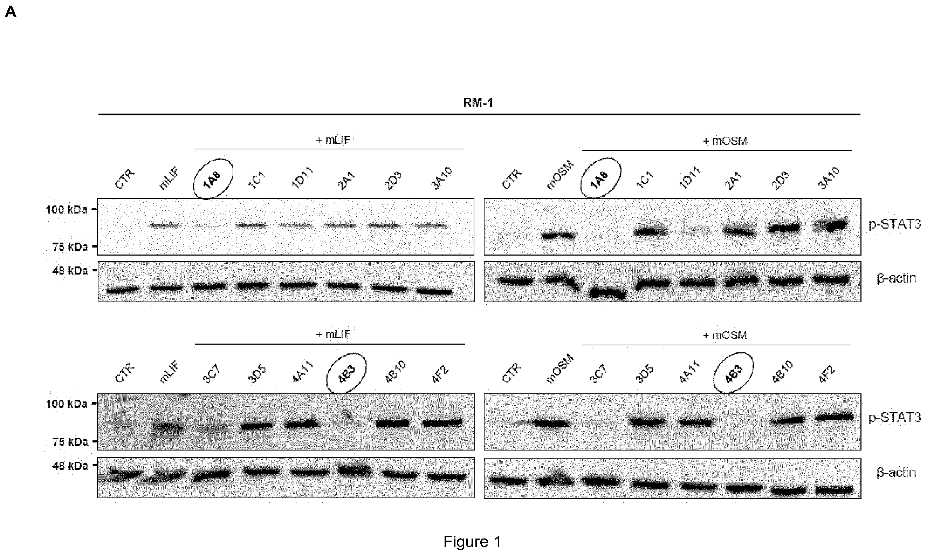

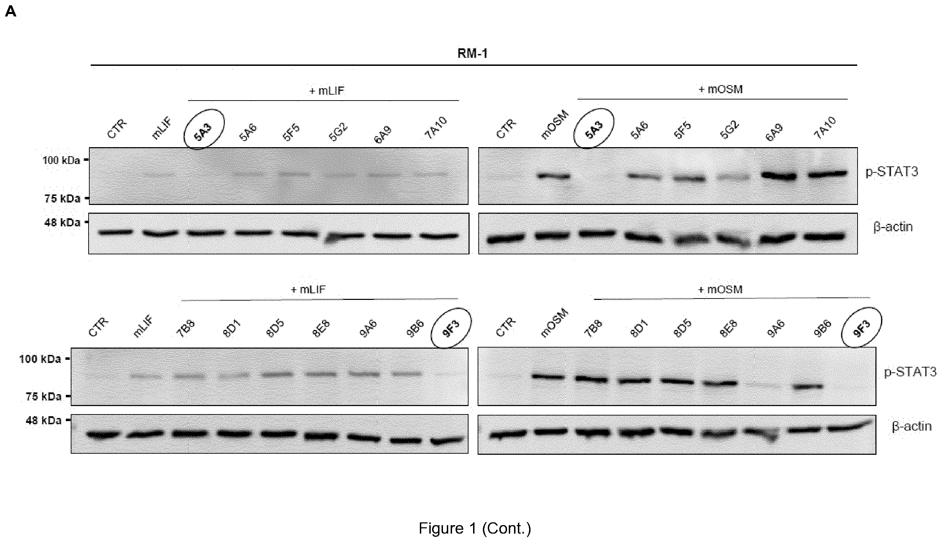

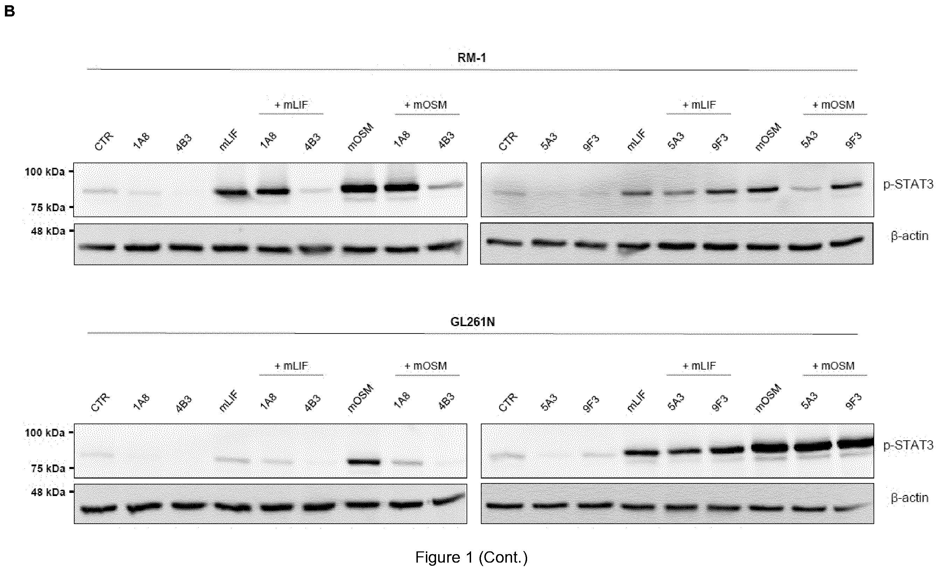

**Figure 1. (A)**Western blot analysis of p-STAT3 (Y705) levels in RM-1 cell line. Lanes correspond to untreated (CTR) or treated cells with recombinant murine LIF (mLIF) or OSM (mOSM) at 20 ng/mL. Pre-treatment with anti-murine gp130 hybridoma supernatant (1A8 to 9F3) was performed for 45 minutes. Selected clones for purification step are circled. **(B)** Western blot analysis of p-STAT3 (Y705) levels in RM-1 and GL261N cell lines. Lanes correspond to untreated (CTR) or treated cells with recombinant murine LIF (mLIF) or OSM (mOSM) at 20 ng/mL. Pre-treatment with anti-murine gp130 monoclonal antibody (1A8, 4B3, 5A3 or 9F3) was performed at 10 µg/mL for 45 minutes.

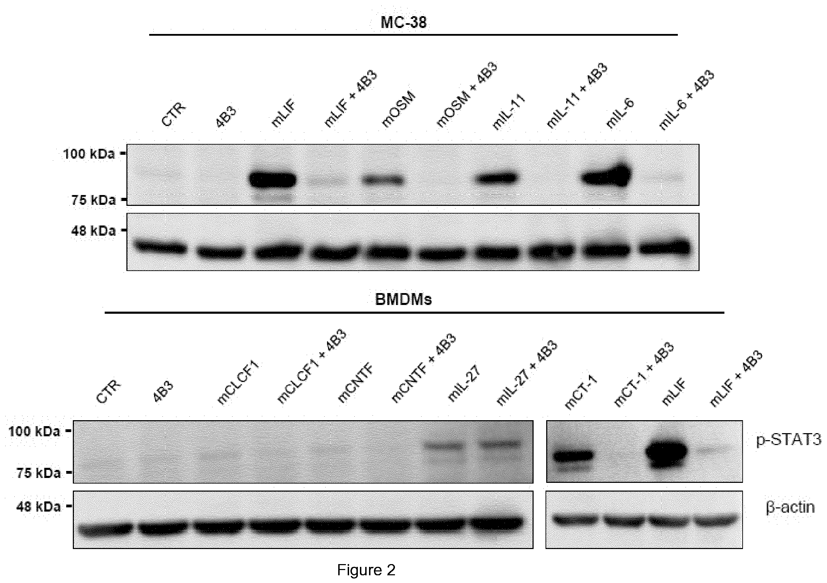

**Figure 2****.** Western blot analysis of p-STAT3 (Y705) levels in MC-38 cell line and in primary culture of bone marrow-derived macrophages (BMDM). Lanes correspond to untreated (CTR) or treated cells with recombinant murine cytokines at 20 ng/mL. Pre-treatment with anti-murine gp130 monoclonal antibody (4B3) was performed at 10 µg/mL for 45 minutes.

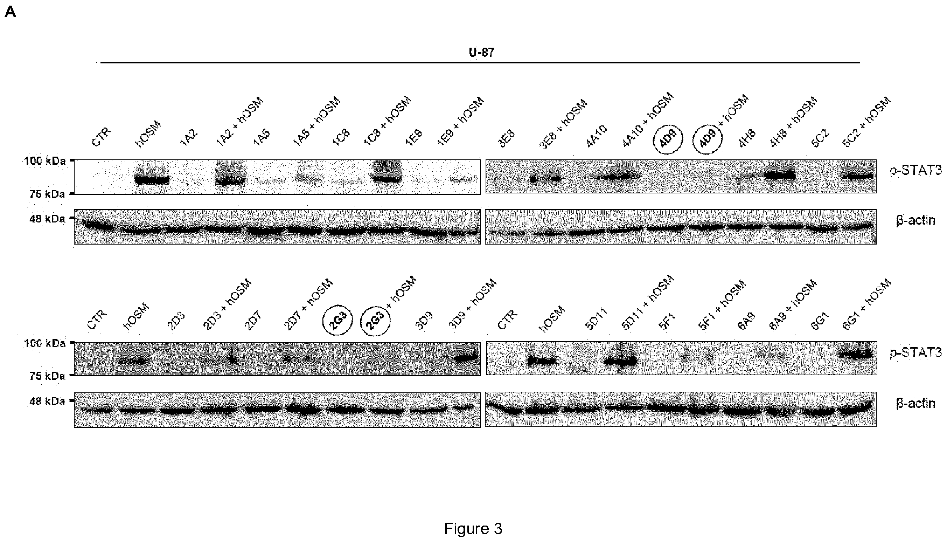

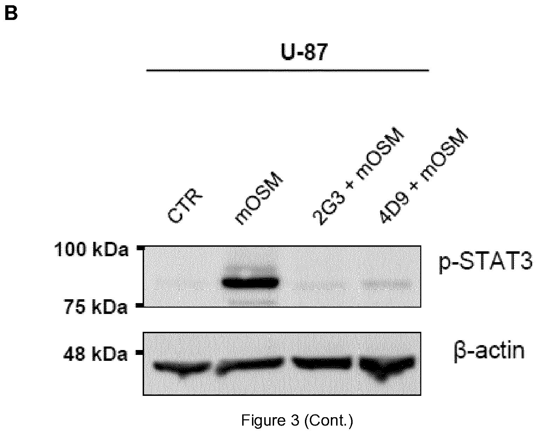

**Figure 3****.** Western blot analysis of p-STAT3 (Y705) levels in U-87 cell line. Lanes correspond to untreated (CTR) or treated cells with recombinant human OSM (hOSM) at 20 ng/mL: **(A)** Pre-treatment with anti-human gp130 hybridoma supernatant (1A2 to 6G1) was performed for 45 minutes. Selected clones for purification step are circled. **(B)** Pre-treatment with anti-human gp130 monoclonal antibody (2G3 or 4D9) was performed at 10 µg/mL for 45 minutes.

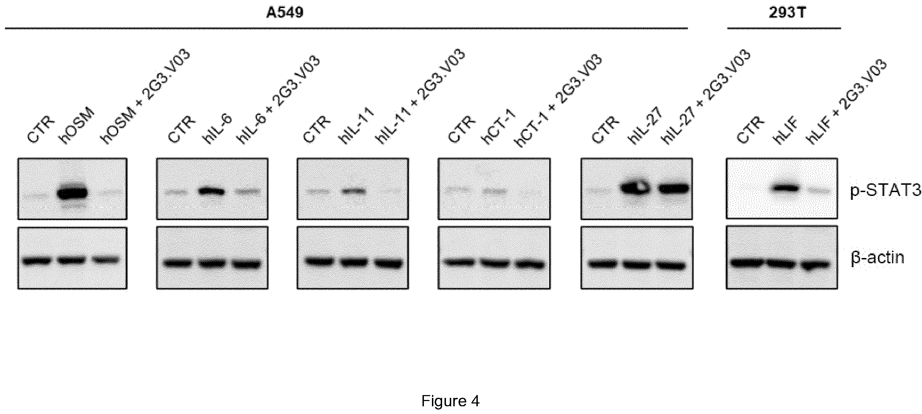

**Figure 4****.** Western blot analysis of p-STAT3 (Y705) levels in 293T **(A)** and A549 **(B)** cell lines. Lanes correspond to untreated (CTR) or treated cells with recombinant human cytokines at 20 ng/mL. Pre-treatment for 45 minutes with humanized anti-human gp130 monoclonal antibody (2G3.V03) was performed at 30 µg/mL.

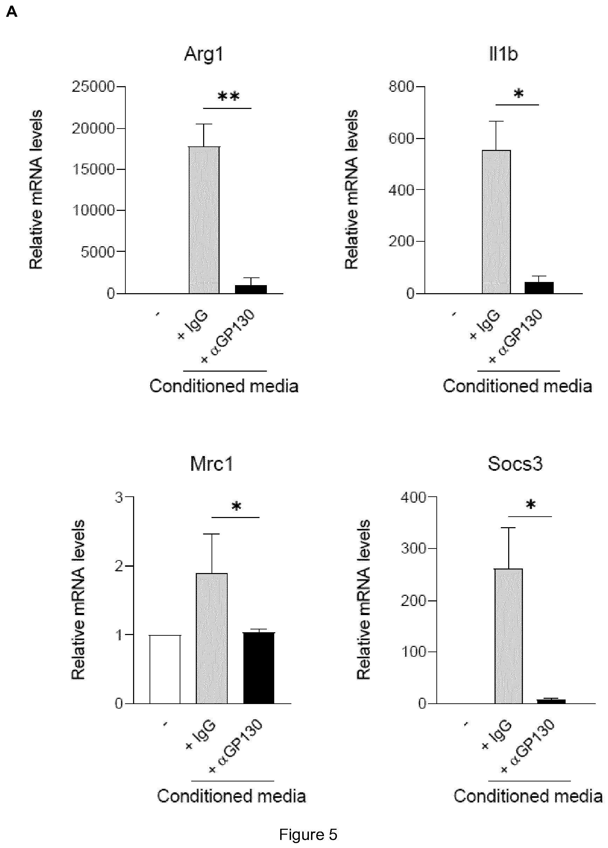

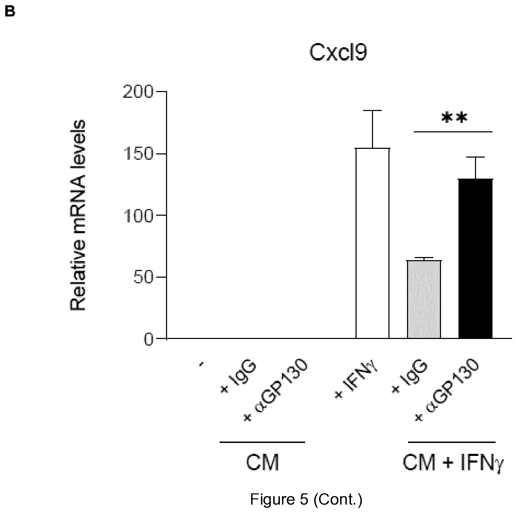

**Figure 5****.** The anti-GP130 antibody transformed immunosuppressive macrophages into immune activator macrophages. The conditioned medium (CM) from lung cancer cells induced an immunosuppressive phenotype in bone marrow-derived macrophages (BMDM). The anti-GP130 antibody inhibited the immunosuppressive phenotype and restored the IFNγ response, a sign of immune activity **(A)** BMDMs were treated with conditioned media (CM) of lung cancer cells (MuH163) for 48h in the presence or absence of anti-GP130 antibody. Cells were lysed and the mRNA expression of immunosuppressive markers was analyzed by qPCR. **(B)** BMDMs were treated with conditioned media (CM) of lung cancer cells (MuH163) for 48h in the presence or absence of anti-GP130 antibody. Cells were then stimulated with IFNγ for 6h and the mRNA expression of IFN-responsive gene Cxcl9 was analyzed by qPCR. Data are represented as mean ± SEM. Statistical significance (*, **) at p < 0.05, < 0.01 respectively; determined by two-tailed unpaired Student's t-test.

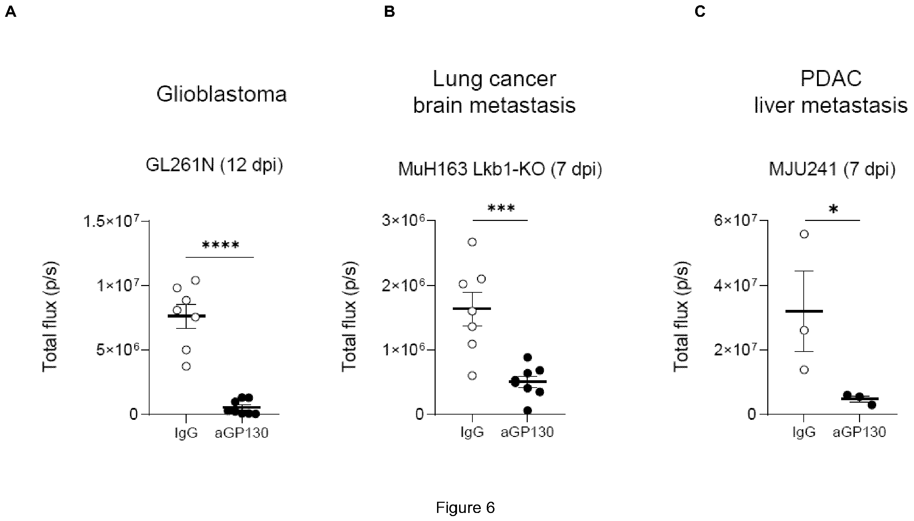

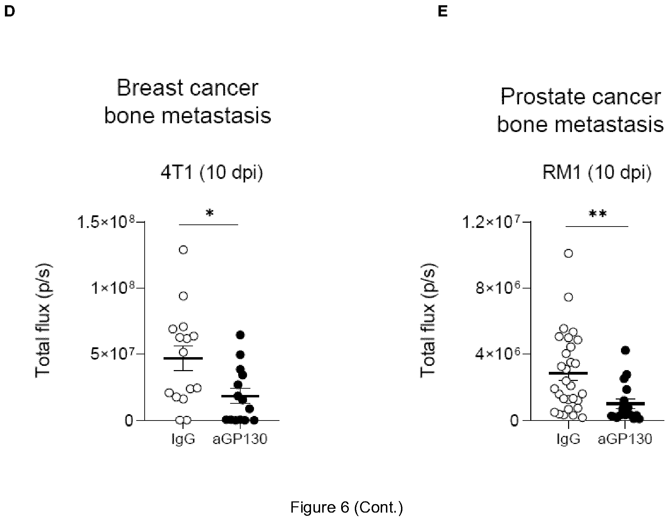

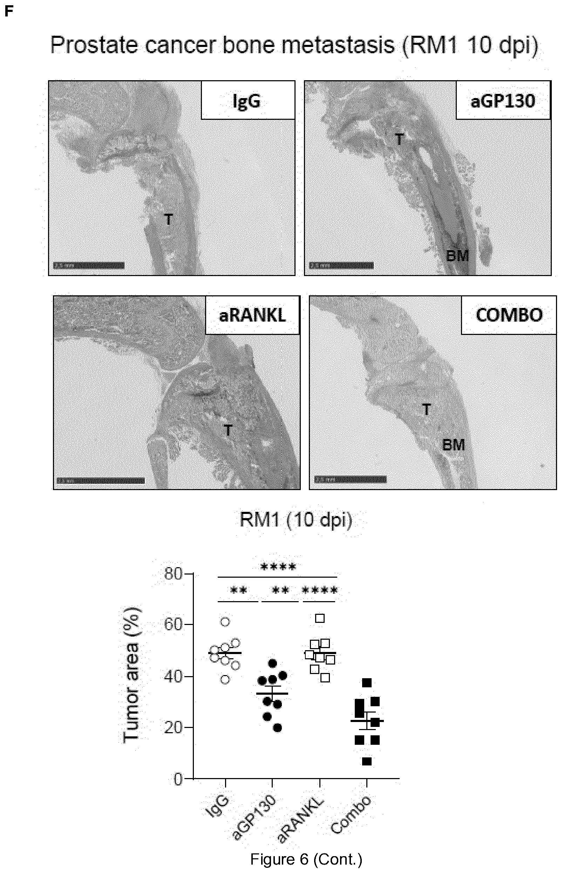

**Figure 6****.** The anti-GP130 antibody showed anti-tumor activity in different *in vivo* cancer models. **(A)** Tumor growth of glioblastoma (GL261N orthotopic syngeneic model); **(B)** prostate cancer bone metastasis (RM1 syngeneic model); **(C)** breast cancer bone metastasis (4T1 syngeneic model); **(D)** lung cancer brain metastasis (MuH163 Lkb1-KO syngeneic model; and **(E)** pancreatic ductal adenocarcinoma (PDAC) liver metastasis (MJU241 syngeneic model). Tumor growth was measured as total flux (p/s) at the indicated days post-inoculation (dpi). Isotype control (IgG) or anti-GP130 treatments started on the day of surgery (GL261N, MuH163 Lkb1-KO and MJU241) or 4 dpi (RM1 and 4T1). **(F)** The anti-GP130 antibody showed synergistic anti-tumor activity in prostate cancer bone metastasis in combination with the standard of care anti-RANKL (denosumab) antibody. Tumor growth of prostate cancer bone metastasis (RM1 syngeneic model) measured as tumor area by H&E. Tumor are labeled as T, whereas bone marrow as BM. Isotype control (IgG), anti-GP130 or anti-RANKL treatments started on 4 dpi. Data are represented as mean ± SEM. Statistical significance (*, **, ***, ****) at p < 0.05, < 0.01, < 0.001, < 0.0001 respectively; determined by two-tailed unpaired Student's t-test.

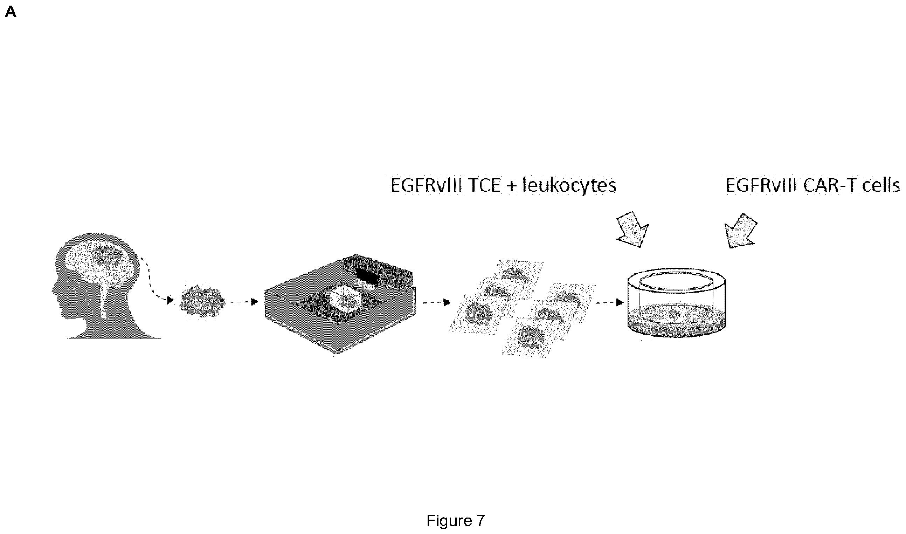

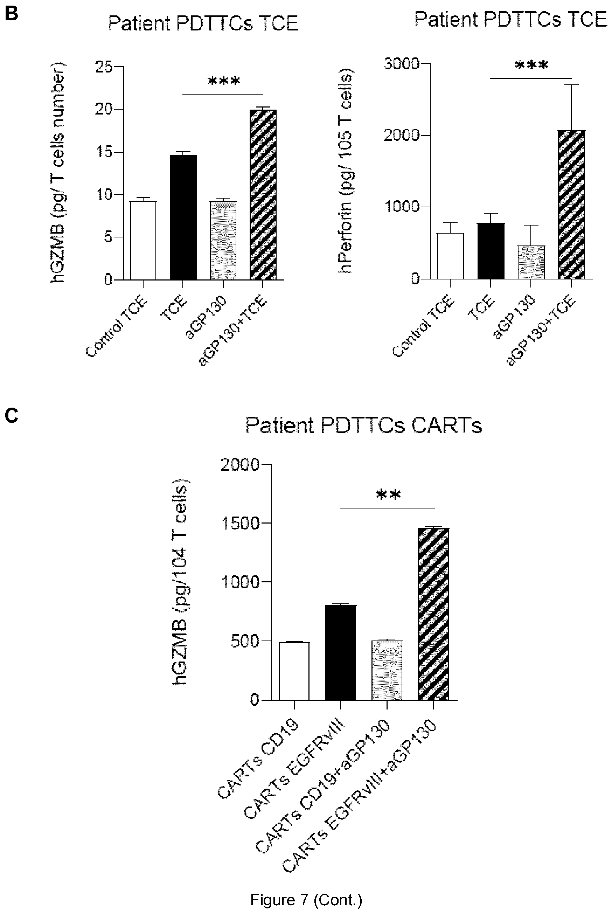

**Figure 7****.** The anti-GP130 antibody enhanced cytotoxicity of T cell engagers (TCE) and CAR-T cells in PDTTCs (patient-derived tumor tissue cultures) human cancer models. PDTTCs of an EGFRvIII positive GBM patient were incubated with autologous PBMCs and EGFRvIII TCE or EGFRvIII CAR-T cells for 72h and treated or untreated with anti-GP130 antibody (V03). **(A),** Scheme of the PDTTCs experiment. Patient tumors were included in agarose matrix and cut into 350µm slices with a vibratome and cultured with autologous PBMCs + EGFRvIII TCE or EGFRvIII CAR-T cells; **(B),** human Granzyme B (hGZMB) and human Perforin (hPerforin) ELISA of medium collected after 72h of EGFRvIII TCE treatment of PDTTCs; **(C),** human Granzyme B (hGZMB) ELISA of medium collected after 72h of EGFRvIII CAR-T cells treated PDTTCs. Data are represented as mean ± SEM. Statistical significance (**, ***) at p < 0.01, < 0.001 respectively; determined by two-tailed unpaired Student's t-test.

### DETAILED DESCRIPTION OF THE INVENTION

The present invention relates to the provision of new therapies for the treatment of cancer and, particular, solid tumors, wherein the solid tumor is characterized by the presence of tumor-associated macrophages (TAMs) with increased gp130 signaling. The present invention also relates to the provision of new therapies for the treatment of inflammatory diseases characterized by increased gp130 signaling. Additionally, the present invention also discloses the provision of combination therapies for the treatment of cancer, said therapies comprising a neutralizing antibody specific against gp130.

Unless otherwise defined, all technical terms used herein have the same meaning as commonly understood by one of ordinary skill in the art to which this invention belongs.

All the embodiments and definitions disclosed in the context of one aspect of the invention are also applicable to the other aspects of the invention.

### Antibody of the invention

The authors of the present invention have obtained a neutralizing antibody which specifically binds to glycoprotein130 (gp130) and which is capable to block the gp130 mediated IL-6 family of cytokines signaling pathway, which in turn inhibits the activation of STAT family proteins (see Figure 4 of the application) and to elicit an anti-tumor activity in different *in vivo* cancer models (see Figure 6 of the application).

The authors of the present invention have also identified glycoprotein 130 (gp130) as a factor that promotes the oncogenic and immunosuppressive features of TAMs, and have developed neutralizing antibodies targeting gp130 which are capable of reversing the TAM phenotype thereby generating a strong anti-tumor response, optionally in combination with immunotherapies in solid tumors (see Figures 6F and 7 of the application).

Thus, in a first aspect, the present invention relates to a neutralizing antibody, an antigen-binding fragment thereof or an antibody construct comprising an antigen-binding fragment thereof capable of specifically binding to and neutralizing gp130, hereinafter referred to as "the antibody of the invention", wherein said antibody, antigen-binding fragment thereof or antibody construct comprises:
(a) within the heavy chain of the variable region, a CDR comprising the amino acid sequence set forth in SEQ ID NO: 1 [CDR-H1], a CDR comprising the amino acid sequence set forth in SEQ ID NO: 2 [CDR-H2], and a CDR comprising the amino acid sequence set forth in SEQ ID NO: 3 [CDR-H3], or a functionally equivalent variant of said CDRs; and
(b) within the light chain of the variable region, a CDR comprising the amino acid sequence set forth in SEQ ID NO: 4 [CDR-L1], a CDR comprising the amino acid sequence set forth in SEQ ID NO: 5 [CDR-L2] (SAN), and a CDR comprising the amino acid sequence set forth in SEQ ID NO: 6 [CDR-L3], or a functionally equivalent variant of said CDRs.

In another aspect, the invention relates to an antibody which is capable of specifically binding to gp130 and of substantially neutralizing one or more of the cytokines of the IL-6 family but which is not capable of substantially neutralizing IL-27. In an embodiment, the anti-gp130 of the invention neutralizes the activity of one or more of Interleukin-6 (IL-6), Interleukin-11 (IL-11), leukemia inhibitory factor (LIF), oncostatin M (OSM), ciliary neurotrophic factor (CNTF), cardiotrophin-1 (CT-1), novel neurotrophin-1/B cell stimulating factor-3 or cardiotrophin-like cytokine factor 1 (CLCF1), and neuropoietin (NP)) but is not capable of neutralizing IL-27. In an embodiment, the anti-gp130 antibody of the invention neutralizes the activity of OSM, IL-6, IL-11, CT- and LIF but is not capable of neutralizing IL-27.

The term "neutralizing" or "neutralize", as used herein, refers to an antibody which is capable of neutralizing the biological gp130-mediated signaling activity of the IL-6 family of cytokines resulting from the specific binding of the cytokine to a complex formed by its specific receptor and gp130. Cytokines of the IL-6 failies include Interleukin-6 (IL-6), Interleukin-11 (IL-11), Interleukin-27 (IL-27), leukemia inhibitory factor (LIF), oncostatin M (OSM), ciliary neurotrophic factor (CNTF), cardiotrophin-1 (CT-1), novel neurotrophin-1/B cell stimulating factor-3 or cardiotrophin-like cytokine factor 1 (CLCF1), and neuropoietin (NP)). The activation of the complex between gp130 and the specific receptor for each member of the IL-6 family of cytokines leads to the phosphorylation of STAT3 at position Y705 It will be appreciated that the term "neutralizing", as used herein, refers to a reduction in biological signaling activity which may be partial or complete. In particular embodiment, the neutralizing antibody according to the invention refers to an antibody which inhibits (partially or completely) the oncogenic and immunosuppressive properties of the tumor associated macrophages (TAMs). For example, said neutralizing activities are typically evaluated according to the assay described in the examples of the application and illustrated in Figure 4 and, in particular, by determining the capacity of the antibody to inhibit STAT3 phosphorylation in response to members of the LIF, OSM, IL-6, IL-11 and/or CT-1 members of IL6 family of cytokines (but not of IL-27) which, as explained above, requires the interaction of the cytokine with a complex containing the cytokine specific receptor and gp130,. The neutralizing antibody of the invention is capable of neutralizing at least 10%, at least 20%, at least 30%, at least 40%, at least 50%, at least 55%, at least 60%, at least 65 %, at least 70%, at least 75%, at least 80%, at least 85%, at least 90%, at least 91 %, at least 92%, at least 93%, at least 94%, at least 95%, at least 96%, at least 97%, at least 98%, at least 99% or 100% of the gp130-mediated activity when determined using any standard assay for determining gp130-mediated signaling. In a preferred embodiment, the gp130-medianted signaling is determined as described in the examples of the application and illustrated in Figure 4, i.e. by determining the capacity of the antibody to block the gp130 mediated signaling by any member of the IL-6 family of cytokines (LIF, OSM, IL-6, IL-11) except IL27, which can be measured, for instance, by detecting the degree of phosphorylation in STAT3.

In an embodiment the antibody of the invention is capable of specifically binding to human gp130.

The term "antibody" or "Ab", as used herein, refers to a molecule which has the ability to specifically bind to an epitope of a molecule ("antigen"). Naturally occurring antibodies typically comprise a tetramer which is usually composed of at least two heavy (H) chains and at least two light (L) chains. Each heavy chain (50-75 KDa) is comprised of a heavy chain variable domain (or heavy chain variable region) (abbreviated herein as VH) and a heavy chain constant domain, usually comprised of three domains (CH1, CH2 and CH3). Heavy chains can be of any isotype, including **α, δ, γ, ε, µ,** which corresponds to five antibodies isotypes, IgA (IgA1 and IgA2), IgD, IgG (IgG1, IgG2, IgG3 and IgG4 subtypes), IgD, and IgM. Each light chain (25 KDa) is comprised of a light chain variable domain (or light chain variable region) (abbreviated herein as VL) and a light chain constant domain (CL). Light chains include kappa chains and lambda chains. The heavy and light chain variable domain is typically responsible for antigen recognition, while the heavy and light chain constant domain may mediate the binding of the immunoglobulin to host tissues or factors, including various cells of the immune system (e.g., effector cells) and the first component (C1 q) of the classical complement system. The VH and VL domains can be further subdivided into domains of hypervariability, termed "complementarity determining regions" that are interspersed with domains of more conserved sequence, termed "framework regions" (FR). Each VH and VL is composed of three CDR domains and four FR domains arranged from amino-terminus to carboxy-terminus in the following order: FR1-CDR1-FR2-CDR2-FR3-CDR3-FR4. The term "complementarity determining region", "CDR", "hypervariable region" or "HVR", as used herein, refers to each of the regions of an antibody variable domain which are hypervariable in sequence and/or form structurally defined loops ("hypervariable loops"), and the region within an antibody where this protein complements an antigen's shape. Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy chain and the light chain are characterized by three CDRs, respectively CDR-VH1, CDR-VH2, CDR-VH3 and CDR-VL1, CDR-VL2, CDR-VL3. The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue:W34-40), by following the numbering provided by Kabat et al, Sequences of Proteins of Immunological Interest, 5th ed.,Public Health Service, National Institutes of Health, Bethesda, Md. (1991), or by following the numbering provided by Chothia et al. (1989, Nature 342:877-83).This particular region has been described by Kabat et al., Sequences of Proteins of Immunological Interest, 5th Ed. Public Health Service, National Institutes of Health, Bethesda, Md. (1991) and by Chothia et al., J. Mol. Biol. 196:901-917 (1987), where the definitions include overlapping or subsets of amino acid residues when compared against each other. The exact residue numbers which encompass a particular CDR will vary depending on the sequence and size of the CDR. Those skilled in the art can routinely determine which residues comprise a particular CDR given the variable region amino acid sequence of the antibody. The CDR sequences given herein are generally according to the Kabat definition. The term "Framework" or "FR" refers to variable domain residues other than hypervariable region (HVR) residues. The FR of a variable domain generally consists of four FR domains: FR1, FR2, FR3, and FR4. Consequently, both the heavy variable chain and the light variable chain are characterized by four FR, respectively FR-VH1, FR-VH2, FR-VH3, FR-VH4 and FR-VL1, FR-VL2, FR-VL3, FR-VL3.

The term "neutralizing" or "neutralize", as used herein, refers to an antibody which is capable of neutralizing the biological signaling activity of the IL-6 family of cytokines gp130 mediated resulting from the specific binding of the cytokine to a complex formed by its specific receptor and gp130. Cytokines of the IL-6 family include Interleukin-6 (IL-6), Interleukin-11 (IL-11), Interleukin-27 (IL-27), leukemia inhibitory factor (LIF), oncostatin M (OSM), ciliary neurotrophic factor (CNTF), cardiotrophin-1 (CT-1), novel neurotrophin-1/B cell stimulating factor-3 or cardiotrophin-like cytokine factor 1 (CLCF1), and neuropoietin (NP)). The activation of the complex between gp130 and the specific receptor for each member of the IL-6 family of cytokines leads to the phosphorylation of STAT3 at position Y705. It will be appreciated that the term "neutralizing", as used herein, refers to a reduction in biological signaling activity which may be partial or complete. In particular embodiment, the neutralizing antibody according to the invention refers to an antibody which inhibits (partially or completely) the oncogenic and immunosuppressive properties of the tumor associated macrophages (TAMs). For example, said neutralizing activities are typically evaluated according to the assay described in the examples of the application and illustrated in Figure 4 and, in particular, by determining the capacity of the antibody to inhibit STAT3 phosphorylation in response to the LIF, OSM, IL-6, IL-11 and/or CT-1 members of the IL-6 family of cytokines (but not of IL-27) which, as explained above, requires the interaction of the cytokine with a complex containing the cytokine specific receptor and gp130 to,. The neutralizing antibody of the invention is capable of neutralizing at least 10%, at least 20%, at least 30%, at least 40%, at least 50%, at least 55%, at least 60%, at least 65 %, at least 70%, at least 75%, at least 80%, at least 85%, at least 90%, at least 91 %, at least 92%, at least 93%, at least 94%, at least 95%, at least 96%, at least 97%, at least 98%, at least 99% or 100% of the gp130-mediated activity when determined using any standard assay for determining gp130-mediated signaling. In a preferred embodiment, the gp130-medianted signaling is determined as described in the examples of the application and illustrated in Figure 4, i.e. by determining the capacity of the antibody to block the gp130 mediated signaling by any member of the IL-6 family of cytokines (LIF, OSM, IL-6, IL-11) except IL-27, which can be measured, for instance, by detecting the degree of phosphorylation in STAT3.

The antibody of the invention is capable of neutralizing the signaling activity in response to the LIF, OSM, IL-6, IL-11 and/or CT-1 members of the IL-6 family of cytokines as a result of their interaction with receptor complexes containing their specific receptors and gp130.

In another embodiment, the antibody of the invention is not capable of neutralizing the signaling activity in response to the IL-27 member of the IL-6 family of cytokines as a result of its interaction with the complex containing its specific receptor and gp130.

The term "specifically binds", "specific binding" or "specifically recognizes", when used in the present invention to refer to the binding of an antibody of the invention to gp130, is understood as the capacity of the antibody of the invention to bind specifically to gp130 by means of the existence of complementarity between the three-dimensional structures of the two molecules with a substantially higher affinity for non-specific binding such that the binding between said antibody of the invention and gp130 preferably takes place before the binding of any of said molecules with respect to the other molecules present in the reaction mixture. This results in that the antibody of the invention does not cross-react with other molecules. Cross-reactivity of the antibody of the invention may be tested, for example, by assessing binding of said antibody of the invention under conventional conditions to the protein of interest as well as to a number of more or less (structurally and/or functionally) closely related proteins. For instance, a binding can be considered specific if the binding affinity between the antibody of the invention and gp130 has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

The capacity of the antibody of the invention as described herein, to bind to gp130 can be determined by a number of assays that are well known in the art. Preferably, the binding capacity of the binding agents is determined by immunoprecipitation or by an *in vitro* binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry.

The term "glycoprotein 130", "gp130" or "GP130", as used herein, also known as IL6-ST, IL6-beta or CD 130, is well known in the art and refers to a transmembrane protein which is the founding member of the class of all cytokine receptors. It forms one subunit of type I cytokine receptors within the IL-6 receptor family. Human GP130 gene refers encodes a 857 amino acid polypeptide provided in the GenPept database under accession number : NP_002175.2 (version of 29 October 2024) and which is encoded by the nucleic acid sequence provided in the GenBank database under accession number NM_002184.4 (version of 29 October 2024).

The antibody of the invention comprises:
(a) within the heavy chain of the variable region, a CDR comprising the amino acid sequence set forth in SEQ ID NO: 1 [CDR-H1], a CDR comprising the amino acid sequence set forth in SEQ ID NO: 2 [CDR-H2], and a CDR comprising the amino acid sequence set forth in SEQ ID NO: 3 [CDR-H3], or a functionally equivalent variant of said CDRs; and
(b) within the light chain of the variable region, a CDR comprising the amino acid sequence set forth in SEQ ID NO: 4 [CDR-L1], a CDR comprising the amino acid sequence set forth in SEQ ID NO: 5 [CDR-L2] (SAN), and a CDR comprising the amino acid sequence set forth in SEQ ID NO: 6 [CDR-L3], or a functionally equivalent variant of said CDRs.

As it is used herein, the term "functionally equivalent variant", when referred to a CDR is used to define a sequence variant of a particular CDR having substantially similar sequence identity with it and substantially maintaining its capacity to bind to its cognate antigen when being part of an antibody or antibody-binding fragments described herein and in the presence of the remaining CDRs of the antibody within the antibody, antigen-binding fragment thereof or antibody construct. For instance, functionally equivalent variants of the CDR1 are those CDR variants which, when inserted into an antibody or antigen-binding fragment thereof, in the presence of the CDR2 and CDR3 of the same antibody, result in an antibody in which the binding activity towards gp130 and the gp130-neutralizing activity is substantially preserved. For example, a functionally equivalent variant of a CDR sequence may be a polypeptide sequence derivative of said sequence comprising the addition, deletion or substitution of one or more amino acids. In one embodiment, the substitution of one amino acid by other in the functionally equivalent variant is a conservative substitution.

As used herein, the term "conservative substitution" refers to the replacement of an amino acid by another amino acid having similar chemical properties. Conservative substitution tables providing functionally similar amino acids are well known in the art. The following six groups each contain amino acids that are conservative substitutions for one another:
- Alanine (A), Serine (S), Threonine (T);
- Aspartic acid (D), Glutamic acid (E);
- Asparagine (N), Glutamine (Q);
- Arginine (R), Lysine (K);
- Isoleucine (I), Leucine (L), Methionine (M), Valine (V); and
- Phenylalanine (F), Tyrosine (Y), Tryptophan (W).

Functionally equivalent variants of a CDR sequence according to the invention include CDR sequences having at least 70%, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequence of any one of the sequences set forth in SEQ ID NO: 1 to 6. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence of any one of the sequences set forth in SEQ ID NO: 1 to 6. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequences of any one of the sequences set forth in SEQ ID NO: 1 to SEQ ID NO: 6.

Functionally equivalent variants of a CDR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence of any one of the sequences set forth in SEQ ID NO: 1 to 6 to bind to its cognate antigen when being part of the antibody or antibody fragment of the invention. This capacity to bind to its cognate antigen may be determined as a value of affinity, avidity, specificity and/or selectivity of the antibody or antibody fragment to its cognate antigen.

In an embodiment, the antibody of the invention is a human antibody. In another particular embodiment, the antibody of the invention is of non-human origin, preferably of murine origin. In a particular embodiment, the antibody of the invention is an antibody of human origin or a humanized antibody. In a particular embodiment, the antibody of the invention is an animal antibody, a chimeric antibody, a human antibody or a humanized antibody.

An "animal antibody", as used herein, is understood as a recombinant antibody that has been genetically manipulated to contain the heavy and light variable domains of an animal antibody and containing sequences of a human constant domain, preferably the constant domain of human gamma 1 or 4 immunoglobulin (or a PE variant).

A "chimeric antibody", as used herein, is understood as antibodies constructed with variable regions of an antibody of a species (usually a mammal in which the monoclonal antibody was generated) and constant regions of another species (that species in which the chimeric antibody is going to be used). The objective of said construct is to obtain an antibody with the original monoclonal antibody but which is less immunogenic and better tolerated in the subject who is going to be treated, with an improved serum half-life and which can be recognized by immunological effector mechanisms, i.e., the complement, the Fc receptor of cytotoxic cells or other specific immunoglobulin receptors which show species specificity. In a preferred embodiment, the chimeric antibodies are formed by murine variable regions and human constant regions.

A "humanized antibody", as used herein, is understood as an antibody from a nonhuman organism, typically a murine antibody, which conserves the antigen binding properties of the parent antibody, but which is less immunogenic in human beings. This can be achieved by means of different processes, which include (a) grafting the complete nonhuman variable domains into human constant regions to generate chimeric antibodies; (b) grating only the nonhuman complementarity determining regions (CDR) in a human framework and the constant regions, with or without retaining the critical framework residues; and (c) transplanting the complete nonhuman variable domains, but "concealing them" with a section similar to the human variable domain by means of replacing the surface residues. Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain.

Humanization can be essentially performed following the method of Winter and coworkers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. In practice, humanized antibodies are typically human antibodies in which some hypervariable region residues and possibly some framework region (FR) residues are substituted by residues from analogous sites in rodent antibodies. The choice of human variable domains, both light and heavy, to be used in making the humanized antibodies is very important to reduce immunogenicity retaining the specificity and affinity for the antigen. According to the so called "best-fit" method, the sequence of the variable domain of a rodent antibody is screened against the entire library of known human variabledomain sequences. The human sequence which is closest to that of the rodent is then accepted as the human framework region (FR) for the humanized antibody (Suns et al., J. Immunol., 151:2296 (1993); Chothia et al., J. Mol. Biol, 196:901 (1987)). Another method uses a particular framework region derived from the consensus sequence of all human antibodies of a particular subgroup of light or heavy chains. The same framework may be used for several different humanised antibodies (Carter et al., Proc. Natl. Acad. Sci. USA, 89:4285 (1992); Presta et al., J. Immunol., 151:2623 (1993)).

It is further important that antibodies are humanized, with retention of high affinity for the antigen and other favourable biological properties. To achieve this goal, according to a preferred method, humanized antibodies are prepared by a process of analysis of the parental sequences and various conceptual humanised products using three-dimensional models of the parental and humanized sequences.

A further step in this approach, to make an antibody more similar to humans, is to prepare the so called primatised antibodies, i.e. a recombinant antibody which has been genetically manipulated to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10: 1458-1460 (1992); US 5,658,570 and US 6,113,898. These antibodies have been reported to exhibit a high degree of homology to human antibodies, i.e., 85-98%, display human effector functions, have reduced immunogenicity, and may exhibit high affinity to human antigens. Another highly efficient means for generating recombinant antibodies is disclosed by Newman, Biotechnology, 10: 1455-1460 (1992).

A "human antibody", as used herein, is understood as an antibody containing human light and heavy chains as well as constant regions, produced by means of any of the known standard methods. The human antibody may include amino acid residues not encoded by human germline immunoglobulin sequences (e.g., mutations introduced by random or site-directed specific mutagenesis *in vitro* or by somatic mutation *in vivo*).

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH gene in chimeric and germ-line mutant mice results in the complete inhibition of endogenous antibody production. Transfer of the human germ-line immunoglobulin gene array in such germ line mutant mice will result in the production of human antibodies after immunization. See, e.g., Jakobovits et al., Proc. Mad. Acad. Sci. USA, 90:255 1 (1993); Jakobovits et al., Nature, 362:255-258 (1993), Lonberg, 2005, Nature Biotech. 23:1117-25. Human antibodies may also be generated by *in vitro* activated B cells or SCID mice with its immune system reconstituted with human cells. Once a human antibody is obtained, its coding DNA sequences can be isolated, cloned and introduced into an appropriate expression system, i.e., a cell line, preferably from a mammal, which subsequently express and liberate it into a culture media from which the antibody can be isolated.

In a particular embodiment, the antibody of the invention is a monoclonal antibody or a fragment of said antibody which retains the capacity to bind to gp130. Said antibodies are preferably human or humanized antibodies.

In an embodiment, the antibody of the invention comprises:
(a) within the heavy chain of the variable region, a FR region comprising the amino acid sequence set forth in SEQ ID NO: 7 [FR-H1], a FR region comprising the amino acid sequence set forth in SEQ ID NO: 8 [FR-H2], a FR region comprising the amino acid sequence set forth in SEQ ID NO: 9 [FR-H3], and a FR region comprising the amino acid sequence set forth in SEQ ID NO: 10 [FR-H4], or a functionally equivalent variant of said FR regions; and
(b) within the light chain of the variable region, a FR region comprising the amino acid sequence set forth in SEQ ID NO: 11 [FR-L1], a FR region comprising the amino acid sequence set forth in SEQ ID NO: 12 [FR-L2], a FR region comprising the amino acid sequence set forth in SEQ ID NO: 13 [FR-L3], and a FR region comprising the amino acid sequence set forth in SEQ ID NO: 14 [FR-L4], or a functionally equivalent variant of said FR regions.

As it is used herein, the term "functionally equivalent variant", when referred to a FR is used to define a sequence variant of a particular FR having substantially similar sequence identity with it and substantially maintaining its capacity to bind to its cognate antigen when being part of an antibody or antibody-binding fragments described herein and in the presence of the remaining FRs of the antibody within the antibody, antigen-binding fragment thereof or antibody construct. For example, a functionally equivalent variant of a FR sequence may be a polypeptide sequence derivative of said sequence comprising the addition, deletion or substitution of one or more amino acids.

Functionally equivalent variants of a FR sequence according to the invention include FR sequences having at least approximately 70% , at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequence of any one of the sequences set forth in SEQ ID NO: 7-14. It is also contemplated that functionally equivalent variants of a FR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence of any one of the sequences set forth in SEQ ID NO: 7-14. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence of any one of the sequences set forth in SEQ ID NO: 7-14.

Functionally equivalent variants of a FR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence of any one of the sequences set forth in SEQ ID NO: 7-14 to bind to its cognate antigen when being part of an antibody or antibody fragment of the invention. This capacity to bind to its cognate antigen may be determined as a value of affinity, avidity, specificity and/or selectivity of the antibody or antibody fragment to its cognate antigen.

In another embodiment, the antibody of the invention comprises:
(a) at least a heavy chain of the variable region comprising the sequence set forth in SEQ ID NO: 15 [VH], or a functionally equivalent variant thereof; and
(b) at least a light chain of the variable region comprising the sequence set forth in SEQ ID NO: 16 [VL], or a functionally equivalent variant thereof.

In another embodiment, the VH of the antibody of the invention comprises a sequence having at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 15. In another embodiment, the VL of the antibody of the invention comprises a sequence having at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 16.

The terms "identity", "identical" or "percent identity" in the context of two or more amino acid or nucleotide sequences, refer to two or more sequences or subsequences that are the same or have a specified percentage of nucleotide or amino acid residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm incorporated into the NBLAST and XBLAST programs (Altschul et al., 1991, Nucleic Acids Res., 25:3389-3402). Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

It will be understood that the preservation of the activity in the functionally equivalent variant will have to be determined by reference to the activity of the reference polypeptide. Accordingly, a functionally equivalent variant of a CDR will be defined as a polypeptide which, when present in an VH or an VL chain in combination with the other CDRs of the VH or VL chain and the corresponding VH or VL chain as the case may be results in an antibody which substantially preserves its ability of specifically binding to the antigen.

In an embodiment, the functionally equivalent variant of a CDR will be defined as a polypeptide which, when present in an VH or an VL chain in combination with the other CDRs of the VH or VL chain and the corresponding VH or VL chain as the case may be results in an antibody which substantially preserves its ability to inhibit the gp130 mediated IL-6 family of cytokines signaling pathway, leading to the inhibition of STAT3 phosphorylation in Y705 in response to LIF, OSM, IL-6, IL-11 and/or CT-1, but not in response to IL-27. This can be tested by a method as defined in the examples of the present application and as illustrated in Figure 4. In an embodiment, the antibody of the invention can be of any isotype. The choice of isotype typically will be guided by the desired effector functions, such as ADCC induction. Exemplary isotypes are IgG1, IgG2, IgG3, and IgG4. Either of the human light chain constant regions, kappa or lambda, may be used.

In another embodiment, the antibody of the invention is an Immunoglobulin G (Ig G).

In a preferred embodiment, the antibody of the invention is an IgG1 isotype.

As used herein, "isotype" refers to the antibody class (e.g., IgG1, IgG2, IgG3, IgG4, IgM, IgA1, IgA2, IgD, and IgE antibody) that is encoded by the heavy chain constant region genes.

The term "antibody", as used herein, includes any type of known antibody, such as, for example, polyclonal antibodies, monoclonal antibodies, and genetically engineered antibodies, such as chimeric antibodies, humanized antibodies, primatized antibodies, human antibodies, antibodies of non-human origin, bispecific antibodies, single domain antibodies, VHH antibodies, caninized antibodies, felinized antibodies, canine antibodies, and feline antibodies.

In an embodiment, the antibody of the invention is a monoclonal antibody.

The term "monoclonal antibody" or "mAb", as used herein, refers to a preparation of antibody molecules of homogeneous molecular composition. i.e., the individual antibodies comprising the population are identical except for possible naturally occurring mutations that may be present in minor amounts. A monoclonal antibody composition displays a single binding specificity and affinity for a particular epitope or antigenic binding site. The monoclonal antibodies are produced by a hybrid cell product of the fusion of a B-cell clone descendent of a single unique parent cell and a tumor plasma cell. Furthermore, in contrast to polyclonal antibody preparations that typically include different antibodies directed against different determinants (epitopes), each monoclonal antibody is directed against a single determinant on the antigen.

In another embodiment, the invention relates to an antigen-binding fragment of the antibody of the invention as defined above.

The term "antigen-binding fragment", "antigen-binding domain" or "antigen-binding portion" of an antibody, as used herein, refers to one or more fragments of an antibody that retain the ability to specifically bind to an antigen (e.g., gp130). Such "fragments" are, for example between about 8 and about 1500 amino acids in length, suitably between about 8 and about 745 amino acids in length, suitably about 8 to about 300, for example about 8 to about 200 amino acids, or about 10 to about 50 or 100 amino acids in length. It has been shown that the antigen-binding function of an antibody can be performed by fragments of a full-length antibody. Examples of binding fragments encompassed within the term "antigen-binding fragment" of an antibody, include, without being limited to, (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) or (vii) a combination of two or more isolated CDRs which may optionally be joined by a synthetic linker. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

In an embodiment, the antibody of the invention is an antigen-binding fragment selected from the group consisting of Fv, Fab, F(ab')₂ and Fab'.

Numerous approaches make use of the molecular biology and genetic techniques such as the good knowledge of the genetics and structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain monoclonal antibodies (mAbs) of human origin avoiding the non-ethically admissible proceedings in healthy humans. In other approaches the molecular weight and size are reduced e.g. in order to improve the distribution of the molecule into solid tumours. Other possibilities are conjugation in a molecule of binding domains for more than one target molecule (bispecific antibody or also triespecific, etc.) or the conjugation of an antibody or a fragment with another molecule with the desired function e.g. a toxic agent, a hormone, growth factor, an immunomodulating agent (immunosuppressor or immunostimulator), an inhibitor of cell growth, etc. In general, all the resultant molecules retain at least one variable domain of an antibody, which gives the high specificity and affinity characteristic of the antigen-antibody binding.

The "Fv" is the minimal antibody fragment containing a complete antigen binding and antigen recognition site. This region consists of a variable domain of a variable light chain and heavy chain dimer in a strong noncovalent association. In this configuration the three hypervariable regions of each variable domain interact to define an antigen binding site on the surface of the VH-VL dimer. As a whole, the six hypervariable regions confer antigen-antibody specificity to the antibody. However, even a single variable domain (or half an Fv, which comprises only three hypervariable regions specific for an antigen) has antigen recognition and binding capacity, although with less affinity than the complete binding site.

The "Fab" fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region.

Various techniques have been developed for the production of antibody fragments. Traditionally, these fragments were derived via proteolytic digestion of intact antibodies but more recently these fragments can be produced directly by recombinant host cells. The Fab and F(ab')₂ fragments can be obtained by means of enzymatic or chemical cleavage of the intact antibodies. Papain digestion of antibodies produces two identical antigen binding fragments, referred to as "Fab" fragments, each with a single antigen binding site, and a residual "Fc" fragment, the name of which reflects its capacity for readily crystallizing. Pepsin treatment yields an F(ab')₂ fragment which has two antigen binding sites and which is still capable of cross-linking to the antigen.

It will be immediately apparent for the person skilled in the art that the antibody of the invention may be modified by genetic engineering to yield constructs with modified avidity and/or functionality. There are numerous approaches in the art to obtain antibody constructs, such as those highlighted in Cuesta et al. 2010 (Trends Biotechnol. 28:355-62).

In another embodiment, the invention relates to an antibody construct which contains an antigen-binding fragment of the antibody of the invention.

The term "antibody construct", as used herein, refers to constructs based on the antibody of the invention that are typically generated by genetic engineering techniques. As used herein, the antibody construct refers to a construct that comprises at least an antigen-binding fragment of an antibody that retain the ability to specifically bind to an antigen (e.g., gp130). Examples of antibody constructs include scFv, (scFv)₂, scFv-Fc, minibody, nanobody, diabody or bispecific antibody. These and other antibody constructs are reviewed in Cuesta *et al.* 2010 (cited *supra*), and are included herein by reference.

In an embodiment, the antibody of the invention is an antibody construct selected from the group consisting of scFv, (scFv)₂, scFv-Fc, minibody, nanobody, diabody and bispecific antibody.

The term "single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer-Verlag, N.Y., pp. 269-315 (1994). The scFvs can be obtained by conventional methods by binding the variable region of the heavy and light chains (Fv region) by means of an amino acid bridge. The scFvs can be prepared by fusing the DNA encoding a linker peptide between the DNAs encoding the polypeptides of the variable regions (VL and VH). The production of scFvs is described in a number of documents, for example, in US patent US 4,946,778, Bird (Science 242: 423, 1988), Huston et al. (Proc. Natl. Acad Sci USA 85: 5879, 1988) and Ward et al. (Nature 334: 544, 1989).

The term "(scFv)₂", as used herein, refers to an antibody construct that comprises a first and a second scFv operably connected (e.g., via a linker). The first and second scFv can specifically bind the same or different antigens. In some embodiments, the first and second scFv are operably connected by an amino via an amino acid linker.

The term "scFv-Fc", as used herein, refers to an antibody construct that comprises a scFv operably linked to an Fc domain or subunit of an Fc domain.

The term "minibody" is used in the art to denote a bivalent homodimeric scFv derivative. It consists of a fusion protein which contains the CH3 region of an immunoglobulin, preferably IgG, most preferably IgG1, as dimerisation region. This connects the scFv fragments by means 25 of a hinge region, also of IgG, and a linker region. Examples of such minibodies are known in the art. Some minibodies have an activity greater than that of a whole antibody (Orita et al., Blood (2005) 105:562-566).

The term "nanobody", as used herein, designates small sized entities (15 kDa) formed solely by the antigen binding region of the heavy chain (VH fragment) of immunoglobulins. Said nanobodies are mainly produced after immunizing animals of the Camelidae family, such as camels, llamas and dromedaries, mainly llamas; and also, of the shark family, which have the particularity of having antibodies which naturally lack the light chain and recognize the antigen by the heavy chain variable domain. Nevertheless, the nanobodies derived from these sources require a humanization process for their therapeutic application. Another potential source for obtaining nanobodies is from antibodies derived from different human samples by separating the VH and VL domains of the variable region. Nanobodies present advantages such as a production cost reduction with respect to whole antibodies, stability and the reduction of immunogenicity

The term "diabody", as used herein, refers to bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (Holliger, P., et al. (1993) Proc. Natl. Acad. Sci. USA 90:6444-6448; Poljak, RJ., et al. (1994) Structure 2:1121-1123).

The term "bispecific antibody" refers to that antibody construct having two different binding specificities, see. e.g., U.S. Patents. 5,922,845 and 5,837,243; Zeilder (1999) J. Immunol. 163:1246-1252; Somasundaram (1999) Hum. Antibodies 9:47-54; Keler (1997) Cancer Res. 57:4008-4014. For example, a bispecific antibody may have one binding site for a cell surface antigen, and a second binding site for an Fc receptor on the surface of an effector cell. The exemplary bispecific antibodies may bind to two different epitopes of gp130. Others of the said antibodies can bind to a first epitope of gp130 and additionally bind to a second epitope of gp130. Alternatively, a binding arm of an anti-tumor cell marker can be combined with an arm which binds to a triggering molecule in a leukocyte, such as a T-cell receptor molecule (for example, CD2 or CD3), or Fc receptors for IgG (FcyR), such as FcyRI (CD64), FcyRII (CD32) and FcyRIll (CD16), such that the mechanisms of cell defense are concentrated in the tumor cell. Bispecific antibodies can also be used to locate cytotoxic agents against the tumor cell. These antibodies have a binding arm to the marker of the lymphocyte and an arm which binds to the cytotoxic agent (for example, saporin, anti-interferon-α, vinca alkaloid, ricin A-chain, methotrexate or a radioactive hapten isotope). Bispecific antibodies can be prepared as whole antibodies or as antibody fragments (for example, F(ab)₂ bispecific antibodies, minibodies or diabodies). Techniques for generating bispecific antibodies from antibody fragments have also been described in the literature. For example, bispecific antibodies can be prepared using chemical linkage.

In an embodiment of the antibody of the invention, of the antigen-binding fragment of the invention or of the antibody construct of the invention, the VH and VL regions form a single polypeptide chain.

In yet another particular embodiment, wherein the antibody of the invention, the antigen-binding fragment of the invention or the antibody construct of the invention form part of a single polypeptide, the VH and VL regions are connected by a linker region.

The term "linker region", "linker", "flexible linker" or "flexible polypeptide linker" refers to a peptide linker that consists of amino acids such as glycine and/or serine residues used alone or in combination, to link variable heavy and variable light chain regions together; or to link any or the regions of the antibody construct.

The linker peptide may have any of a variety of amino acid sequences. Proteins can be joined by a spacer peptide, generally of a flexible nature, although other chemical linkages are not excluded. A linker can be a peptide of between about 6 and about 40 amino acids in length, or between about 6 and about 25 amino acids in length. These linkers can be produced by using synthetic, linker-encoding oligonucleotides to couple the proteins. Peptide linkers with a degree of flexibility can be used. The linking peptides may have virtually any amino acid sequence, bearing in mind that suitable linkers will have a sequence that results in a generally flexible peptide. The use of small amino acids, such as glycine and alanine, are of use in creating a flexible peptide. The creation of such sequences is routine to those of skill in the art.

Suitable linkers can be readily selected and can be of any of a suitable of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids.

Exemplary flexible linkers include the linker having the sequence TGSTSGSGKPGSGEGS (SEQ ID NO: 17). Suitable linkers include as glycine polymers (G) n, glycine-serine polymers (including, for example, (GS)ₙ, (GSGGS)ₙ (SEQ ID NO: 18) and (GGGS)ₙ (SEQ ID NO: 19), where n is an integer of at least one), glycine-alanine polymers, alanine-serine polymers, and other flexible linkers known in the art. In a particular embodiment the linker comprises a glycine polymer of formula (G₄S)₃. Glycine and glycine-serine polymers are of interest since both of these amino acids are relatively unstructured, and therefore may serve as a neutral tether between components. Glycine polymers are of particular interest since glycine accesses significantly more phi-psi space than even alanine, and is much less restricted than residues with longer side chains. Exemplary flexible linkers include, but are not limited to GGSG (SEQ ID NO: 20), GGSGG (SEQ ID NO: 21), GSGSG (SEQ ID NO: 22), GSGGG (SEQ ID NO: 23), GGGSG (SEQ ID NO: 24), GSSSG (SEQ ID NO: 25), and the like. The ordinarily skilled artisan will recognize that design of a peptide conjugated to any elements described above can include linkers that are all or partially flexible, such that the linker can include a flexible linker as well as one or more portions that confer less flexible structure.

In a particular embodiment, the linker is located between the VH and the VL regions of the antibody construct. In an embodiment, the antibody construct comprises the structure VL-linker-VH. In another embodiment, the antibody construct may have the structure VH-linker-VL or VL-linker-VH. In a particular embodiment, the linker is located C-terminally with respect to the VL region and N-terminally with respect to the VH region, that is, VL-linker-VH.

Functional fragments of antibodies or antibody constructs which bind to gp130 included within the present invention retain at least one binding function and/or modulation function of the full-length antibody from which they are derived. Preferred functional fragments retain an antigen-binding function of a corresponding full-length antibody (e.g., the ability to bind a mammalian gp130).

According to a different approach, antigen-binding fragments or antibody constructs with the desired binding specificities (antibody-antigen combining sites) may be fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are cotransfected into a suitable host organism. This provides great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

As used herein a "recombinant antibody" is an antibody that comprises an amino acid sequence derived from two different species or, from two different sources, and includes synthetic molecules, for example, an antibody that comprises a non-human CDR and a human framework or constant region. In certain embodiments, recombinant antibodies of the present invention are produced from a recombinant DNA molecule or synthesized.

The person skilled in the art will understand that the amino acid sequences of the antibody of the invention can include one or more amino acid substitutions such that, even though the primary sequence of the polypeptide is altered, the capacity of the antibody to bind to gp130 antigen is maintained. Said substitution can be a conservative substitution and is generally applied to indicate that the substitution of one amino acid with another amino acid with similar properties (for example, the substitution of glutamic acid (negatively charged amino acid) with aspartic acid would be a conservative amino acid substitution).

Amino acid sequence modification(s) of the antibody of the invention described herein in positions other than the CDRs or the binding sites are also contemplated. For example, it may be desirable to improve the binding affinity and/or other biological properties of the binding agent. Amino acid sequence variants of the binding agent are prepared by introducing appropriate nucleotide changes into the antibody encoding nucleic acid, or by peptide synthesis. Such modifications include, for example, deletions from, and/or insertions into and/or substitutions of, residues within the amino acid sequences of the binding agent. Any combination of deletion, insertion, and/or substitution is made, provided that the final binding agent possesses the desired characteristics. The amino acid changes may also alter post-translational processes of the protein, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

Another type of variant is an amino acid substitution variant. These variants have at least one amino acid residue in the molecule replaced by a different residue. The sites of greatest interest for substitution mutagenesis of antibodies include the hypervariable regions.

In a particular embodiment of the antibody of the invention, another type of amino acid variant of the antibody alters its original glycosylation pattern. By altering is meant deleting one or more carbohydrate moieties found in the molecule, and/or adding one or more glycosylation sites that are not present in it. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of any of these tripeptide sequences in a polypeptide creates a potential glycosylation site. O-linked glycosylation refers to the attachment of one of the monosaccharides or monosaccharide derivatives N-acetylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites). Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a nonvariant version of the antibody.

Also, it may be desirable to modify the antibody of the invention in order to improve their effector function, e.g. so as to enhance ADCC and/or CDC of the antibody. This may be achieved by introducing one or more amino acid substitutions in an Fc region of an antibody. Glycosyl groups added to the amino acid backbone of glycoproteins e.g. antibodies are formed by several monosaccharides or monosaccharide derivatives in resulting in a composition which can be different in the same antibody produced in cell from different mammals or tissues. In addition, it has been shown that different composition of glycosyl groups can affect the potency in mediating antigen-dependent cell-mediated cytotoxicity (ADCC) and/or complement dependent cytotoxicity (CDC) of the antibody. Therefore it is possible to improve those properties by mean of studying the pattern of glycosilation of antibodies from different sources.

Other modifications suitable for the antibody of the invention include the introduction of cysteine residue(s) in the Fc region, thereby allowing interchain disulfide bond formation in this region to improve the internalisation capability and/or increase complement-mediated cell killing and antibody-dependent cellular cytotoxicity (ADCC).

In order to increase the serum half-life of the antibody of the invention, one may incorporate a salvage receptor binding epitope into the antibody of the invention. As used herein, the term "salvage receptor binding epitope" refers to an epitope of the Fc region of an IgG molecule (e.g., IgG1, IgG2, IgG3, or IgG4) that is responsible for increasing the *in vivo* serum half-life of the IgG molecule.

### Nucleic acids, gene constructs, expression cassettes, vectors and cells of the invention

The antibody of the invention can be obtained by the use of nucleic acids which encode said antibody.

Thus, in a second aspect, the present invention relates to a nucleic acid, hereinafter referred to as "the nucleic acid of the invention", encoding the antibody of the invention.

The term "antibody" and its particulars have been described in detail the context of the previous aspect of the invention and apply equally to these aspects of the invention and their embodiments.

The terms "nucleic acid", "polynucleotide", "nucleotide sequence" and "nucleic acid sequence" are used interchangeably herein, and relate to any polymeric form of nucleotides of any length and composed of ribonucleotides or deoxyribonucleotides, e.g., deoxyribonucleic acids (DNA) or ribonucleic acids (RNA) and their polymers in either single or double stranded form. The terms include both single-stranded and double-stranded polynucleotides, as well as modified polynucleotides (e.g., methylated, protected). Unless specifically limited, the term encompasses nucleic acids containing known analogs of natural nucleotides that have binding capabilities similar to those of a reference nucleic acid and which are metabolized similarly to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues. Typically, the nucleic acid is a "coding sequence" which, as used herein, refers to a DNA sequence that is transcribed and translated into a polypeptide in a host cell when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A transcription termination sequence will usually be located 3' to the coding sequence. Exemplary nucleic acids or polynucleotides of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, a-LNA having an a-L- ribo configuration (a diastereomer of LNA), 2'-amino-LNA having a 2 '-amino functionalization, and 2'-amino- a-LNA having a 2'-amino functionalization), ethylene nucleic acids (ENA), cyclohexenyl nucleic acids (CeNA) or hybrids or combinations thereof.

In different embodiments of the invention, the nucleic acid of the invention is selected from the group consisting of:
(i) a nucleic acid construct comprising a first region encoding the VH region and a second region encoding the VL region wherein the first and second regions are independent transcriptional units,
(ii) a nucleic acid construct comprising a first region encoding the VH region and a second region encoding the VL region wherein said first and second regions form a single transcriptional unit and the VH and VL regions appear as independent open reading frames and wherein, optionally, both independent open reading frames are separated by an IRES,
(iii) a nucleic acid construct comprising a first region encoding the VH region and a second region encoding the VL region wherein said first and second regions form a single open reading frame and wherein the VH and VL regions are separated by a self-cleaving peptide region.

In preferred embodiments:
- If the nucleic acid construct comprises a first region encoding the VH region and a second region encoding the VL region wherein the first and second regions are independent transcriptional units, then each transcriptional unit further comprises a sequence encoding a signal sequence which is fused to the regions encoding the VH and VL regions in the same open reading frame,
- If the nucleic acid construct comprises a first region encoding the VH region and a second region encoding the VL region wherein said first and second regions form a single transcriptional unit and the regions encoding the VH and VL regions appear as independent open reading frames and wherein, optionally, both independent open reading frames are separated by an IRES, then each of the regions encoding the VH and VL regions contain an additional region encoding a signal sequence which forms a single open reading frame with the VH or the VL region,
- If the nucleic acid construct comprises a first region encoding the VH region and a second region encoding the VL region wherein said first and second regions form a single open reading frame, then the construct contains a region encoding a signal sequence within the same open reading frame as the open reading frame comprising the VH and VL region.

The term "nucleic acid construct" as used herein refers to one single nucleotide sequence which comprises all the elements required to encode the VH and VL regions, i.e., a first region encoding the VH region and a second region encoding the VL region.

The term "signal sequence", also known as "leader peptide", is used herein according to its ordinary meaning in the art and refers to a peptide having a length of about 5-30 amino acids. A leader peptide is present at the N-terminus of newly synthesized proteins that form part of the secretory pathway. Proteins of the secretory pathway include, but are not limited to proteins that reside either inside certain organelles (the endoplasmic reticulum, Golgi or endosomes), are secreted from the cell, or are inserted into a cellular membrane. In some embodiments, the leader peptide forms part of the transmembrane domain of a protein.

In a particular embodiment the signal which is encoded by the nucleic acids according to the invention is selected form the group comprising the CD8 signal sequence and the IgK signal sequence.

It will be evident to the expert in the field that the nucleic acid construct of the invention, which comprises two regions encoding each a different polypeptide (e.g, the VH sequence or the VL sequence) allows two separate polypeptides to be obtained. Several mechanisms are available to produce two or more polypeptides from the same nucleic acid construct. One such way is the existence in said nucleic acid of different transcription promoting elements, e.g., promoters and enhancers, for each of the different coding regions (see aspects and embodiments further below). Another way is the existence of single promoting elements which originate a unique messenger RNA (mRNA) which encodes for both polypeptides. In said case the nucleic acid can be monocistronic, i.e., both regions are contained in the same open reading frame of the mRNA, or bicistronic wherein the mRNA contains two different opening reading frames, one encoding for the first polypeptide and the other encoding for the second polypeptide.

In a particular embodiment, the nucleic acid construct of the invention is a monocistronic construct wherein the region encoding the first polypeptide and the region encoding the second polypeptide are found in the same open-reading frame and are separated by a self-cleaving peptide region.

As used herein, the term "self-cleaving peptide region" means a peptide sequence having a cleavage activity that occurs between two amino acid residues in the peptide sequence itself. Examples of the self-cleaving peptide include a 2A peptide and a 2A-like peptide. For example, in 2A or 2A-like peptides, cleavage occurs between glycine and proline residues on these peptides. This is caused by the "ribosome skipping mechanism", which prevents the formation of normal peptide bonds between glycine residues and proline residues during translation, and does not affect downstream translation. The ribosome skip mechanism is known in the art and is used for the expression of multiple proteins encoded by a single molecule of mRNA. The self-cleaving peptide used in the present invention can be obtained from a viral 2A peptide or a 2A-like peptide having an equivalent function. Examples of 2A self-cleaving peptides include 2A peptides (F2A) derived from foot-and-mouth disease virus (FMDV) (VKQTLNFDLLKLAGDVESNPGP (SEQ ID NO: 26)), 2A peptides (E2A) derived from horse rhinitis A virus (ERAV) (QCTNYALLKLAGDVESNPGP (SEQ ID NO: 27)), 2A peptide (P2A) derived from *Porcine teschovirus* (PTV-1) (ATNFSLLKQAGDVEENPGP (SEQ ID NO: 28)), and 2A peptides (T2A) derived from *Thosea signa* virus (TaV) (EGRGSLLTCGDVEENPGP (SEQ ID NO: 29)). Mutations may be appropriately introduced into the self-cleaving peptide domain as long as its activity is not significantly impaired.

In a particular embodiment the nucleic acid construct of the invention is a monocistronic construct wherein the region encoding the VH region and the region encoding the VL region are found in the same open-reading frame and are separated by a cis acting hydrolase element.

A "cis-acting hydrolase element" or "CHYSEL" refers to a peptide sequence that causes a ribosome to release the growing polypeptide chain that it is being synthesized without dissociation from the mRNA. In this respect, the ribosome continues translating and therefore produces a second polypeptide. Peptides such as the FMDV 2A sequence (GSGSRVTELLYRMKRAETYC PRPLLAIHPTEARHKQKIVAPVKQLLNFDLLKLAGDVESNPGP, SEQ ID NO: 30), Sponge *(Amphimedon queenslandica)* 2A sequence (LLCFLLLLLSGDVELNPGP, SEQ ID NO: 31; or HHFMFLLLLLAGDIELNPGP, SEQ ID NO: 32); acorn worm (*Saccoglossus kowalevskii*) (WFLVLLSFILSGDIEVNPGP, SEQ ID NO: 33) 2A sequence; amphioxus (*Branchiostoma floridae*) (KNCAMYMLLLSGDVETNPGP, SEQ ID NO: 34; or MVISQLMLKLAGDVEENPGP, SEQ ID NO: 35) 2A sequence *Porcine teschovirus-1* (GSGATNFSLLKQAGDVEENPGP, SEQ ID NO: 36) 2A sequence; Thoseaa *signa* virus (GSGEGRGSLLTCGDVEENPGP, SEQ ID NO: 37) 2A sequence; and *Equine rhinitis* A virus (GSGQCTNYALLKLAGDVESNPGP, SEQ ID NO: 38) 2A sequence are CHYSELs of use in this invention. In some embodiments, the 2A sequence is a naturally occurring or synthetic sequence that includes the 2A consensus sequence D-X-E-X-NPGP (SEQ ID NO: 39), in which X is any amino acid residue.

In a particular embodiment the nucleic acid construct of the invention is a multicistronic construct wherein the first region and the second region are found in different open-reading frames and are separated by an internal ribosome entry site or a cis-acting hydrolase element.

The term "internal ribosome entry site" or "IRES" defines a sequence motif that promotes attachment of ribosomes to that motif on internal mRNA sequences. Consequently, an mRNA containing an IRES sequence motif results in two translational products, one initiating from the 5'-end of the mRNA and the other by an internal translation mechanism mediated by the IRES. A number of IRES have been described and can be used in the nucleic acid construct of this invention. See, e.g., US 8,192,984; WO 2010/119257; and US 2005/0112095.

In some cases, the nucleic acid of the invention provides for production of the antibody of the invention, e.g., in a mammalian cell. In other cases, the nucleic acid of the invention provides for amplification of the antibody-encoding nucleic acid.

The nucleic acid of the invention can contain a regulatory sequence operatively linked for the expression of the nucleotide sequence encoding the antibody of the invention, thereby forming a gene construct. Thus, in a third aspect, the invention relates to a gene construct, hereinafter referred to as "the gene construct of the invention", comprising the nucleic acid of the invention.

The term "gene construct" or "nucleic acid construct" as used herein relates to a functional unit required to transfer, and preferably express, a gene or genes of interest in a host cell. This term refers to a nucleic acid molecule, either single- or double-stranded, which is modified to contain segments of nucleic acids in a manner that would not otherwise exist in nature.

As used herein, the term "operatively linked" means that the antibody of the invention encoded by the nucleic acid of the invention is expressed in the correct reading frame under control of the expression control or regulating sequences.

Therefore, the third aspect of the invention also provides an expression cassette, hereinafter "the expression cassette of the invention", comprising the nucleic acid of the invention. In an embodiment, the nucleic acid of the invention is operatively linked to an expression control sequence. In another embodiment, the expression cassette of the invention comprises the gene construct of the invention. In a particular embodiment, the gene construct of the invention is operatively linked to an expression control sequence. The gene construct of the invention can be obtained through the use of techniques widely known in the prior art (Sambrook et al., 2001 "Molecular cloning: to Laboratory Manual", 3a ed., Cold Spring Harbor Laboratory Press, N.Y., Vol. 1-3).

Control sequences are sequences that control and regulate transcription and, where appropriate, the translation of said antibody, and include promoter sequences, transcriptional regulators encoding sequences, ribosome binding sequences (RBS) and/or transcription terminating sequences. The expression cassette of the invention may additionally include an enhancer, which may be adjacent to or distant from the promoter sequence and can function to increase transcription from the same. In a particular embodiment, said expression control sequence is functional in prokaryotic cells and organisms, such as bacteria, etc. Whereas in another particular embodiment, said expression control sequence is functional in eukaryotic cells and organisms, for example, insect cells, plant cells, mammalian cells, etc.

Any available promoter can be used in this methodology. Suitable promoter and enhancer elements are known in the art. For expression in a bacterial cell, suitable promoters include, but are not limited to, lacl, lacZ, T3, T7, gpt, lambda P and trc. For expression in a eukaryotic cell, suitable promoters include, but are not limited to, light and/or heavy chain immunoglobulin gene promoter and enhancer elements; cytomegalovirus immediate early promoter; herpes simplex virus thymidine kinase promoter; early and late SV40 promoters; promoter present in long terminal repeats from a retrovirus; mouse metallothionein-I promoter; and various art-known tissue specific promoters. In a preferred embodiment of the present invention, the promoter used in the gene construct of the present invention is active in the specific cell population to be transfected. Illustrative, non-limiting examples of ubiquitous promoters which can be present in the expression cassette of the invention include the human cytomegalovirus promoter (hCMV), SV40 promoter, the EF1-alpha promoter to, and the ubiquitin promoter C. Illustrative, non-limiting examples of cell-type specific promoters and/or tissue specific promoters such as albumin include which is specific for liver, lymphoid-specific promoters, and so on.

Suitable reversible promoters, including reversible inducible promoters are known in the art. Such reversible promoters may be isolated and derived from many organisms, e.g., eukaryotes and prokaryotes. Modification of reversible promoters derived from a first organism for use in a second organism, e.g., a first prokaryote and a second a eukaryote, a first eukaryote and a second a prokaryote, etc., is well known in the art. Such reversible promoters, and systems based on such reversible promoters but also comprising additional control proteins, include, but are not limited to, alcohol regulated promoters (e.g., alcohol dehydrogenase I (alcA) gene promoter, promoters responsive to alcohol transactivator proteins (AlcR), etc.), tetracycline regulated promoters, (e.g., promoter systems including TetActivators, TetON, TetOFF, etc.), steroid regulated promoters (e.g., rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g., metallothionein promoter systems, etc.), pathogenesis-related regulated promoters (e.g., salicylic acid regulated promoters, ethylene regulated promoters, benzothiadiazole regulated promoters, etc.), temperature regulated promoters (e.g., heat shock inducible promoters (e.g., HSP-70, HSP-90, soybean heat shock promoter, etc.), light regulated promoters, synthetic inducible promoters, and the like.

In some instances, the locus or construct or transgene containing the suitable promoter is irreversibly switched through the induction of an inducible system. Suitable systems for induction of an irreversible switch are well known in the art, e.g., induction of an irreversible switch may make use of a Cre-lox-mediated recombination. Any suitable combination of recombinase, endonuclease, ligase, recombination sites, etc. known to the art may be used in generating an irreversibly switchable promoter. Methods, mechanisms, and requirements for performing site-specific recombination, described elsewhere herein, find use in generating irreversibly switched promoters and are well known in the art.

In some cases, the promoter is a CD8 cell-specific promoter, a CD4 cell-specific promoter, a neutrophil-specific promoter, or an NK-specific promoter. For example, a CD4 gene promoter can be used. As another example, a CD8 gene promoter can be used. NK cell-specific expression can be achieved by use of an Ncr1 (p46) promoter; see, e.g., Eckelhart et al. (2011) Blood 117:1565.

In some embodiments, e.g., for expression in a yeast cell, a suitable promoter is a constitutive promoter such as an ADH1 promoter, a PGK1 promoter, an ENO promoter, a PYK1 promoter and the like; or a regulatable promoter such as a GAL1 promoter, a GAL10 promoter, an ADH2 promoter, a PHO5 promoter, a CUP1 promoter, a GAL7 promoter, a MET25 promoter, a MET3 promoter, a CYC1 promoter, a HIS3 promoter, an ADH1 promoter, a PGK promoter, a GAPDH promoter, an ADC1 promoter, a TRP1 promoter, a URA3 promoter, a LEU2 promoter, an ENO promoter, a TP1 promoter, and AOX1 (e.g., for use in Pichia). Selection of the appropriate vector and promoter is well within the level of ordinary skill in the art.

Suitable promoters for use in prokaryotic host cells include, but are not limited to, a bacteriophage T7 RNA polymerase promoter; a trp promoter; a lac operon promoter; a hybrid promoter, e.g., a lac/tac hybrid promoter, a tac/trc hybrid promoter, a trp/lac promoter, a T7/lac promoter; a trc promoter; a tac promoter, and the like; an araBAD promoter; *in vivo* regulated promoters, such as an ssaG promoter or a related promoter, a pagC promoter, a nirB promoter, and the like; a sigma70 promoter, e.g., a consensus sigma70 promoter; a stationary phase promoter, e.g., a dps promoter, an spv promoter, and the like; a promoter derived from the pathogenicity island SPI-2; an actA promoter; an rpsM promoter; a tet promoter; an SP6 promoter; and the like. Suitable strong promoters for use in prokaryotes such as Escherichia coli include, but are not limited to Trc, Tac, T5, T7, and P Lambda. Non-limiting examples of operators for use in bacterial host cells include a lactose promoter operator (Lacl repressor protein changes conformation when contacted with lactose, thereby preventing the Lacl repressor protein from binding to the operator), a tryptophan promoter operator (when complexed with tryptophan, TrpR repressor protein has a conformation that binds the operator; in the absence of tryptophan, the TrpR repressor protein has a conformation that does not bind to the operator), and a tac promoter operator.

Any of the polynucleotides described above may further include additional nucleic acids, encoding, e.g. a signal peptide to direct secretion of the encoded antibody constant regions as described herein. Also, as described in more detail elsewhere herein, the present invention includes compositions comprising one or more of the polynucleotides described above.

In one embodiment, the invention includes compositions comprising a first polynucleotide and second polynucleotide wherein said first polynucleotide encodes a VH domain as described herein and wherein said second polynucleotide encodes a VL domain as described herein.

The present invention also includes fragments of the polynucleotides of the invention. Additionally, polynucleotides that encode fusion polypeptides, Fab fragments, and other derivatives, as described herein, are also contemplated by the invention.

The polynucleotides may be produced or manufactured by any method known in the art. For example, if the nucleotide sequence of the antibody is known, a polynucleotide encoding the antibody may be assembled from chemically synthesized oligonucleotides (e.g., as described in Kutmeier et al., Bio Techniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding the antibody of the invention, may be generated from nucleic acid from a suitable source. If a clone containing a nucleic acid encoding a particular antibody is not available, but the sequence of the antibody molecule is known, a nucleic acid encoding the antibody may be chemically synthesized or obtained from a suitable source (e.g., an antibody cDNA library, or a cDNA library generated from, or nucleic acid, preferably poly A+RNA, isolated from, any tissue or cells expressing the antibody or other anti-gp130 antibody, such as hybridoma cells selected to express an antibody by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody of the invention is determined, its nucleotide sequence may be manipulated using methods well known in the art for the manipulation of nucleotide sequences, e.g., recombinant DNA techniques, site directed mutagenesis, PCR, etc. (see, for example, the techniques described in Sambrook et al. (1990) Molecular Cloning, A Laboratory Manual (2nd ed.; Cold Spring Harbor Laboratory, Cold Spring Harbor, N.Y.) and Ausubel et al., eds. (1998) Current Protocols in Molecular Biology (John Wiley & Sons, NY), which are both incorporated by reference herein in their entireties), to generate antibodies having a different amino acid sequence, for example to create amino acid substitutions, deletions, and/or insertions.

A polynucleotide encoding the antibody of the invention can be composed of any polyribonucleotide or polydeoxyribonucleotide, which may be unmodified RNA or DNA or modified RNA or DNA. For example, a polynucleotide encoding the antibody of the invention can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, a polynucleotide encoding the antibody of the invention can be composed of triple-stranded regions comprising RNA or DNA or both RNA and DNA.

A polynucleotide encoding the antibody of the invention may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

An isolated polynucleotide encoding a non-natural variant of a polypeptide derived from an immunoglobulin (e.g., an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. Preferably, conservative amino acid substitutions are made at one or more non-essential amino acid residues.

In one embodiment, the polynucleotides have a modular design to encode at least the antibody of the invention. As a non- limiting example, the polynucleotide construct may encode any of the following designs: (1) the heavy chain of an antibody, (2) the light chain of an antibody, (3) the heavy and light chain of the antibody, (4) the heavy chain and light chain separated by a linker, (5) the VHI, CHI, CH2, CH3 domains, a linker and the light chain and (6) the VHI, CHI, CH2, CH3 domains, VL region, and the light chain. Any of these designs may also comprise optional linkers between any domain and/or region.

In a particular embodiment the nucleic acid of the invention encodes a Fv, Fab, F(ab')₂, Fab', a single domain antibody, a single chain variable fragment (scFv), a (scFv)₂, a scFv-Fc, a minibody, a nanobody, a diabody or a bispecific antibody.

Advantageously, the expression cassette of the invention further comprises a marker or gene encoding a motif or phenotype which allows selecting the transformed host cell with said expression cassette. Illustrative examples of said markers that could be present in the expression cassette of the invention include antibiotic resistance genes, genes for resistance to toxic compounds, and in general, all those that allow selecting the genetically transformed cells.

The nucleic acid of the invention, the gene construct of the invention or the expression cassette of the invention can be inserted into appropriate vectors. Thus, the third aspect of the invention also relates to a vector, hereinafter referred to as "the vector of the invention", comprising the nucleic acid of the invention. In some embodiments, the vector of the invention comprises the gene construct of the invention. In other embodiments, the vector of the invention comprises the expression cassette of the invention.

The term "vector" or "cloning vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs, which allow for random or site-directed integration of the targeting construct into genomic DNA. Such targeting constructs, preferably, comprise DNA of sufficient length for either homologous recombination or heterologous integration. In a particular embodiment, the vector is an expression vector. The term "expression vector" refers to a replicative DNA construct used for expressing the nucleic acid construct of the invention in a cell, preferably a eukaryotic cell, more preferably a mammalian cell. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

The choice of vector depends on the host cell in which it will be subsequently introduced. As an example, the vector into which is inserted the said nucleic acid sequences may be a plasmid or a vector which, when introduced into a host cell, is integrated or not in the genome of said cell. Obtaining this vector can be performed by conventional methods known to those skilled in the art (Sambrook et al. 2001, cited *supra*). In a particular embodiment, said recombinant vector is a vector useful to transfect animal cells.

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors (e.g. viral vectors based on vaccinia virus; poliovirus; adenovirus; adeno-associated virus; SV40; herpes simplex virus; human immunodeficiency virus); a retroviral vector (e.g., Murine Leukemia Virus, spleen necrosis virus), and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

As noted above, in some embodiments, a nucleic acid comprising the antibody of the invention will in some embodiments be RNA, e.g., *in vitro* synthesized RNA. Methods for *in vitro* synthesis of RNA are known in the art; any known method can be used to synthesize RNA comprising a nucleotide sequence encoding the antibody of the invention. Methods for introducing RNA into a host cell are known in the art. Introducing RNA comprising a nucleotide sequence encoding the antibody of the invention into a host cell can be carried out *in vitro* or *ex vivo* or *in vivo.* For example, a host cell (e.g., an NK cell, a cytotoxic T lymphocyte, etc.) can be electroporated *in vitro* or *ex vivo* with RNA comprising a nucleotide sequence encoding the antibody of the invention.

In order to assess the expression of the antibody thereof, the expression vector to be introduced into a cell can also contain either a selectable marker gene or a reporter gene or both to facilitate identification and selection of expressing cells from the population of cells sought to be transfected or infected through viral vectors; in other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Said vector can be used to transform, transfect, transduce or infect cells susceptible of being transformed, transfected, transduced or infected by said vector. Such cells can be prokaryotic or eukaryotic. Therefore, in a fourth aspect, the invention relates to a cell, hereinafter referred to as "the cell of the invention", comprising the nucleic acid of the invention, the gene construct of the invention, the expression cassette of the invention or the vector of the invention.

The term "cell" or "host cell", as used herein, refers to a cell into which a nucleic acid of the invention, such as the gene construct of the invention, expression cassette of the invention or vector of the invention, has been introduced and is capable of expressing the antibody of the invention. The terms "cell", "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal.

In order to obtain the cell of the invention, the cell may need to be transformed, transfected, transduced or infected with the nucleic acid of the invention, the gene construct of the invention, the expression cassette of the invention or the vector of the invention. Said transformed, transfected, transduced or infected cell comprises, therefore, the nucleic acid of the invention, the gene construct of the invention, the expression cassette of the invention or the vector of the invention. Transformed, transfected, transduced or infected cells may be obtained by conventional methods known to those skilled in the art (Sambrook et al. 2001, cited *supra*).

Cells suitable for performing the invention include, without limitation, mammalian, plant, insect, fungal and bacterial cells. Bacterial cells include, without limitation, cells from Gram positive bacteria such as species of the genus *Bacillus, Streptomyces* and *Staphylococcus* and Gram-negative bacterial cells such as cells of the genus *Escherichia* and *Pseudomonas.* Fungal cells preferably include yeast cells such as *Saccharomyces, Pichia pastoris* and *Hansenula polymorpha.* Insect cells include, without limitation, *Drosophila* cells and Sf9 cells. Plant cells include, among others, cells of crop plants such as cereals, medicinal, ornamental or bulbs. Mammalian cells suitable for the present invention include epithelial cell lines, osteosarcoma cell lines, neuroblastoma cell lines, epithelial carcinomas, glial cells, hepatic cell lines, CHO cells, COS cells, BHK cells, HeLa cells, 911 cells, AT1080 cells, A549 cells, HEK 293 and 293T cells, PER.C6 cells, NTERA-2 human ECCs cells, D3 cells of the mESCs line, human embryonic stem cells such as HS293, hMSCs and BGV01, SHEF1, SHEF2 and HS181, NIH3T3 cells, REH and MCF-7 cells.

In one embodiment, the mammalian cell comprises the antibody of the invention. The mammalian cell or tissue can be of human, primate, hamster, rabbit, rodent, cow, pig, sheep, horse, goat, dog or cat origin, but any other mammalian cell may be used. In a preferred embodiment of any aspect, the mammalian cell is human.

In some instances, the cell is not an immortalized cell line, but is instead a cell (e.g., a primary cell) obtained from an individual. For example, in some cases, the cell is an immune cell obtained from an individual.

In another embodiment, the engineered cells may be obtained from peripheral blood, cord blood, bone marrow, tumor infiltrating lymphocytes, lymph node tissue, or thymus tissue. The host cells may include placental cells, embryonic stem cells, induced pluripotent stem cells, or hematopoietic stem cells. The cells may be obtained from humans, monkeys, chimpanzees, dogs, cats, mice, rats, and transgenic species thereof. The cells may be obtained from established cell lines.

Host cells are transformed with the above-described expression or cloning vectors for anti-gp130 antibody production and cultured in conventional nutrient media modified as appropriate for inducing promoters, selecting transformants, or amplifying the genes encoding the desired sequences.

As such, additionally to all cell types described above, the term "cell of the invention", by extension, also includes hybridoma cells producing the antibody of the invention. The term "hybridoma", as used herein, refers to the hybrid cell line formed by fusing a specific antibody-producing B cell with a myeloma (B cell cancer) cell that is selected for its ability to grow in tissue culture and for an absence of antibody chain synthesis. The antibodies produced by the hybridoma are usually of a single specificity and are therefore monoclonal antibodies (in contrast to polyclonal antibodies).

All the terms and embodiments described in any of the previous aspects of the invention are equally applicable to the present aspects of the invention and their embodiments.

### Antibody-drug conjugates (ADCs) and detectable conjugates of the invention

In a further aspect, the antibody of the invention is conjugated to at least one agent. Said agent may be a therapeutic agent, such as, but not limited to, a radioisotope, radionuclide, toxin, toxoid, a polypeptide or chemotherapeutic agent. Said agent, may also be a detectable label. When the antibody of the invention is conjugated to a therapeutic agent, it is referred to as an antibody-drug conjugate (ADC). When the antibody of the invention is conjugated to a toxin, it is referred to as an immunotoxin. Both, ADCs and immunotoxins are included within the scope of the present invention. When administered to a patient, ADCs and immunotoxins bind to the target cell via their antibody portions and are internalized allowing drugs or toxins to exert their effects.

When the antibody of the invention is conjugated to a detectable label, it is referred to as a detectable conjugate.

Thus, in a fifth aspect, the present invention relates to a conjugate selected from the group consisting of:
(i) an antibody-drug conjugate (ADC) comprising a therapeutic agent and the antibody of the invention, hereinafter referred to as "the ADC of the invention"; and
(ii) a detectable label and the antibody of the invention, hereinafter referred to as "the detectable conjugate of the invention".

The term "antibody" and its particulars have been described in detail the context of the previous aspects of the invention and apply equally to this aspect of the invention and its embodiments.

### The ADC of the invention

The fifth aspect of the present invention relates to an antibody-drug conjugate (ADC) comprising a therapeutic agent and the antibody of the invention, referred to as "the ADC

### of the invention".

The term "therapeutic agent", "active compound" or "drug", as used herein, refers to a pharmacologically active substance or substances that are used to treat, or prevent diseases or conditions. Drugs act by altering the physiology of a living organism, tissue, cell, or *in vitro* system that they are exposed to. In a particular embodiment, the therapeutic agent is a cytotoxic agent.

As mentioned above, the term "antibody-drug conjugate" or "ADC" refers to antibodies, antigen-binding domains, or antibody constructs, such as the ones previously defined within the context of the antibody of the invention, also including antibody derivatives, that bind to gp130 and are conjugated to a drug such as a cytotoxic, cytostatic, and/or therapeutic agent.

Therefore, the ADC of the invention relates to the antibody of the invention recombinantly fused or chemically conjugated (covalent bonds and chemically) to heterologous agents to create fusion proteins. The heterologous agent is a polypeptide (or portion thereof, preferably a polypeptide of at least 10, at least 20, at least 30, at least 40, at least 50, at least 60, at least 70, at least 80, at least 90 or at least 100 amino acids), nucleic acid, small It may be a molecule (less than 1000 daltons) or an inorganic or organic compound. The fusion need not be direct and may occur through a linker sequence.

In an embodiment the antibody of the invention forming part of the ADC of the invention is humanized.

In another embodiment, the antibody of the invention forming part of the ADC of the invention comprises at least one humanized framework region.

In another embodiment, the VH region of the antibody of the ADC of the invention comprises at least one humanized FR region, at least 2 humanized FR regions, at least 3 humanized FR regions or at least 4 humanized FR regions, and wherein said FR regions are selected from SEQ ID NOs: 7, 8, 9 and 10.

In another embodiment, the VL region of the antibody of the ADC of the invention comprises at least one humanized FR region, at least 2 humanized FR regions, at least 3 humanized FR regions or at least 4 humanized FR regions, and wherein said FR regions are selected from SEQ ID NOs: 11, 12, 13 and 14.

In another embodiment, the antibody of the ADC of the invention is Fv, Fab, F(ab')₂, Fab', a single domain antibody, a single chain variable fragment (scFv), a (scFv)₂, a scFv-Fc, a minibody, a nanobody, a diabody or a bispecific antibody.

The above related terms have already been described within the context of the antibody of the invention and are equally applicable to the present aspect of the invention and its embodiments.

In a particular embodiment, the therapeutic agent is a cytotoxic agent.

The term "cytotoxic agent" as used herein refers to a substance that inhibits or prevents the function of cells and/or causes destruction of cells. The term is intended to include radioactive isotopes (e.g., At, I, Y, Re, Re, Sm, TBi, P, C, and radioactive isotopes of Lu), chemotherapeutic agents; and toxins such as small molecule toxins or enzymatically active toxins of bacterial, fungal, plant or animal origin, including synthetic analogs and derivatives thereof.

In another particular embodiment, the cytotoxic agent is a chemotherapeutic agent, an agent for targeted therapy or immunotherapy.

A "chemotherapeutic agent" is a chemical agent (e.g., compound or drug) useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors.

"Targeted cancer therapies" are drugs or other substances that block the growth and spread of cancer by interfering with specific molecules ("molecular targets") that are involved in the growth, progression, and spread of cancer. Targeted cancer therapies are sometimes called "molecularly targeted drugs", "molecularly targeted therapies", "precision medicines", or similar names. Many different targeted therapies have been approved for use in cancer treatment. These therapies include hormone therapies, signal transduction inhibitors, gene expression modulators, apoptosis inducers, angiogenesis inhibitors, immunotherapies, and toxin delivery molecules.

Examples of targeted therapy and chemotherapeutic agents include, but are not limited to, Erlotinib (TARCEVA^{®}, Genentech/OSI Pharm.), Bortezomib (VELCADE^{®}, Millenium Pharm.), Fulvestrant (FASLODEX^{®}, Astrazeneca), Sutent (SU11248, Pfizer), Letrozole (FEMARA^{®}, Novartis), Imatinib mesylate (GLEEVEC^{®}, Novartis), PTK787/Z 222584 (Novartis), Oxaliplatin (Eloxatin^{®}, Sanofi), 5-FU (5-fluorouracil), Leucovorin, Rapamycin (Sirolimus, RAPAMUNE^{®}, Wyeth), Lapatinib (GSK572016, GlaxoSmithKline), Lonafarnib (SCH 66336), Sorafenib (BAY43-9006, Bayer Labs.), and Gefitinib (IRESSA^{®}, Astrazeneca), AG1478, AG1571 (SU 5271; Sugen), alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphorarnide and trimethylomelarnine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, -2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g calicheamicin, especially calicheamicin gammall and calicheamicin omegall (Angew Chem Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinoraysins, actinomycin, authramycin, azaserine, bleomycins, cactinomycin, carabicin, canninomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptpnigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as anunoglutetWmide, mitotane, trilostane; folic acid replenisher such as frolinic acid; aceglatone; aldophosphamide glycoside; aminolevulinic acid; eniluracil; amsacrine; bestrabucil; bisanrrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE^{®} doxetaxel (Rhone- Poulenc Rorer, Antony, France); chloranbucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeioda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY 1 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RI VISor^{®} (vorozole), FEMARA^{®} (letrozole; Novartis), and ARIMIDEX^{®} (anastrozole; AstraZeneca); (iii) antiandrogens such as flutamide, nilutamide, bicalutamide, leuprolide, and goserelin; as well as troxacitabine (a 1 ,3-dioxolane nucleoside cytosine analog); (iv) protein kinase inhibitors such as ME inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAX1 D^{®}; PROLEUKIN^{®} rIL-2; topoisomerase 1 inhibitors such as LURTOTECAN^{®}; ABARELIX^{®} rmRH; (ix) anti-angiogenic agents such as bevacizumab (AVASTIN^{®}, Genentech); and pharmaceutically acceptable salts, acids and derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX^{®}, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA^{™}; Astra Zeneca), Erlotinib HC1 (CP-358774, TARCEVA^{™}; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis (4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, the ADC cytotoxic agent of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In addition, powerful chemotherapeutic agents such as CC-1065 analogs, calikiamycin, maytansine, dolastatin 10 analogs, lysoxin, and palytoxin can be linked to ADCs using conditionally stable linkers to form potent immunoconjugates.

In other embodiments, the antibody of the invention is conjugated to a therapeutic agent or drug moiety that modifies a given biological response. The therapeutic agent or drug moiety is not limited to classic chemotherapeutic agents. For example, the drug moiety may be a protein or polypeptide that possesses the desired biological activity. Such proteins include, for example, toxins such as abrin, ricin A, Pseudomonas exotoxin, cholera toxin, or diphtheria toxin; proteins such as tumor necrosis factor, α-interferon, β-interferon, nerve growth factor, platelet-derived growth factor Tissue plasminogen activator, apoptotic agents such as TNF-α, TNF-β, AIMI, AIMII, Fas ligand, and VEGf, thrombotic or antiangiogenic agents such as angiostatin or endostatin; or biological response modifiers For example, lymphokines (e.g., interleukin-1 (IL-1), interleukin-2 (IL-2), interleukin-4 (IL-4), interleukin Kin-6 (IL-6), Interleukin-7 (IL-7), Interleukin-9 (IL-9), Interleukin-15 (IL-15), Interleukin-12 (IL-12), Granules Sphere macrophage colony stimulating factor (GM-CSF), and granulocyte colony stimulating factor (G-CSF)), or growth factors (e.g., growth hormone (GH)).

In another embodiment the cytotoxic agent is an agent for immunotherapy.

Cancer immunotherapy attempts to stimulate the immune system to reject and destroy tumors. Tumors have been found to be able to escape host immunity by manipulating the tumor microenvironment and driving immunosuppression, such that although the components necessary for mounting an effective anti-tumor immune response are present in patients with cancer, the host usually fails to arrest tumor progression. Numerous molecular and cellular mechanisms have been proposed in the past to explain this counterintuitive scenario, largely focusing on the "immune escape" of tumors and suggesting that tumors develop the capability to avoid tumor-specific immune responses generated by the host, or they altogether disable the host anti-tumor immunity. This process, referred to as "tumor-induced immune suppression" has been recognized in recent years and is being intensively investigated. It appears that tumors can interfere with all components of the immune system, affecting all stages of the anti-tumor immune response.

Molecular alterations that occur in tumor cells as the tumor progresses from the premalignant to metastatic phenotype are a result of genetic instability, now recognized as a principal characteristic of all tumors. Genetic changes are detectable during early stages of tumorigenesis, become more pronounced as the tumor progresses, are the greatest in metastatic cells and are responsible for tumor heterogeneity and alterations in the antigenic epitope profile of tumor cells. It has been suggested that the immune system might drive these antigenic changes via "immune editing" by eliminating those malignant cells that are sensitive to immune intervention and allowing for the selection and survival of immune resistant variants. The net result of immune editing is that the tumor escapes from the host immune system. Regulatory T cells (Treg) and myeloidderived suppressor cells (MDSC) are two types of cells used by the tumor to execute an immune escape. Protection of immune cells from the adverse effects of Treg, MDSC or inhibitory factors, and thus enhancement of their effector functions, can restore effective anti-tumor immunity in patients with cancer.

CD4⁺ CD25^{high} FOXP3⁺ T cells (Treg cells) accumulate in human tumors and in the peripheral circulation of patients with cancer. It is unclear whether these cells migrate to tumors or expand in situ. Because tumor-associated antigens (TAA) are self-antigens, it is possible that Treg accumulation is a response to enforce immune tolerance. Treg cells contribute to the down-regulation of immune activity of effector T cells by a variety of mechanisms including IL-10 and TGF- 1 production, enzymatic degradation of ATP to immunosuppressive adenosine, or the engagement of the Fas/FasL and granzyme/perforin pathways. Tumors benefit from immunosuppressive effects mediated by Treg cells.

Bone marrow-derived immature myeloid cells MDSC (CD34⁺CD33⁺CD13⁺CD15⁻) are present at an elevated frequency in the peripheral circulation and tumors of nearly all cancer patients. They are recruited by tumor-derived soluble factors such as TGF-31, IL-10, VEGF, GMCSF, IL-6, PGE2. They promote tumor growth by suppressing T-cell responses via several mechanisms, including production of arginase-1, an enzyme involved in L-arginine metabolism, as well as activation of inducible nitric oxide synthase (iNOS). They also control the tumor's production of indoleamine-2, 2 dioxygenase (IDO), which is involved in the catabolism of tryptophan, an amino acid essential for T-cell differentiation.

Bacillus Calmette-Guerin (BCG) immunotherapy for early stage (non-invasive) bladder cancer utilizes instillation of attenuated live bacteria into the bladder, and is effective in preventing recurrence in up to two thirds of cases. The immune response to BCG can be summarized as follows: infection of urothelial and bladder tumor cells by BCG results in internalization of BCG, which increases the expression of antigen-presenting molecules. This induces an immune response via the release of cytokines, such as Th1 cytokines (IL-2, tumor necrosis factor, IL-12, and IFN-γ) and Th2 cytokines (IL-4, IL-5, IL 6, and IL-10) along with IL-8 and IL-17. This complex immune cascade induces antitumor activity mediated by cytotoxic T lymphocytes, natural killer cells, neutrophils, and macrophages. In another study of BCG in an orthotopic mouse bladder tumor model, the population of MDSCs was significantly downregulated following the high-dose BCG therapy compared with the low-dose therapy. In the same study, CD4⁺/Foxp3⁺ Tregs also exhibited the same change. Following the BCG treatment, the population of CD4⁺/Foxp3⁺ Tregs was decreased in the blood. These inhibitory effects on the immune-suppressive factors may explain the robust antitumor therapeutic effects of the BCG therapy.

Immune checkpoint regulators, which can be both costimulatory and coinhibitory molecules, regulate the immune system. The balance between these checkpoints signals regulates lymphocyte activation and consequently the immune response. Tumors can use these checkpoint regulators to protect themselves from the immune system. Immune checkpoint therapies can enhance the proliferation, migration, persistence, and/or cytotoxic activity of T cells in a subject and, in particular, by increasing the numbers of tumor infiltrating T cells. The best characterized immune checkpoint receptors are cytotoxic T-lymphocyte-associated antigen 4 (CTLA-4; also known as CD152), programmed cell death protein 1 (PD-1; also known as CD279), and indoleamine 2,3-dioxygenase (IDO), and agents targeting these molecules are either approved or being extensively tested in clinical trials for treatment of multiple solid or hematological cancers.

CTLA-4, an inhibitory receptor, is a global immune checkpoint regulator engaged in priming immune responses via down-regulating the initial stages of T-cell activation. CTLA-4 was the first clinically validated checkpoint pathway target. CTLA-4 is homologous to the T-cell costimulatory protein CD28, and both molecules bind to CD80 and CD86 on antigen-presenting cells. CTLA-4 binds CD80 and CD86 with a markedly higher affinity and avidity than CD28 does, enabling CTLA-4 to outcompete CD28 for its ligands, resulting in an effective inhibition of T cell activation. Blocking CTLA-4 activity by using an antagonistic antibody interferes with this mechanism and thus preserves the activity of the T cells. Currently, Bristol-Myers Squibb's anti-CTLA-4 mAb, ipilimumab, has received US Food and Drug Administration (FDA) approval for patients with metastatic melanoma. Additionally, AstraZeneca's anti-CTLA-4 mAb, tremelimumab, has been granted orphan drug designation by the FDA for the treatment of patients with malignant mesothelioma.

PD-1 is another inhibitory receptor expressed on activated T and B cells, and it functions to dampen the immune response. PD-1 acts as an immune checkpoint regulator, and upon binding of one of its ligands, PD-L1 (B7-H1, CD274) or PD-L2 (B7 DC, CD273), PD-1 inhibits proliferation and cytokine production of T cells. Overexpression of PD-L1 or PD-L2 in the tumor microenvironment leads to the inhibition of the intratumoral immune responses. Inhibition of the interaction between PD-1 and PD-L1 by anti-PD-1/PD-L1 antibodies can inhibit the deactivation of T cells, and thus enhance anti-tumor responses, delay tumor growth, and facilitate tumor rejection. Two anti-PD-1 mAbs, Bristol-Myers Squibb's nivolumab, and Merck's pembrolizumab, have received US FDA approval for patients with metastatic melanoma and non-small-cell lung cancer. Recently, the FDA approved nivolumab as a treatment for patients with metastatic renal cell carcinoma. In 2016, Roche's anti-PD-Li mAb, atezolizumab, was approved by the FDA for treating metastatic urothelial cancer and non-small cell lung cancer. Other anti-PD-LI mAbs, such as AstraZeneca's durvalumab and Pfizer's avelumab, are in late-stage clinical trials.

Indoleamine 2,3-dioxygenase (IDO) is an enzyme that catalyzes the oxidative cleavage of tryptophan. IDO plays a role in suppressing the immune system because T cells undergoing antigen-dependent activation require tryptophan for cell proliferation and survival. IDO is overexpressed in most tumors and/or tumor-draining lymph nodes and it plays a significant role in helping tumors to evade attack from the immune system. IDO inhibitors block the IDO enzyme which reduces the depletion of tryptophan and ultimately may help to promote an enhanced immune response against the tumor. Currently, a number of clinical trials are underway to evaluate IDO inhibitors for both monotherapy and combination cancer therapies.

In addition to PD-1, CTLA-4, and IDO, other immune checkpoints are also involved in the occurrence and development of malignant tumors, including T cell membrane protein-3 (TIM-3), LAG3, T-cell immunoreceptor with Ig and immunoreceptor tyrosine-based inhibitory motif (ITIM) domains (TIGIT), BTLA, inducible T-cell costimulator (ICOS), killer inhibitory receptors (KIR), and V-domain Ig-containing suppressor of T cell activation (VISTA). Similar to PD-1, CTLA-4 and IDO, these immune checkpoints inhibit lymphocyte activity and/or induce lymphocyte anergy, and thus are ideal targets for cancer immunotherapy. Blocking antibodies for these immune checkpoints have shown specific anti-tumor activities in animal models, and some are being tested in clinical trials.

Thus, the cytotoxic agent can also be any immunotherapy agent that enhances the anti cancer effect of the antibodies anti-gp130, or an analogue or pharmaceutically acceptable salt thereof. In some embodiments, the cytotoxic agent is an immunologic agent that can stimulate an effective immune response and/or inhibit immune-suppression. In some embodiments, the cytotoxic agent is an agent that modulates, particularly inhibits and downregulates immune-suppressive factors such as regulatory T cells and MDSCs, including any inhibitors or antibodies of the programmed cell death ligand 1 (PD-L1)/ programmed cell death protein 1 (PD-1) pathway. In some embodiments, the cytotoxic agent are any effective inhibitors/antibodies capable of modulating immunocytes activities including but not limited to targeting cytotoxic T lymphocyte-associated antigen 4 (CTLA-4), CD20, CD19, IL-i7a, CD25, arginase I (ARG1), indoleamine-2,3-dioxygenase (IDO), or tryptophan 2,3 dioxygenase (TDO2). In some embodiments, the second inhibitor is BCG. In particular embodiments, the cytotoxic agent is an antibody against PD-1 or an antigen binding fragment thereof. In other particular embodiments, the cytotoxic agent is an antibody against PD-L1 or an antigen binding fragment thereof. In other particular embodiments, the cytotoxic agent is an antibody against CTLA-4 or an antigen binding fragment thereof. In other particular embodiments, the cytotoxic agent is a BCG therapy, preferably an attenuated BCG therapy, more preferably mycobacterial cell wall fragments, and most preferably mycobacterial cell wall fragments with biologically active nucleic acids derived from Mycobacterium phlei.

Techniques for conjugating therapeutic moieties to antibodies are well known. The therapeutic moiety may be conjugated to the antibody by any method known in the art including, but not limited to, aldehyde / Schiff bond, sulfhydryl bond, acid labile bond, cis-aconityl (Aconityl) bond, hydrazone bond, and enzyme-degradable bond. The fusion with the therapeutic portion of the antibody need not be direct, but may occur through a linker sequence. Such linker molecules are generally known in the art.

In some embodiments, the conjugation of the drug to the antibody is performed by a linker that is sensitive to the environment (e.g., the lysosomal environment due to the endosomal environment or pH or protease sensitivity).

In one embodiment, the linker is an acid labile hydrazone or hydrazide group that is hydrolyzed within the lysosome. In other embodiments, the drug can be conjugated to the antibody via other acid labile linkers such as cis-aconitic amides, orthoesters, acetals and ketals. Such linkers are relatively stable under neutral pH conditions, such as blood conditions, but are unstable at pH 5 or lower, i.e., at a pH close to lysosomes.

In other embodiments, the drug binds to the antibody of the invention using a peptide spacer that is cleaved by intracellular proteases. Target enzymes include cathepsins B and D and plasmin, all of which are known to hydrolyze dipeptide drug derivatives resulting in the release of active drugs inside the target cell

In certain non-limiting embodiments of the present invention, the linker region between the drug and antibody portions of the ADC can be cleaved under certain conditions, with linker cleavage or hydrolysis. The drug moiety is released from the antibody moiety. Preferably, this linker is sensitive to cleavage or hydrolysis under intracellular conditions.

In one embodiment, the linker region between the drug and antibody portions of the ADC can be hydrolyzed if the pH changes by a certain value or exceeds a certain value.

In yet another specific embodiment, the linker is a disulfide linker. Various disulfide linkers are known in the art, including but not limited to SATA (N-succinimidyl-S-acetylthioacetate), SPDP (N-succinimidyl-3- (2-pyridyldithio) propionate) , SPDB (N-succinimidyl-3-(2-pyridyldithio) butyrate) and SMPT (N-succinimidyl-oxycarbonyl-α-methyl-α- (2-pyridyldithio) toluene) Is included. SPDB and SMPT.

### The detectable conjugate of the invention

The fifth aspect of the present invention also relates to a detectable label conjugated to the antibody of the invention, hereinafter referred to as "the detectable conjugate of the invention".

In an embodiment, said detectable label can be detected by means of a change in at least one of its physical, chemical, electrical or magnetic properties

As mentioned above, the term "detectable conjugate" refers to antibodies, antigen-binding domains, or antibody constructs, such as the ones previously defined within the context of the antibody of the invention, also including antibody derivatives, that bind to gp130 and are conjugated to a detectable label.

The term "detectable label", "labelling agent", "detectable reagent" or "marker" as used herein, refers to a molecular label with capacity to be detected either directly or indirectly, thus, allowing the detection, localization or identification of the molecule to which it is attached using suitable procedures and equipment for detection, for example by means of enzymatic, radioactive, fluorescence, spectroscopic, photochemical, biochemical, immunochemical or chemical methods. Examples of detectable labels suitable for the invention include, without limitation, radionuclides, radioactively labelled compounds or a radioactive isotope, fluorophores, fluorescent or bioluminescent proteins, enzymes, chemiluminescent reagents, an enzymatic substrate or cofactor, an enzymatic inhibitor, a particle, a dye, derivatives, and the like, which are well-known in the art. This reagent may be detected by, for example, microscopy, fluorimetry or colorimetry using apparatus suitable for the type of reagents and sample type, which are known to the skilled artisan.

In a particular embodiment, the detectable label is a compound radioactively labeled by means of radioactive isotopes, also called radioisotopes or radionuclides. Non-limitative examples of radioisotopes suitable for the invention include ³H, ¹¹C, ¹⁴C, ¹⁸F, ³²P, ¹⁵N, ⁶⁴Cu, ⁶⁸Ga, ³⁵S, ⁸⁶Y, ⁹⁰Y, ⁹⁹Tc, ¹¹¹In, ¹²³I, ¹²⁴I, ¹²⁵I, ¹³¹I, ¹³³Xe, ¹¹¹Lu, ¹⁷⁷Lu, ²¹¹At, ⁸⁹Zr or ²¹³B. Radioisotope labelling is performed typically by using chelating ligands that are capable of complexing metal ions such as DOTA, DOTP, DOTMA, DTPA and TETA. Methods for conjugating radioisotopes to proteins are well known in the prior art.

In another particular embodiment, the detectable label is a fluorescent label. Fluorescent labels include fluorescent groups which can be attached to the side chains of the amino acids directly or through a linking group. Methods for conjugating polypeptides fluorescent reagents are well known in the prior art. Thus, in an embodiment, the detectable label is a fluorescent group.

The fluorescent labels suitable for the invention may include, without limitation, ethidium bromide, SYBR Green, phosphorus-lanthanides, fluorescein isothiocyanate (FITC), rhodamine tetramethyl isotiol (TRIT), 5-carboxyfluorescein, 6-carboxyfluorescein, fluorescein, HEX (6-carboxy-2',4,4',5',7,7'-hexachlorofluorescein), Oregon Green 488, Oregon Green 500, Oregon Green 514, Joe (6-carboxy-4',5'-dichloro-2',7'-dimethoxyfluorescein), 5-carboxy-2',4',5',7'-tetrachlorofluorescein,5-carboxyrhodamine, rhodamine, tetramethylrhodamine (Tamra), Rox (carboxy-X-rhodamine), R6G (rhodamine 6G), phthalocyanines, azometazinas, cyanines (Cy2, Cy3 and Cy5), Texas Red, Princeston Red, BODIPY FL-Br2, BODIPY 530/550, BODIPY TMR, BODIPY 558/568, BODIPY 564/570, BODIPY 576/589, BODIPY 581/591, BODIPY TR, BODIPY 630/650, BODIPY 650/665, DABCYL, eosin, erythrosin, ethidium bromide, green fluorescent protein (GFP) and its analogues, inorganic-based fluorescent semiconductor nanocrystals (Quantum Dot), fluorescent labels based onlanthanide such as Eu³⁺ and Sm³⁺ and the like.

The enzymatic labels may include, without limitation, horseradish peroxidase, β-galactosidase, luciferase or alkaline phosphatase.

In another particular embodiment, the antibody of the invention is labelled by conjugation to a first member of a binding pair, thus, the detectable label is a first member of a binding pair. In a preferred embodiment, this modification is covalent biotinylation. The term "biotinylation", as used herein, refers to the covalent attachment of biotin to a molecule (typically a protein). Biotinylation is performed using biotin reagents capable of conjugating to the side chain of the proteins, wherein said conjugation occurs primarily on the primary amino groups and thiol groups contained in the side chains of proteins. Suitable reagents for biotinylation of amino groups include molecules containing biotin and a group capable of reacting with amino groups such as succinimide esters, pentafluorophenyl ester or alkyl halides, wherein the biotin moiety and the reactive group separated by a spacer of any length (for example, 8-40 A in length). Some examples of these agents include agents biotinylation NHS -biotin (containing an ester bond of five carbon atoms between biotin and NHS group), sulfo-NHS-biotin, NHS-LC-biotin, sulfo-NHS-LC-biotin, NHS-LC-LC-biotin, sulfo-NHS-LC-LC-biotin, sulfo-NHS-SS-biotin, NHS-PEO4-biotin, PFP-biotin, TFP-PEO-biotin and the like, where "NHS" indicates an N-hydroxysuccinimide, "LC" refers to an amide bond of 6 carbon atoms located between NHS and biotin group, "PEO" refers to a etileneoxyde group, where the subscript indicates the number of units PEO, "PFP" refers to a pentafluorophenyl group "TFP" refers to a tetrafluorophenyl group, "sulfo" refers to a sulfonate group (SO3) and "SS" refers to a disulfide group. Examples of biotinylation reagents with thiol groups include molecules comprising a group of biotin and maleimide or alkyl halide type, separated by a spacer of any length. Examples of biotinylation reagents include maleimide-PEG-biotin, biotin-BMCC (containing a maleimido group N-terminal and a cyclohexyl group, 2 amide and 9 carbon atom linkers), PEO-iodoacetil biotin, iodoacetil-LC-biotin, biotin-HPDP (containing a pyridyl disulfide) and the like.

In another particular embodiment, the antibody of the invention is labelled with metal ions such as gold (Au) or iron (Fe), thus, the detectable label is a metal ion. Suitable metal ions include colloidal gold nanoparticles or ferromagnetic nanoparticles, which can be attached directly to the antibody via electrostatic interactions. In another particular embodiment, the colloidal gold nanoparticles or ferromagnetic nanoparticles are pre-coupled to biotin and can be covalently attached to the antibody.

The preferred labeling include, but are not limited to, radioisotopes, such as ⁸⁹Zr, fluorescein, a phosphatase such as alkaline phosphatase, biotin, avidin, a peroxidase such as horseradish peroxidase and compounds related to biotin or compounds related to avidin (for example, streptavidin or ImmunoPure^{®} NeutrAvidin available from Pierce, Rockford, IL).

The coupling of the antibody of the invention, the antigen-binding fragment thereof or the antibody construct to a therapeutic agent in order to obtain an ADC or to a detectable label in order to obtain a detectable conjugate is usually carried out using compounds which contain chemical groups which show ability to react with the various groups listed in the side chains of the proteins, including amino groups and thiol groups. Thus, chemical groups that can be used to modify the antibodies according to the present invention include, without limitation, maleimide, haloacetyl, iodoacetamide succinimidyl ester (e.g. NHS, N-hydroxysuccinimide), isothiocyanate, sulfonyl chloride, 2,6-dichlorotriazinyl, pentafluorophenyl ester, phosphoramidite and the like. An example of suitable reactive functional group is N -hydroxysuccinimide ester (NHS) of a detectable group modified with a carboxyl group. Typically , the carboxyl group modifying the therapeutic or the detectable compound is activated by the contacting of said compound with a carbodiimide reagent (for example, dicyclohexylcarbodiimide, diisopropylcarbodiimide, uranium or a reagent such as TSTU (0-(N-Succinimidyl)-N,N,N',N'-tetramethyluronium tetrafluoroborate), HBTU((0-benzotriazol- 1 -yl)-N,N,N',N'-tetramethyluronium hexafluorophosphate), or HATU (0-(7-azabenzotriazol-I-yl)-N,N,N',N'-tetramethyluronium hexafluorophosphate), an activator of the type of 1-hydroxybenzotriazole (HOBt) and N-hydroxysuccinimide to give the NHS ester of the label.

All the terms and embodiments described in any of the previous aspects of the invention are equally applicable to the present aspect of the invention and its embodiments.

### Detection and/or quantification methods

A sixth aspect of the present invention relates to an *in vitro* method for detecting and/or quantifying the presence of gp130 in a sample, hereinafter referred to as "the first method of the invention", comprising:
(a) contacting the antibody of the invention, or the detectable conjugate of the invention with the sample; and
(b) detecting and/or quantifying the level of gp130 in the sample by measuring the formation of immune complexes formed between the gp130 in the sample and the antibody of the invention or the detectable conjugate of the invention.

The terms and expressions "antibody", "detectable conjugate", "gp130" and their particulars have been described in detail the context of the previous aspects of the invention and apply equally to this aspect of the invention and its embodiments.

The term *"in vitro",* as used herein, refers to the fact that an experimental protocol or a method is not carried out in the body of a human or animal subject or patient, but in samples isolated from said subject, and already present in a laboratory tool, such as a test tube, dish or plate.

*"In vitro* method for detecting and/or quantifying the presence of gp130 in a sample", as used herein, relates to a method which allows the detection and/or quantification of gp130 in a sample.

As used herein, the term "detecting" or "detect" and derivations of this term refer to determining the presence, absence, amount, localization, distribution or organization of an analyte in a sample. According to the present invention, "detecting" also includes visualizing, determining, observing, evaluating, quantifying, measuring or imaging gp130. As used herein, the term "quantifying" or "quantify" is to be understood as to determine an absolute measure of an analyte in a sample, or determine the relative amount of an analyte in a sample with respect to some other substance(s) in the sample. In the present case, the analyte is gp130.

The term "sample", as used herein, relates to any sample which can be obtained from the subject or patient and which contains any biological material suitable for detecting DNA, RNA or protein levels. In a particular embodiment, the sample contains genetic material, e.g., DNA, genomic DNA (gDNA), complementary DNA (cDNA), RNA, heterogeneous nuclear RNA (hnRNA), mRNA, etc., from the patient under study. In another particular embodiment the sample contains proteins. The sample can comprise cell and/or non-cell material of the patient. The present method can be applied to any kind of biological sample from a patient, such as a biopsy sample, tissue, cell or biological fluid or biofluid (blood, plasma, serum, saliva, urine, semen, sputum, cerebrospinal fluid (CSF), tears, mucus, sweat, milk), feces, brain extracts, bone marrow, nipple aspirate, samples obtained by bronchial lavage, bronchoscopy, fine needle aspiration biopsy (FNAB), solid tumor biopsy sample, a buccal or buccal pharyngeal swab and the like. Said sample can be obtained by conventional methods, e.g., biopsy, surgical excision or aspiration, by using methods well known to those of ordinary skill in the related medical arts. Methods for obtaining the sample from the biopsy include gross apportioning of a mass, or microdissection or other art-known cell-separation methods. In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze. The samples can also be a cell suspension, cell pellet, cell slide, frozen solid tumor biopsy.

In an embodiment, the sample according to the first method of the invention is a tissue sample, preferably a tissue sample of a tumor, either fresh or frozen.

In a particular embodiment, the sample according to the first method of the invention is a solid tumor.

The term "solid tumor", as used herein, refers to a malignant solid tumor. Although the term includes various types of solid tumors named for the cell types that form them, namely sarcomas, carcinomas, and lymphomas, the term does not include leukemia. In various embodiments, the term "solid tumor" refers to cancers (called sarcomas) that arise from connective or supporting tissue (e.g., bone or muscle), cancers that arise from the body's glandular cells and the epithelial cells that line the body, cancers of lymphoid organs such as the lymph nodes, spleen, and thymus (called lymphomas). Lymphoid cells are present in almost all tissues, and therefore lymphomas can develop in a variety of organs. As used herein the term "solid tumor" includes, but is not limited to, colorectal cancer, ovarian cancer, prostate cancer, breast cancer, brain cancer, cervical cancer, bladder cancer, anal cancer, uterine cancer, colon cancer, liver cancer, pancreatic cancer, lung cancer, endometrial cancer, bone cancer, testicular cancer, skin cancer, kidney cancer, stomach cancer, esophageal cancer, head and neck cancer, salivary gland cancer, myeloma, hepatocellular carcinoma, non-small cell lung cancer, head and neck squamous cell carcinoma, basal cell carcinoma, cutaneous squamous cell carcinoma, chondrosarcoma, Includes cancers including angiosarcoma, cholangiocellular carcinoma, soft tissue sarcoma, melanoma, Merkel cell carcinoma, and glioblastoma multiforme.

In another particular embodiment, the sample according to the first method of the invention is a biofluid, preferably a biofluid from affected organs.

A "biofluid", "biological fluid sample" or "bodily fluid sample", as used herewith, refers to any biological secretion or fluid, whether physiological or pathological, which is produced in the body of a subject. Such biofluids include, without limitation, blood, plasma, serum, bronchoalveolar washing fluid, urine, nasal secretion, ear secretion, urethral secretion, cerebrospinal fluid, pleural fluid, synovial fluid, peritoneal fluid, ascites fluid, pericardial liquid, amniotic fluid, gastric juice, lymphatic fluid, interstitial fluid, saliva, sputum, liquid deposition, tears, mucus, sweat, milk, semen, vaginal secretions, fluid coming from ulcer, blisters, abscesses and other surface eruptions. Said samples can be obtained by conventional methods using processes known in the state of art by the person skilled in the art, such as blood extraction, instillation and aspiration of liquid during bronchofibroscopy, cisternal, ventricular or lumbar puncture, pleural puncture or thoracocentesis, joint or synovial percutaneous puncture, abdominal puncture, amniocentesis, expectoration, peritoneal percutaneous puncture, pericardial percutaneous puncture, etc., or by simple harvesting.

In a preferred embodiment, the biofluid according to the first method of the invention is selected from the group consisting of blood, plasma, serum and cerebrospinal fluid (CSF).

The term "subject" or "patient", as used herein, is understood as any animal classified as mammal and includes, without limitation, domestic and farm animals, primates and humans, e.g., human beings, non-human primates, cows, horses, pigs, sheep, goats, dogs, cats, or rodents. Preferably, the subject is a male or female human of any age or race.

In a first step [step (a)], the first method of the invention comprises contacting the antibody of the invention or the detectable conjugate of the invention with the sample.

The term "contacting", as used herein, refers to the process by which the antibody of the invention or the detectable conjugate of the invention comes into contact with the sample and includes any possible method that allows the introduction of the antibody of the invention or the detectable conjugate of the invention into the sample, to specifically bind to gp130.

The antibody of the invention or the detectable conjugate of the invention is applied to the sample in a suitable buffer to allow binding of the antibody of the invention or the detectable conjugate of the invention to gp130 that may be present in the sample. Non-limiting examples of suitable buffers to allow binding of the antibody of the invention or the the detectable conjugate of the invention include PBS, TBS, phosphate buffer and citrate buffer. The amount of the antibody of the invention or the detectable conjugate of the invention needed to detect gp130 present in the sample will depend on the size of said sample and the amount of gp130 present in said sample. This can be readily determined by optimisation procedures common in the art. As an indication, the concentration of the antibody of the invention or the detectable conjugate of the invention is at least 1 fM, at least 10 fM, at least 100 fM, at least 1 pM, at least 10 pM, at least 100 pM, at least 1 nM, at least 10 nM, at least 100 nM, at least 1 µM, at least 10 µM, at least 100 µM or more. Preferably, the concentration of the antibody of the invention or the detectable conjugate of the invention is between 100 fM and 1 µM, more preferably between 1 pM and 100 nM, most preferably between 100 pM and 1 nM.

The antibody of the invention or the detectable conjugate of the invention is incubated with the sample at a suitable temperature and for a time sufficient to allow binding of the antibody of the invention or the detectable conjugate of the invention to gp130 that may be present in the sample. The temperature is preferably between 20°C and 37°C, or may be overnight at about 4°C, or so. For example, the antibody of the invention or the detectable conjugate of the invention is incubated with the sample for at least 5 min, at least 10 minutes, at least 15 minutes at least 20 minutes, at least 30 minutes, at least 60 minutes, at least 120 min or more.

In a second step [step (b)], the first method of the invention comprises detecting and/or quantifying the level of gp130 in the sample by measuring the formation of immune complexes formed between gp130 in the sample and the antibody of the invention or the detectable conjugate of the invention in the sample.

The term "immune complex" or "antigen-antibody complex", as used herein, describes the result from the interaction between an epitope (antigen) with an antibody directed against this epitope, i.e. the interaction between the antigen with the antibody of the invention or the detectable conjugate of the invention.

In some embodiments, the second step [step (b)] of the first method of the invention comprises detecting and/or quantifying the level of gp130 in the sample by measuring the formation of immune complexes formed between gp130 in the sample and the antibody of the invention.

In a particular embodiment, the antibody or reagent specific for the antibody of the invention is suitably labelled with a detectable label.

In other embodiments, the second step [step (b)] of the first method of the invention comprises detecting and/or quantifying the level of gp130 in the sample by measuring the formation of immune complexes formed between gp130 in the sample and the detectable conjugate of the invention. As mentioned above, the detectable conjugate of the invention comprises the antibody of the invention and a detectable label.

The term "detectable label" and its particulars have been described in detail the context of the detectable conjugate of the invention in the previous aspect of the invention and apply equally to this aspect of the invention and its embodiments.

In addition to the methods described within the context of the detectable conjugate of the invention in the previous aspect of the invention, and which apply equally to the first method of the invention, there is a wide range of other conventional assays that can be used in the first method of the invention to detect and/or quantify the level of gp130 in a sample and which may not include a detectable label. As it will be understood, the methods that do not include a detectable label apply to the antibody of the invention.

It will also be understood that antibodies that are not labelled need to be detected with an additional reagent, for example, a secondary antibody that is labelled, which will be labelled. This is particularly useful in order to increase the sensibility of the detection method, since it allows the signal to be amplified.

Thus, in some embodiments, the antibody of the invention is not in itself a detectable molecule. In these cases, the second step [step (b)] of the first method of the invention is a step of indirect detection via a second detectable molecule that specifically binds to the antibody of the invention. The detection of the antibody of the invention bound to gp130 can be carried out with virtually any antibody or reagent known to bind with high affinity to the antibody of the invention. However, it is preferable to use a specific antibody to the antibody of the invention, for example polyclonal sera, hybridoma supernatants, or monoclonal antibodies and fragments thereof.

Non-limiting examples include the use the antibody of the invention that is not labelled (primary antibody) followed by a labelled antibody (secondary antibody) for detection of the antibody of the invention (primary antibody), or the antibody of the invention that may (or may not) be labelled with a marker and does not require a secondary antibody for detection. These techniques include Western blot or immunoblot, bicinchoninic acid assay, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical and immunohistochemical techniques, immunohistochemistry, immunofluorescence, confocal microscopy, optical microscopy, electron microscopy, flow cytometry, fluorescence-activated cell sorting (FACS) or multiplex detection techniques based on using protein microspheres, biochips or microarrays which include the antibody of the invention. Other ways of detecting and/or quantifying the level of gp130 using the antibody of the invention include, but are not limited to, affinity chromatography techniques, ligand binding assays, lectin binding assays, particle-enhanced turbidimetric immunoassay (PETIA), etc.

In a particular embodiment, the detection and/or quantification of the level of gp130 in a sample by measuring the formation of immune complexes formed between gp130 and the antibody of the invention in the sample is carried out by immunohistochemical techniques, Western blot, bicinchoninic acid assay, immunofluorescence techniques, confocal microscopy, optical microscopy, electron microscopy, flow cytometry, or fluorescence-activated cell sorting (FACS).

In addition, the detection of the antibody can also be carried out by detecting changes in the physical properties in the sample that occur as a result of the binding of the antibody to its cognate antigen. These assays include determining a transmission-related parameter in a sample, which are known in the art. The term "transmission-related parameter", as used herein, relates to a parameter indicating or correlating with the ratio of transmitted light versus incident light of a sample or to a parameter derived therefrom.

In an embodiment, a transmission-related parameter is determined by turbidimetry or by nephelometry.

Turbidimetry, as used herein, refers to the measurement of light-scattering species in solution by means of a decrease in intensity of the incident beam after it has passed through solution. For turbidimetric assays, the change in the amount of light absorbed (inverse of amount transmitted) can be related to the amount of agglutination which occurs. Hence, the amount of analyte (the species causing agglutination) in the sample can be easily determined.

Nephelometry, as used herein, refers to a technique for measuring the light-scattering species in solution by means of the light intensity at an angle away from the incident light passing through the sample. Nephelometric assays present an indirect method of measurement of the amount of analyte in a sample by measuring the amount of light scattered or reflected at a given angle (typically 90°) from the origin. In the presence of the protein antigen, the antibody reacts with the antigen, and a precipitation reaction begins. The measurement is taken early in this precipitation reaction time sequence. A quantitative value is obtained by comparison with a standard curve, which has been established previously. In order to increase the sensitivity of the detection, the antibody can be adsorbed or covalently attached to polymeric microspheres. In this way, a greater signal is produced with less reagent.

The detection method based on turbidimetry or nephelometry according to the present disclosure works with all known agglutination tests with and without microparticles enhancement. Typically used within the present disclosure is a "microparticle-enhanced light scattering agglutination tests" which is also called "particle-enhanced turbidimetric immunoassays" (PETIA). Agglutination-based immunoassays are routinely used in clinical diagnostics for the quantitation of serum proteins, therapeutic drugs and drugs of abuse on clinical chemistry analyzers, because they have the benefits of being quasi-homogeneous assays which do not require any separation or wash step. To enhance the optical detection between the antigen to be detected and the specific antibody in the reaction mixture, the antibody may be linked to suitable particles. Thereby, the antigen reacts and agglutinates with the particles which are coated with antibody. With increasing amount of antibody, the agglutination and the size of the complexes are increasing, leading further to a change of light scattering.

In another embodiment, the binding of the antibody to its cognate antigen can be detected by Surface plasmon resonance (SPR).

As used herein, SPR refers to a phenomenon that the intensity of a reflected light decreases sharply at a particular angle of incidence (i.e., an angle of resonance) when a laser beam is irradiated to a metal thin film. SPR is a measurement method based on the phenomenon described above and is capable of assaying a substance adsorbed on the surface of the metal thin film, which is a sensor, with high sensitivity. According to the present invention, for example, the target substance in the sample can then be detected by immobilizing one or more antibodies according to the present invention on the surface of the metal thin film beforehand, allowing the sample to pass through the surface of the metal thin film, and detecting the difference of the amount of the substance adsorbed on the surface of the metal thin film resulting from the binding of the antibody and the target antigen, between before and after the sample passes therethrough.

All the terms and embodiments described in any of the previous aspects of the invention are equally applicable to the present aspect of the invention and its embodiments.

### Pharmaceutical compositions of the invention

A seventh aspect of the present invention relates to a pharmaceutical composition, hereinafter referred to as "the pharmaceutical composition of the invention", comprising the antibody of the invention, or the ADC of the invention, and a pharmaceutically acceptable excipient and/or carrier.

The terms and expressions "antibody", "ADC" and their particulars have been described in detail the context of the previous aspects of the invention and apply equally to this aspect of the invention and its embodiments.

In an embodiment, the pharmaceutical composition of the invention comprises a therapeutically effective amount of the antibody of the invention or of the ADC of the invention.

The expression "therapeutically effective amount", as used herein, means the amount of an active substance that, when administered to a subject for treating a disease, disorder, or other undesirable medical condition, is sufficient to have a beneficial effect with respect to that disease, disorder, or condition. The therapeutically effective amount will vary depending on the chemical identity and formulation form of the active substance, the disease or condition and its severity, and the age, weight, and other relevant characteristics of the patient to be treated. Determining the therapeutically effective amount of a given active substance is within the ordinary skill of the art and typically requires no more than routine experimentation.

The term "pharmaceutical composition" is such a form that allows the biological activity of the active ingredient contained therein to be effective and has non-toxicity for the subject to which the composition is administered.

The term "pharmaceutically acceptable carrier" refers to an ingredient of a pharmaceutical composition other than an active ingredient that is non-toxic to a subject. Pharmaceutically acceptable carriers include but are not limited to buffers, excipients, stabilizers or preservatives.

The expression "pharmaceutically acceptable excipient", as used herein, includes any and all solvents, dispersion media, coatings, antibacterial and antifungal agents, isotonic and absorption delaying agents that are physiologically compatible with the antibody of the invention.

Pharmaceutical compositions and formulations as described herein can be prepared by mixing the active ingredients having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn- protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.).

Supplementary active compounds can also be incorporated into the pharmaceutical composition of the invention. Thus, in a particular embodiment, the pharmaceutical composition of the invention may further comprise more than one active compound or therapeutic agent as necessary for the particular indication being treated, preferably those with complementary activities that do not adversely affect each other. The effective amount of such other active compound depends, among other things, on the amount of antibody of the invention or the ADC of the invention present in the pharmaceutical composition of the invention, the type of disease or disorder or treatment, etc.

The term "therapeutic agent" and its particulars have been described in detail the context of the ADC of the invention and apply equally to this aspect of the invention and its embodiments.

In an embodiment, the antibody of the invention or the ADC of the invention may be on the same formulation as the therapeutic agent(s) or may be administered in different formulations. Administration can be concurrent or sequential, and may be effective in either order.

In an embodiment, the antibody of the invention or the ADC of the invention is prepared with excipients that will protect said compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. These can be prepared according to methods known to those skilled in the art, for example, as described in U.S. 4,522, 811.

In a particular embodiment, the pharmaceutical composition of the invention can be administered by any type of suitable route, such as by oral route, topical route, by inhalation or parenteral route; preferably by parenteral route.

The term "parenteral", as used herein, includes intravenous, intraperitoneal, intramuscular, subcutaneous, rectal or vaginal administration. The intravenous form of parenteral administration is generally preferred. In addition, the pharmaceutical composition of the invention may suitably be administered by pulse infusion, e.g., with declining doses. Preferably the dosing is given by injections, most preferably intravenous or subcutaneous injections, depending in part on whether the administration is brief or chronic.

In a preferred embodiment, the pharmaceutical compositions of the invention may be adapted for parenteral administration, such as sterile solutions, suspensions or lyophilized products in the appropriate unit dosage form. Adequate excipients can be used, such as bulking agents, buffering agents or surfactants. Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, CremophorEM (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and/or gelatin. The mentioned formulations can be prepared using standard methods such as those described or referred to in the European and US Pharmacopoeias and similar reference texts.

Sterile injectable solutions can be prepared by incorporating the active compound (e.g., the antibody of the invention or the ADC of the invention) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

It is especially advantageous to formulate the pharmaceutical compositions in dosage unit form for ease administration and uniformity of dosage. Dosage unit form as used herein refers to physically discrete units suited as unitary dosages for the subject to be treated; each unit containing a predetermined quantity of active compound (e.g., the antibody of the invention) calculated to produce the desired therapeutic effect in association with the required pharmaceutical excipient or carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Generally an effective administered amount of the antibody of the invention or the ADC of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.001 to 1,000 mg/kg body weight/day, preferably about 0.01 to about 100 mg/kg body weight/day, most preferably from about 0.05 to 10 mg/kg body weight/day.

The pharmaceutical compositions can be included in a container, pack, or dispenser together with instructions for administration.

All the terms and embodiments described in any of the previous aspects of the invention are equally applicable to the present aspects of the invention and their embodiments.

### Therapeutic methods of the invention

The antibody of the invention, the ADC of the invention or the pharmaceutical composition of the invention can be used therapeutically.

In an embodiment, the present invention provides methods for immunotherapy comprising administering a therapeutic effective amount of the antibody of the invention or of the ADC of the invention.

Therefore, an eighth aspect of the present invention relates to the antibody of the invention, the ADC of the invention, or the pharmaceutical composition of the invention, for use in medicine, hereinafter referred to as "the first use of the invention".

Alternatively, the eighth aspect of the invention relates to the use of the antibody of the invention, the ADC of the invention, or the pharmaceutical composition of the invention, in the manufacture of a medicament.

The term "medicament", as used herein, is understood as a pharmaceutical composition comprising the antibody of the invention or the ADC of the invention.

The authors of the present invention have discovered that neutralizing antibodies that block the gp130 mediated IL-6 family of cytokines signaling pathway promotes anti-tumor responses in different *in vivo* cancer models, namely glioblastoma, prostate cancer bone metastasis, breast cancer bone metastasis, lung cancer brain metastasis and PDAC liver metastasis (see Figure 6 of the application).

Thus, a ninth aspect of the present invention relates to the antibody of the invention, the ADC of the invention, or the pharmaceutical composition of the invention, for use in the treatment and/or prevention of cancer and/or an inflammatory disease, hereinafter referred to as "the second use of the invention".

Alternatively, the ninth aspect of the present invention relates to the use of the antibody of the invention, the ADC of the invention, or the pharmaceutical composition of the invention, in the manufacture of a medicament for the treatment and/or prevention of cancer and/or an inflammatory disease.

Alternatively, this ninth aspect of the present invention may be reformulated as a method of treatment and/or prevention of cancer and/or an inflammatory disease, comprising administering the antibody of the invention, the ADC of the invention, or the pharmaceutical composition of the invention, to a subject in need thereof.

The terms and expressions "antibody", "ADC", "gp130", "pharmaceutical composition", "subject" and their particulars have been described in detail the context of the previous aspects of the invention and apply equally to these aspects of the invention and their embodiments.

The term "treat", "treatment", or "amelioration", as used herein, refer to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (i.e., not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment). In some embodiments, treating cancer includes reducing or stabilizing tumor volume, reducing or stabilizing the number of cancer cells, suppressing cancer metastasis, prolonging life, reducing cancer cell growth, reducing cell survival, or reducing cancerous status. It involves amelioration of the various physiological symptoms involved.

As used herein, the terms "prevent", "prevention" and "preventing" refer to the reduction in the risk of acquiring or developing a given disease or disorder, e.g., cancer, or the reduction or inhibition of the recurrence or a disease or disorder, e.g., cancer. Prevention also refers to a decrease in the occurrence of a given disease or condition. The prevention may also be partial, such that, for example, when the occurrence of the disease or condition in a subject is less than that which would have occurred without the present invention. Prevention also refers to reduced susceptibility to a clinical condition.

The term "cancer", "tumor" or "tumor disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. Cancers usually share some of the following characteristics: sustaining proliferative signaling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor. Examples of cancer or tumor include without limitation, breast cancer, heart cancer, lung cancer, small intestine cancer, colon cancer, rectal cancer, colorectal cancer, spleen cancer, kidney cancer, bladder cancer, head cancer, neck cancer, ovarian cancer, prostate cancer, brain cancer, pancreatic cancer, skin cancer, bone cancer, bone marrow cancer, blood cancer, thymus cancer, uterin cancer, testicular cancer, hepatobiliary cancer, liver cancer, tongue cancer, adenocarcinoma, carcinoma, melanoma, epithelial cancer, oral cancer, salivary gland cancer, esophageal cancer, thyroid cancer, endometrial cancer, stomach cancer, glial-derived tumor, neural crest-derived tumor, adrenal cancer, carcinoid tumors, nervous system tumors, glioma, adenoma, angiosarcoma, astrocytoma, germinoma, glioblastoma, glioma, hemangioendothelioma, hepatoblastoma, leukaemia, lymphoma, medulloblastoma, neuroblastoma, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, or Wilm's tumor.

In the context of the present invention, "metastasis" is understood as the propagation of a cancer from the organ where it started (primary tumor) to a different organ. It generally occurs through the blood or lymphatic system. When the cancer cells spread and form a new tumor, the latter is called a secondary or metastatic tumor. The cancer cells forming the secondary tumor are like those of the original tumor. If a lung cancer, for example, spreads (metastasizes) to the brain, the secondary tumor is formed of malignant lung cancer cells. The disease in the brain is metastatic lung cancer and not brain cancer. In a particular embodiment of the second use of the invention, the metastasis is lung cancer which has spread (metastasized) to the brain.

If a PDCA (Pancreatic Ductal Adenocarcinoma) cancer, for example, spreads (metastasizes) to the liver, the secondary tumor is formed of malignant PDCA cancer cells. The disease in the liver is metastatic PDCA cancer and not liver cancer. In a particular embodiment of the second use of the invention, the metastasis is PDCA cancer which has spread (metastasized) to the liver.

If a breast cancer, for example, spreads (metastasizes) to the bone, the secondary tumor is formed of malignant breast cancer cells. The disease in the bone is metastatic breast cancer and not bone cancer. In a particular embodiment of the second use of the invention, the metastasis is breast cancer which has spread (metastasized) to the bone.

If a prostate cancer, for example, spreads (metastasizes) to the bone, the secondary tumor is formed of malignant prostate cancer cells. The disease in the bone is metastatic prostate cancer and not bone cancer. In a particular embodiment of the second use of the invention, the metastasis is prostate cancer which has spread (metastasized) to the bone.

In a preferred embodiment of the second use of the invention, the cancer is a primary tumor and/or a secondary or metastatic tumor.

In a more preferred embodiment of the second use of the invention, the secondary or metastatic tumor is bone metastasis, brain metastasis or liver metastasis. In a yet more preferred embodiment, the secondary or metastatic tumor is bone metastasis.

In an embodiment, the cancer is characterized by the presence of tumor-associated macrophages (TAMs) with increased gp130 signaling.

The term "tumor-associated macrophages" or "TAMs", as used herein, refers to an immunosuppressive macrophage subtype found in the tumor microenvironment that is involved in the progression and metastasis of cancer. TAMs are broadly considered M2-like, which can be further classified into the M2a phenotype (induced by IL-4 or IL- 13), M2b phenotype (IL- 10 high, IL- 12 low) and M2c phenotype (TNF-a low) according to distinct signal stimuli. They produce abundant growth factors, extracellular matrix (ECM) remodeling molecules and cytokines for the regulation of cancer proliferation via noncoding RNAs, exosomes and epigenetics (Yan, S. and Wan, G. The FEBS Journal (2021) 288 (21): 6174-86, citing Qian, BZ and Pollard, JW. (Cell (2010) 141: 39-51). Activated M2 macrophages distinctively express arginase 1 (ARG1). TAMs can demonstrate direct inhibition on the cytotoxicity of T-lymphocyte through multiple mechanisms and characteristics of tumor evolution, including immune checkpoint engagement via expression, production of inhibitory cytokines [such as IL- 10 and transforming growth factor (TGF)-P] and metabolic activities consisting of depletion of 1-arginine (or other metabolites) and the production of reactive oxygen species (ROS). The suppressive immune response renders cancer cells capable of escaping from immune surveillance.

The tumor-associated macrophages (TAMs) in the context of the present invention have increased gp130 signaling. As used herein, the expression "increased gp130 signaling" or "increased gp130 activity" in a tumor-associated macrophages (TAMs), refers to higher total gp130 activity in the TAMs of the test subject or biological sample in comparison with a control, e.g., a healthy subject or a standard sample. Preferably, although not necessarily, the activity is at least 10%, more preferably at least 50%, even more preferably at least 100%, and still more preferably at least 150% higher in the test subject or sample than in the control. The increased activity, for example, may result from increased basal gp130 activity in the TAMs, prolonged stimulation, delayed degradation, or over-expression, e.g., due to enhanced ligand binding, promiscuous or inappropriate ligand binding, constitutive receptor activation, impaired recycling resulting in augmentation of signaling or delayed degradation.

As mentioned in the context of the antibody of the invention, an anti-gp130 neutralizing antibody is capable of neutralizing the biological signaling activity of gp130 for example by blocking or inhibiting the binding of Interleukin (IL)-6 family of cytokines (i.e. Interleukin-6 (IL-6), Interleukin-11 (IL-11), Interleukin-27 (IL-27), leukemia inhibitory factor (LIF), oncostatin M (OSM), ciliary neurotrophic factor (CNTF), cardiotrophin-1 (CT-1), novel neurotrophin-1/B cell stimulating factor-3 or cardiotrophin-like cytokine factor 1 (CLCF1), and neuropoietin (NP)) to gp130.

The authors of the present invention have surprisingly discovered that, the antibody of the invention is capable of inhibiting STAT3 phosphorylation at position Y705 in response to the LIF, OSM, IL-6, IL-11 and/or CT-1 members of the IL-6 family of cytokines (but not of IL-27) which, as previously explained in the context of the antibody of the invention, requires the interaction of the cytokine with a complex containing the cytokine specific receptor and gp130 (see Figure 4 of the application).

Thus, the antibody of the invention is capable of neutralizing the signaling activity in response to LIF, OSM, IL-6, IL-11 and/or CT-1 members of the IL-6 family of cytokines as a result of its interaction with receptor complexes containing their specific receptors and gp130, but it is not capable of neutralizing the signaling activity in response to the IL-27 member of the IL-6 family of cytokines as a result of its interaction with the complex containing its specific receptor and gp130. This is specially advantageous since all gp130 mediated IL-6 family of cytokines are oncogenic except for IL-27 that has been described to potentiate the anti-tumor immune response (Salcedo et al., J Immunol 2004, 173(12):7170-82; Hisada et al., Cancer Research 2004, 64(3):1152-6; Chiyo et al., Int. J. Cancer 2005, 115(3):437-42; Rolvering et al., Biochim Biophys Acta Mol Cell Res 2017, 1864(3):516-526; Zhu et al., JCI Insight 2018, 3(7):e98745).

In an embodiment, the antibody of the invention, the ADC of the invention, or the pharmaceutical composition of the invention is capable of neutralizing the signaling activity in response to LIF, OSM, IL-6, IL-11 and/or CT-1 of the IL-6 family of cytokines as a result of its interaction with receptor complexes containing their specific receptors and gp130.

In another embodiment, the antibody of the invention, the ADC of the invention, or the pharmaceutical composition of the invention is not capable of neutralizing the signaling activity in response to IL-27 member of the IL-6 family of cytokines as a result of its interaction with the complex containing its specific receptor and gp130.

The term "control" or "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. In one embodiment, the reference value corresponds to the gp130 signaling levels in TAMs determined in a healthy subject, whereby a healthy subject is understood as a subject that shows TAMs with no increased gp130 signaling at the moment the levels of gp130 activation is determined.

In another embodiment, the reference value corresponds to an average or mean level of the corresponding gp130 signaling in the TAMs from a pool of samples obtained from a group of patients who are well documented from the clinical point of view, and who present no disease, particularly who are not suffering cancer. In said samples, the gp130 activation levels in the TAMs can be determined, for example by verification of gp130 mediated STAT3 phosphorylation level in Y705 upon stimulation with LIF, OSM, IL-6, IL-11 and/or CT-1, upon stimulation with IL-27, as shown in the examples of the present application. In the determination of the reference value, it is necessary to take into consideration some characteristics of the type of sample, such as age, gender, the physical state or other characteristics of the patient. For example, the reference sample can be obtained from identical amounts of a group of at least 2, at least 10, at least 100 to more than 1000 individuals, such that the population is statistically significant.

The term "tumor microenvironment", "TME" or "microenvironment of solid tumors" or, as used herein, refers to the cellular environment in which the tumor exists, including the area immediately surrounding fibroblasts, leukocytes and endothelial cells and the extracellular matrix (ECM).

Tumors have been found to be able to escape host immunity by manipulating the tumor microenvironment and driving immunosuppression, such that although the components necessary for mounting an effective anti-tumor immune response are present in patients with cancer, the host usually fails to arrest tumor progression. Numerous molecular and cellular mechanisms have been proposed in the past to explain this counterintuitive scenario, largely focusing on the "immune escape" of tumors and suggesting that tumors develop the capability to avoid tumor-specific immune responses generated by the host, or they altogether disable the host anti-tumor immunity. This process, referred to as "tumor-induced immune suppression" has been recognized in recent years and is being intensively investigated. It appears that tumors can interfere with all components of the immune system, affecting all stages of the anti-tumor immune response.

In a particular embodiment, the cancer is a solid tumor.

The term "solid tumor" and its particulars have been described in detail the context of the previous aspects of the invention and apply equally to these aspects of the invention and their embodiments.

In a more particular embodiment, the cancer is selected from the group consisting of glioblastoma, bone cancer, liver cancer, brain cancer, lung cancer, breast cancer, ovarian cancer, uterine cancer, pancreatic cancer, prostate cancer, hepatobiliary cancer, kidney cancer, renal cancer, head and neck cancer, tongue cancer, colorectal cancer, adenocarcinoma, carcinoma, melanoma, epithelial cancer, oral cancer, salivary gland cancer, esophageal cancer, thyroid cancer, endometrial cancer, skin cancer, gall bladder cancer, sarcoma, bladder cancer, urethral cancer, gastric cancer, stomach cancer, testicular cancer, cervical cancer, a glial-derived tumor, a neural crest-derived tumor, adrenal cancer, carcinoid tumors, nervous system tumors, glioma, neuroblastoma, lymphoma and metastasis thereof.

In a yet more particular embodiment, the cancer is glioblastoma, bone metastasis, liver metastasis and/or brain metastasis.

The term "glioblastoma", "glioblastoma multiforme", "GM", GBM" or "malignant glioma", as used herein, refers to a primary brain tumor involving glial cells. Glioblastoma is an anaplastic, highly cellular tumor with poorly differentiated, round, or pleomorphic cells, occasional multinucleated cells, nuclear atypia, and anaplasia. Variants of the tumor include gliosarcoma, multifocal GBM, or gliomatosis cerebii (in which the entire brain may be infiltrated with tumor cells). Glioblastoma seldomly metastasizes to the spinal cord or outside the nervous system. Glioblastoma is graded by their microscopic and histological appearance. Glioblastoma is, preferably, diagnosed based on the histological presence of proliferative glial tumor cells, vascular proliferation and preferentially necrotic tissue areas.

The term "bone metastasis", as used herein, refers to cancer cells that have migrated from the original location of the cancer (e.g., primary cancer) to the bone. In an embodiment, the primary cancer is breast cancer or prostate cancer.

The term "liver metastasis", as used herein, refers to cancer cells that have migrated from the original location of the cancer (e.g., primary cancer) to the liver. In an embodiment, the primary cancer is PDAC (Pancreatic Ductal Adenocarcinoma) cancer.

The term "brain metastasis", as used herein, refers to cancer cells that have migrated from the original location of the cancer (e.g., primary cancer) to the brain. In an embodiment, the primary cancer is lung cancer.

The term "metastasis" and its particulars have been previously described in detail and apply equally herein.

The term "inflammatory disease" or "inflammatory disorders", as used herein, refers to a condition in a subject characterized by inflammation, e.g., chronic inflammation. Illustrative, non-limiting examples of inflammatory disorders include, but are not limited to, arthritis, rheumatoid arthritis (RA), fibrosis, Inflammatory Bowel Disease (IBD), Chrohn's disease, ulcerative colitis, asthma, pulmonary hypertension, chronic obstructive pulmonary disease (COPD), bronchopulmonary displasia, encephalomyelitis, diabetes, lupus, psoriasis, rosacea, vasculitis, hepatitis, systemic sclerosis, gout, myositis, scleroderma, Celiac Disease, encephalitis, inflammatory osteolysis, allergic disorders, septic shock, pulmonary fibrosis (e.g., idiopathic pulmonary fibrosis), inflammatory vaculitis (e.g., polyarteritis nodosa, Wegner's granulomatosis, Takayasu's arteritis, temporal arteritis, and lymphomatoid granulomatosus), post-traumatic vascular angioplasty (e.g., restenosis after angioplasty), undifferentiated spondyloarthropathy, undifferentiated arthropathy, inflammatory osteolysis, chronic hepatitis, and chronic inflammation resulting from chronic viral or bacteria infections. The term "inflammatory disease" also includes immune mediated inflammatory disease, which shall be taken to mean any disease mediated by the immune system and characterized by chronic or acute inflammation, resulting from, associated with or triggered by, a dysregulation of the normal immune response e.g. Crohn's disease, type 1 diabetes mellitus, rheumatoid arthritis, inflammatory bowel disease, psoriasis, psoriatic arthritis, ankylosing spondylitis, systemic lupus erythematosus, Hashimoto's disease, graft-versus-host disease, Sjogren's syndrome, pernicious anemia, Addison disease, scleroderma, Goodpasture's syndrome, ulcerative colitis, autoimmune hemolytic anemia, sterility, myasthenia gravis, multiple sclerosis, Basedow's disease, thrombopenia purpura, Guillain-Barré syndrome, allergy, asthma, atopic disease, arteriosclerosis, myocarditis, cardiomyopathy, glomerular nephritis, hypoplastic anemia, or rejection after organ transplantation.

In an embodiment, the inflammatory disease is characterized by increased gp130 signaling.

As used herein, the expression "increased gp130 signaling" or "increased gp130 activity" refers to higher total gp130 activity of the test subject or biological sample in comparison with a control, e.g., a healthy subject or a standard sample. Preferably, although not necessarily, the activity is at least 10%, more preferably at least 50%, even more preferably at least 100%, and still more preferably at least 150% higher in the test subject or sample than in the control. The increased activity, for example, may result from increased basal gp130 activity, prolonged stimulation, delayed degradation, or over-expression, e.g., due to enhanced ligand binding, promiscuous or inappropriate ligand binding, constitutive receptor activation, impaired recycling resulting in augmentation of signaling or delayed degradation.

The term "control" or "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. In one embodiment, the reference value corresponds to the gp130 signaling levels determined in a healthy subject, whereby a healthy subject is understood as a subject that shows no increased gp130 signaling at the moment the levels of gp130 activation is determined.

In another embodiment, the reference value corresponds to an average or mean level of the corresponding gp130 signaling from a pool of samples obtained from a group of patients who are well documented from the clinical point of view, and who present no disease, particularly who are not suffering cancer. In said samples, the gp130 activation levels can be determined, for example by verification of gp130 mediated STAT3 phosphorylation level in Y705 upon stimulation with LIF, OSM, IL-6, IL-11 and/or CT-1, but not upon stimulation with IL-27, as shown in the examples of the present application. In the determination of the reference value, it is necessary to take into consideration some characteristics of the type of sample, such as age, gender, the physical state or other characteristics of the patient. For example, the reference sample can be obtained from identical amounts of a group of at least 2, at least 10, at least 100 to more than 1000 individuals, such that the population is statistically significant.

In a particular embodiment, the inflammatory disease is selected from the group consisting of arthritis, rheumatoid arthritis, fibrosis, inflammatory bowel disease, Crohn's disease, ulcerative colitis, asthma, pulmonary hypertension, chronic obstructive pulmonary disease, bronchopulmonary displasia, encephalomyelitis, diabetes, lupus, psoriasis, rosacea, vasculitis, hepatitis, systemic sclerosis, gout, myositis and scleroderma.

In a more particular embodiment, the inflammatory disease is rheumatoid arthritis and/or fibrosis.

The term "rheumatoid arthritis" or "RA", as used herein, refers to a long-term autoimmune disorder that primarily affects joints that typically results in warm, swollen, and painful joints. Pain and stiffness often worsen following rest. Most commonly, the wrist and hands are involved, with the same joints typically involved on both sides of the body. The disease may also affect other parts of the body, including skin, eyes, lungs, heart, nerves and blood. This may result in a low red blood cell count, inflammation around the lungs, and inflammation around the heart. Fever and low energy may also be present. Often, symptoms come on gradually over weeks to months.

The term "fibrosis", as used herein, refers to the pathological process of formation of excess fibrous connective tissue in an organ or tissue. Fibrosis is characterized by fibroblast accumulation and collagen deposition in excess of normal deposition in any particular tissue. In response to inflammation or an injury to a tissue, nearby fibroblasts can migrate into the wound, proliferate, and produce large amounts of collagenous extracellular matrix. When fibrosis occurs in response to injury, the term "scarring" can be used as synonym.

As the person skilled in the art will recognize, the antibody of the invention, the ADC of the invention, or the pharmaceutical composition of the invention may be useful in therapeutic applications in various forms, which include without limitation, in the form of intact antibody or intact ADC, conjugated to another therapeutic agent, or in the form of a fusion protein with another therapeutic agent. These therapeutic applications will comprise the administration of a therapeutically effective amount of the antibody of the invention or of the ADC of the invention.

The term "therapeutically effective amount" and its particulars have been described in detail in the context of the pharmaceutical composition of the invention, and apply equally to these aspects of the invention and their embodiments.

In a particular embodiment of the first and the second uses of the invention, the antibody of the invention or the ADC of the invention is in the form of intact antibody or an intact ADC, i.e., in the form of immunoglobulin. The antibody of the invention, when is in the form of immunoglobulin, typically uses a combination of mechanisms in directing cytotoxic effects to a gp130-expressing cell: (i) it interacts with components of the immune system through antibody-dependent cellular cytotoxicity (ADCC) or (ii) through complement-dependent cytotoxicity (CDC).

Antibody-dependent cellular cytotoxicity (ADCC) occurs when antibodies bind to antigens on cells and the antibody Fc domains engage Fc receptors (FcR) on the surface of immune effector cells. Several families of Fc receptors have been identified, and specific cell populations characteristically express defined Fc receptors. For example, neutrophils commonly express human FcγRI (CD64), FcγRII (CD32) and the B (lipid anchored) isoform of FcγRIII (CD16). In contrast, human natural killer (NK) cells express only the A (transmembrane) isoform of CD16. This structure facilitates recruitment of adaptor proteins and activation of NK cells by antibody engagement of CD16.

Complement-dependent cytotoxicity (CDC) is another cell-killing method that can be directed by antibodies. As with ADCC, the different subclasses of antibodies have varying abilities to elicit CDC responses. IgM is the most effective isotype for complement activation, and IgG1 and IgG3 are both very effective at directing CDC via the classical complement activation pathway. In this cascade, the formation of antigen-antibody complexes results in the uncloaking of multiple C1q binding sites in close proximity on the CH2 domains of participating IgG molecules (C1q is one of three subcomponents of complement C1). These uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. The complement cascade ends in the formation of a membrane attack complex, which creates 100 Å pores in the cell membrane that facilitate free passage of water and solutes into and out of the cell.

In another particular embodiment of the first and second uses of the invention, the antibody of the invention or the ADC of the invention is conjugated to or in the form of a fusion protein with another therapeutic agent.

The term "therapeutic agent" and its particulars have been described in detail in the context of the previous aspects of the invention, and apply equally to these aspects of the invention and their embodiments.

In a preferred embodiment of the first and second uses of the invention, the antibody of the invention or the ADC of the invention is conjugated to a detectable label, such as a radionuclide. The term "detectable label" and its particulars have been described in detail within the context of the detectable conjugate of the invention, and apply equally to these aspects of the invention and their embodiments.

The therapeutic agent can be an immunosuppressive agent, i.e., a substance that acts to suppress or mask the immune system of the mammal being treated herein. This would include substances that suppress cytokine production, down-regulate or suppress self-antigen expression, or mask the MHC antigens.

The therapeutic agent can also be a cytotoxic agent, i.e., a substance that inhibits or prevents the function of cells and/or causes destruction of cells. The term is intended to include radioactive isotopes (as described above), chemotherapeutic agents, i.e., chemical compounds useful in the treatment of cancer, and toxins such as small molecule toxins or enzymatically active toxins of bacterial, fungal, plant or animal origin, or fragments thereof.

The therapeutic agent can also be a cytokine, a hormone, growth factor, necrosis factor, i.e., a protein or peptide released by one cell population which act on another cell as intercellular mediators or even in the same cell population. As used herein, the term cytokine includes proteins and peptides from natural sources or from recombinant cell culture and biologically active equivalents of the native sequence cytokines.

In a preferred embodiment of the first and second use of the invention, the antibody of the invention or the ADC of the invention is conjugated to one or more toxin molecules. Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes.

The present invention further contemplates the antibody of the invention or the ADC of the invention to be conjugated with a compound with nucleolytic activity (e.g. a ribonuclease or a DNA endonuclease such as a deoxyribonuclease; DNase) or other compound capable of damaging a cellular structure or organelle and therefore killing or diminishing the vitality of the cell.

Conjugates of the antibody of the invention or of the ADC of the invention and cytotoxic agent may be made using a variety of bifunctional protein coupling agents or linkers. The linker may be a "cleavable linker" facilitating release of the cytotoxic drug in the cell. For example, an acid-labile linker, peptidase-sensitive linker, dimethyl linker or disulfide-containing linker may be used.

Alternatively, a fusion protein comprising the antibody of the invention or the ADC of the invention and cytotoxic agent may be made, e.g. by recombinant techniques or peptide synthesis.

The therapeutic agent can also be a prodrug which refers to a precursor or derivative form of a pharmaceutically active substance that is less cytotoxic to cells expressing gp130 compared to the parent drug and is capable of being, for instance, enzymatically, temperature, light or pH dependent activated or converted into the more active parent form.

In a preferred embodiment of the first and second uses of the invention, the antibody of the invention or the ADC of the invention may also be conjugated with a prodrug activating agent which converts a prodrug to an active cytotoxic drug. The agent component of such conjugates includes any agent capable of acting on a prodrug in such a way so as to convert it into its more active, cytotoxic form. This is particularly useful in antibody-directed enzyme prodrug therapy approaches (ADEPT).

Enzymes useful in the present invention include any enzyme that can convert a prodrug into an active therapeutic agent (drug). In a specific embodiment, the enzyme is an enzyme from a mammal, for example, a human enzyme. In another specific embodiment, the enzyme is an enzyme from an organism other than a mammal; in this last embodiment, the immunogenicity of the enzyme is optionally reduced, for example, by conjugation to polyethylene glycol (PEG) and the like. In accordance with a particular embodiment, neither the enzyme nor an enzyme with similar substrate specificity is endogenous to the subject along the route of administration or biodistribution of the prodrug. In a particular embodiment, enzymes useful in the present invention are lysosomal enzymes (such as, but not limited to alpha-galactosidase, acid sphingomyelinase, hyaluronidase, glucosidase-alpha, beta-glucocerebrosidase, etc.). Thrombolytic enzymes (such as, but not limited to heparin) and antioxidant enzymes (such as but not limited to catalase, superoxide dismutase, etc.).

Proteases, glycosidases, esterases and the like are general types of enzymes that can be used in accordance with the present invention. Specific examples of suitable enzymes include, but are not limited to, glycosidases (beta-glucuronidase, beta-glucosidase, beta-galactosidase), beta-lactamase, cellulase, dextranase, fructase, aminopeptidase, lysozyme, cytosine deaminase, carboxypeptidase, penicillin amidase, methionine γ liase, and carboxyesterase. The enzyme is selected for its ability to convert the selected prodrug into its active drug form. For example, if the prodrug comprises a conjugate of dextran and a therapeutic agent, an appropriate enzyme would be dextranase. Similarly, cellulase could be used with a prodrug comprising a cellulose substrate, and glucuronidase could be used with a prodrug comprising a glucuronide.

In a preferred embodiment, the enzyme is selected from the group consisting of glycosidases (glucuronidase, beta-glucosidase, beta-galactosidase), beta-lactamase, cellulase, dextranase, fructase, aminopeptidase, lysozyme, cytosine deaminase, carboxypeptidase, penicillin amidase, methionine γ liase, and carboxyesterase, or a functional variant or fragment thereof having catalytic activity. More preferably, the enzyme is a glycosidase (e.g. beta-galactosidase) or a cytosine deaminase.

Drugs useful in the context of this invention include toxins, antibiotic or chemotherapeutic drugs, radioisotopes, paramagnetic ions, boron addends, cytokines, photosensitizers, radiosensitizers, vasodilators, immunomodulator agents, immune-suppressive agents, (gluco)corticoids, analgesic agents, anti-inflammatory agents, antiviral agents, antithrombotic agents, hormones, vitamins, cofactors, steroids, thrombolytic agents, recombinant enzymes, another antibody, gene therapy agents, gene silencing agents, gene editing agents, cytotoxins, radionuclides, etc.

In a particular embodiment, the drug is a drug having a cytotoxic activity. The cytotoxic activity of a drug or a prodrug can be performed by means of assays that are well known in the art. For example, assessing cell membrane integrity is one of the most common ways to measure cell viability and cytotoxic effects, since compounds that have cytotoxic effects often compromise cell membrane integrity. Vital dyes, such as trypan blue or propidium iodide are normally excluded from the inside of healthy cells; however, if the cell membrane has been compromised, they freely cross the membrane and stain intracellular components. Alternatively, membrane integrity can be assessed by monitoring the passage of substances that are normally sequestered inside cells to the outside. One commonly measured molecule is lactate dehydrogenase (LDH).

Cytotoxicity can also be monitored using the MTT assay. This assay measures the reducing potential of the cell using a colorimetric reaction. Viable cells will reduce the dimethyl thiazolyl diphenyl tetrazolium salt (MTT) reagent to a coloured formazan product. A similar redox-based assay has also been developed using the fluorescent dye, resazurin. In addition to using dyes to indicate the redox potential of cells in order to monitor their viability, assays that use ATP content as a marker of viability can also be used. Such ATP-based assays include bioluminescent assays in which ATP is the limiting reagent for the luciferase reaction. Cytotoxicity can also be measured by the sulforhodamine B (SRB) assay, water soluble tetrazolium salt (WST) assay and clonogenic assay. A label-free approach to follow the cytotoxic response of adherent animal cells in real-time is based on electric impedance measurements when the cells are grown on gold-film electrodes. This technology is referred to as electric cell-substrate impedance sensing (ECIS).

In another particular embodiment, the drug is more cytotoxic than its corresponding prodrug from which the drug is released. The cytotoxicity of a compound is typically expressed as its IC₅₀ value. The IC₅₀ value, as used herein, refers to the half maximal inhibitory concentration and represents the concentration of a compound required for obtaining 50% of a maximum effect *in vivo.* In a particular preferred embodiment, the active drug is more cytotoxic than its corresponding prodrug. This can be estimated with the QIC₅₀ value, which is the ratio of the IC₅₀ of the prodrug / IC₅₀ of the drug. The QIC₅₀ value can be 5 or higher than 5, for example, 10 or higher than 10, 10² or higher than 10², 10³ or higher than 10³, 10⁴ or higher than 10⁴, or even higher. Preferably, the QIC₅₀ is higher than 10², and more preferably, higher than 10³.

Illustrative, non-limitative, examples of prodrugs which can be used in the context of the present invention include glycosidic prodrugs, for example, duocarmycin-derived galactosyl prodrugs, N-(beta-D galactopyranosyloxycarbonyl)-doxorubicin and N-[4-(beta-D-galactopyranosyl)-3-nitrobenzyloxycarbonyl]daunomycin; 5-fluorocytosine; cephalosporin prodrugs, such as PROTAX (cephalosporin derivative of taxol), C-DOX (cephalosporin derivative of doxorubicin), CCM (cephalosporin mustard prodrug), etc.; palytoxin prodrug NHPAP, doxorubicin prodrug DPO, combretastatin prodrugs such as combretastatin A-4 prodrug (CA-4PD), bisphosphonate prodrugs like bisphosphonamidate clodronate etc. Prodrugs can usually be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

Alternatively, fusion proteins comprising at least the antibody of the invention or at least the ADC of the invention linked to at least a functionally active portion of an enzyme of the invention can be constructed using recombinant DNA techniques well known in the art.

Although human, partially human, or humanised antibodies will be suitable for many applications, particularly those involving uses of the antibody in the preparation of a medicament for treatment in a human subject, other types of antibodies, i.e., of different origin, will be suitable for certain applications. The non-human antibodies of the invention can be, for example, derived from any antibody-producing animal, such as mouse, rat, rabbit, goat, donkey, or non-human primate such as monkey (e.g., cynomologous or rhesus monkey) or ape (e.g., chimpanzee). Non-human antibodies of the invention can be used, for example, in *in vitro* and cell-culture based applications, or any other application where an immune response to the antibody of the invention does not occur, is insignificant, can be prevented, is not a concern, or is desired.

The antibody of the invention, the ADC of the invention, the detectable conjugate of the invention, or the pharmaceutical composition of the invention can be used for targeting a therapeutic agent or for detection purposes.

Thus, in a tenth aspect, the present invention relates to the use of the antibody of the invention, the ADC of the invention, the detectable conjugate of the invention, or the pharmaceutical composition of the invention:
- for targeting a therapeutic agent to a specific site wherein said specific site is characterized by the presence of tumor-associated macrophages (TAMs) with increased gp130 signaling or said specific site is characterized by increased gp130 signaling; and/or
- for detecting and/or quantifying the presence of gp130 in a sample.

Alternatively, the tenth aspect of the present invention may be reformulated as the use of the antibody of the invention, the ADC of the invention, the detectable conjugate of the invention, or the pharmaceutical composition of the invention in the manufacture of a composition:
- for targeting a therapeutic agent to a specific site wherein said specific site is characterized by the presence of tumor-associated macrophages (TAMs) with increased gp130 signaling or said specific site is characterized by increased gp130 signaling; and/or
- for detecting and/or quantifying the presence of gp130 in a sample.

Alternatively, the tenth aspect of the present invention may be reformulated as a method:
- for targeting a therapeutic agent to a specific site wherein said specific site is characterized by the presence of tumor-associated macrophages (TAMs) with increased gp130 signaling or said specific site is characterized by increased gp130 signaling; and/or
- for detecting and/or quantifying the presence of gp130 in a sample.
comprising administering the antibody of the invention, the ADC of the invention, the detectable conjugate of the invention, or the pharmaceutical composition of the invention, to a subject in need thereof.

The terms and expressions "antibody", "ADC", "detectable conjugate", "pharmaceutical composition", "gp130", "sample", "subject", "therapeutic agent", tumor-associated macrophages (TAMs)", "increased gp130 signaling", "detecting", "quantifying" and their particulars have been described in detail the context of the previous aspects of the invention and apply equally to this aspect of the invention and its embodiments.

The antibody of the invention or the ADC of the invention can be thus considered to be a vehicle, such as a nano-vehicle, to the specific site. For this application, the antibody of the invention or the ADC of the invention will preferably be administered in the pharmaceutical composition of the invention, whose particulars have been described elsewhere in the present specification and which are incorporated herein by reference. In a particular embodiment, the therapeutic agent is selected from the group consisting of toxins, antibiotic, chemotherapeutic drugs, cytotoxic agents, protein kinase inhibitors, radioisotopes, paramagnetic ions, boron addends, cytokines, photosensitizers, radiosensitizers, vasodilators, immunomodulator agents, immune-suppressive agents, and (gluco)corticoids, analgesic agents, anti-inflammatory agents, antiviral agents, antithrombotic agents, hormones, vitamins, cofactors, steroids, thrombolytic agents, recombinant enzymes, another antibody, gene therapy agents, gene silencing agents, gene editing agents, cytotoxins, and radionuclides, whose particulars have been described above within the context of the therapeutic agent of the ADC of the invention and which are incorporated herein by reference.

In a particular embodiment, said specific site is characterized by the presence of tumor-associated macrophages (TAMs) with increased gp130 signaling or said specific site is characterized by increased gp130 signaling.

In another particular embodiment, the antibody of the invention or the ADC of the invention is conjugated to a detectable label. The term "detectable label" and its particulars have been described in detail within the context of the detectable conjugate of the invention, and apply equally to this aspect of the invention and its embodiments. Methods for detecting and/or quantifying the presence of gp130 in a sample have been described in the context of the first method of the invention and apply equally to this aspect of the invention and its embodiments.

All the terms and embodiments described in any of the previous aspects of the invention are equally applicable to the present aspects of the invention and their embodiments.

### Combination therapy of the invention

The authors of the present invention have discovered a combination therapy comprising a neutralizing antibody which specifically binds to gp130 and immunotherapies with enhanced anti-tumor effect (see Figures 6F and 7 of the application).

Therefore, an eleventh aspect of the present invention relates to a combination therapy, hereinafter referred to as "the combination therapy of the invention", comprising a neutralizing antibody, antigen-binding fragment thereof or antibody construct comprising an antigen-binding fragment thereof specifically binding to gp130 and at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC).

The terms and expressions "antibody", "antigen-binding fragment", "antibody construct", "neutralizing", "specifically binding", "gp130" and their particulars have been described in detail the context of the antibody of the invention and apply equally to this aspect of the invention and its embodiments.

In a preferred embodiment of the combination of the invention, the neutralizing antibody is the antibody according to the first aspect of the invention.

As used herein, the term "combination therapy" refers to the administration of two or more therapeutic substances, e.g. an anti-gp130 antibody (first agent) and a second therapy (second agent). The second therapy may be administered concomitant with, prior to, or following the administration of the anti-gp130 antibody.

The term "combination" includes co-administration of a first agent and a second agent, which for example may be dissolved or intermixed in the same pharmaceutically acceptable carrier, or administration of a first agent, followed by the second agent, or administration of the second agent, followed by the first agent.

The term "concomitant" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed stepwise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (e.g., human).

The term "adoptive T cell therapy", "ACT", "engineered T-cell receptor (TCR) therapy", or "TCR T cell therapy" and the like refer to a process whereby autologous or allogeneic T-cells are transferred to a patient or subject to treat disease. The term "adoptive T cell therapy" refers to a cellular immunotherapy that relies on the use of the cells of a subject's or a donor's immune system to eliminate cancer cells. Adoptive T cell therapy involves the isolation and *ex vivo* expansion of tumor specific T cells to achieve greater number of T cells and the infusion back into patients with cancer in an attempt to give their immune system the ability to overwhelm remaining tumor via T cells which can attack and kill cancer cells. There are many forms of adoptive T cell therapy being used for cancer treatment; culturing tumor-infiltrating lymphocytes (TIL) from the tumor, isolating and expanding one particular T cell or clone, exogenous T cell receptor (TCR), and even using T cells that have been engineered to potently recognize and attack tumors. The adoptive T cell therapy may be an allogenic chimeric antigen receptor (CAR) T cell therapy, CAR natural killer cell (NK) therapy, involve allogenic T cells engineered with an additional TCR or T-cell engagers (TCE).

In an embodiment of the combination of the invention, the adoptive T cell therapy (ATC) is a therapy using a CAR T-cell, a T-cell engager (TCE), an engineered T cell, a tumor infiltrating lymphocyte (TIL), a CAR natural killer cell (NK) and/or an exogenous T cell receptor (TCR).

In a preferred embodiment of the combination of the invention, the adoptive T cell therapy (ATC) is a therapy using a CAR T-cell and/or a T-cell engager (TCE).

The term "CAR-T" or "CAR-T cell", as used herein, refers to a genetically engineered T cell that produces a chimeric antigen receptor (CAR) on the surface of the cell. The backbone of a CAR-T or CAR T cell is a T cell, which is often collected and separated from a subject, such as a patient, who is going to receive the CAR-T or CAR T cell. A CAR T cell is usually an engineered patient's immune cell used to treat the patient's cancer by binding to a target antigen on a target cell, e.g., a tumor cell.

The term "T-cell engager" or "TCE", as used herein, relates to a protein simultaneously binding a target antigen on a target cell, e.g., a tumor cell, and an antigen on a T cell to form an artificial immune synapse that is independent of the T cell receptor (TCR). Typically, the antigen of the T cell to which the TCE binds is the invariant CD3 epsilon subunit of the T-cell receptor complex (TCR).

The term "immune checkpoint inhibitor" or "ICI", as used herein, refers to a therapeutic agent that targets at least one immune checkpoint protein to alter the regulation of an immune response, e.g., down-modulating, inhibiting, up-modulating, or activating an immune response. The term "immune checkpoint blockade" may be used to refer to a therapy comprising an immune checkpoint inhibitor. Immune checkpoint proteins are known in the art and include, without limitation, programmed cell death ligand 1 (PD-L1), TIGIT, cytotoxic T-lymphocyte antigen 4 (CTLA-4), programmed cell death 1 (PD-1), programmed cell death ligand 2 (PD-L2), V-domain Ig suppressor of T cell activation (VISTA), B7-H2, B7-H3, B7-H4, B7-H6, 2B4, ICOS, HVEM, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, LAG-3, BTLA, IDO, 0X40, and A2aR. In some embodiments, an immune checkpoint protein may be expressed on the surface of an activated T cell. Therapeutic agents that can act as immune checkpoint inhibitors useful in the combination therapy of the present invention, include, but are not limited to, therapeutic agents that target one or more of PD-L1, TIGIT, PD-1, CTLA-4, PD-L2, VISTA, B7-H2, B7-H3, B7-H4, B7-H6, 2B4, ICOS, HVEM, CD160, gp49B, PIR-B, KIR family receptors, TIM-1, TIM-3, TIM-4, LAG- 3, BTLA, SIRPalpha (CD47), CD48, 2B4 (CD244), B7.1, B7.2, ILT-2, ILT-4, LAG-3, BTLA, IDO, 0X40, and A2aR. In some aspects, an immune checkpoint inhibitor enhances or suppresses the function of one or more targeted immune checkpoint protein

The term "agent which avoids and/or prevents bone degradation", as used herein, refers to any molecule capable of preventing, inhibiting, treating, reducing, or stopping bone degradation either by stimulating the osteoblast proliferation or inhibiting the osteoclast proliferation or fixing the bone structure.

In an embodiment, the agent which avoids and/or prevents bone degradation is selected from the group consisting of a RANKL inhibitor, a bisphosphonate, PTH and PTHLH inhibitor or a PRG analog, strontium ranelate, a DKK-1 inhibitor, a dual MET and VEGFR2 inhibitor, an estrogen receptor modulator, Radium-223 calcitonin, and a cathepsin K inhibitor. In a more preferred embodiment, the agent which avoids and/or prevents bone degradation is a RANKL inhibitor. In a yet more preferred embodiment, the RANKL inhibitor is denosumab.

"RANKL inhibitor", as used herein, refer to any compound which is capable of reducing the RANK activity. RANKL (Receptor activator of nuclear factor kappa-B ligand) is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL.

The term "antibody-drug conjugate" or "ADC", as used herein, refers to antibodies, antigen-binding domains, or antibody constructs, also including antibody derivatives, that bind to a corresponding epitope of an antigen and are conjugated to a drug such as a cytotoxic, cytostatic, and/or therapeutic agent. The terms "cytotoxic, cytostatic, and/or therapeutic agents" and their particulars have been described in detail in the context of the ADC of the invention and apply equally to this aspect of the invention and its embodiments. An antibody-drug conjugate (ADC) is a kind of biomedicine, in which a biologically active cytotoxin (drug) links with an antibody through a chemical linker. After the toxin and the antibody are conjugated, the ADC specifically recognizes and binds to the target antigen or receptor on the surface of the cell, e.g. a cancer cell, by using the targeting ability of monoclonal antibody, then enters the cell through endocytosis, and releases cytotoxin by using the protease in the cell to prevent the cells, e.g., cancer cells, from reproduction and kill cancer cells.

In some embodiments, the target of the antibody-drug conjugate is selected from the group consisting of BCMA, CD79B, c-Met, GPNMB, IL2RA, LY6E, CD1, CD1a, CD2, CD3, CD4, CD5, CD8, CD11A, CD14, CD15, CD16, CD171, CD18, CD19, CD20, CD21, CD22, CD23, CD25, CD29, CD30, CD32, CD32b, CD33, CD37, CD38, CD40, CD40L, CD44, CD45, CD46, CD52, CD54, CD55, CD59, CD64, CD67, CD70, CD74, CD79a, CD79b, CD80, CD83, CD95, CD126, CD133, CD138, CD147, CD154, CD166, CD276, CSPG4, HER1, HER2, HER3, MUC1, PTK7, STEAP1, VTCN1, AXL, BCMA, CA9, CASP, CASP3, CDH3, CDKs, CEACAM5, CLDN18, c-Met, Cripto-1, CTL4, DLL3, EF2, EFNA4, EGFR, EGFRvIII, ENPP3, EphA2, ETBR, FGFR2, FGFR3, FOLR1, FOLR1, Ganglioside, GCPII, HER2, HER3, HGFR, HLA-DR, IGF1R, IL3RA, IL13RA, ITGAV, ITGB3, KIT, LAMP1, Lewis-Y, LRRC15, LY75, LYPD3, MCP, MELTF, MSLN, MUC1, MUC16, NaPi-2b, NCAM1, NECTIN4, NOTCH3, Prolactin receptor, RNA polymerase II, ROR1, SDC1, SGLT2, SLAMF6, SLAMF7, SLITRK6, STAR, STING, TfR, TIM1, TLR8, TNF, TOP1, TPBG, Trop-2, VEGF, ZIP6, cytokines, tubulins and combinations thereof.

In a particular embodiment of the combination of the invention, the target of the CAR T-cell, of the T-cell engager (TCE) or of the antibody-drug conjugate (ADC) is any target commonly expressed in a solid tumor.

The term "target", as used herein, refers to a target antigen which the CAR T-cell, the T-cell engager (TCE) or the antibody-drug conjugate (ADC) specifically recognizes and binds to. In an embodiment, said target is commonly expressed in a solid tumor.

The term "solid tumor" and its particulars have been described in detail the context of the previous aspects of the invention and apply equally to this aspect of the invention and its embodiments.

Exemplary targets commonly expressed in a solid tumors include, but are not limited to, CD79B, c-Met, GPNMB, IL2RA, LY6E, CD1, CD1a, CD2, CD3, CD4, CD5, CD8, CD11A, CD14, CD15, CD16, CD171, CD18, CD19, CD20, CD21, CD22, CD23, CD25, CD29, CD30, CD32, CD32b, CD33, CD37, CD38, CD40, CD40L, CD44, CD45, CD46, CD52, CD54, CD55, CD59, CD64, CD67, CD70, CD74, CD79a, CD79b, CD80, CD83, CD95, CD126, CD133, CD138, CD147, CD154, CD166, CD276, CSPG4, HER1, HER2, HER3, MUC1, PTK7, STEAP1, VTCN1, AXL, CA9, CASP, CASP3, CDH3, CDKs, CEACAM5, CLDN18, c-Met, Cripto-1, CTL4, DLL3, EF2, EFNA4, EGFR, EGFRvIII, ENPP3, EphA2, ETBR, FGFR2, FGFR3, FOLR1, FOLR1, Ganglioside, GCPII, HER2, HER3, HGFR, HLA-DR, IGF1R, IL3RA, IL13RA, ITGAV, ITGB3, KIT, LAMP1, Lewis-Y, LRRC15, LY75, LYPD3, MCP, MELTF, MSLN, MUC1, MUC16, NaPi-2b, NCAM1, NECTIN4, NOTCH3, Prolactin receptor, RNA polymerase II, ROR1, SDC1, SGLT2, SLAMF6, SLAMF7, SLITRK6, STAR, STING, TfR, TIM1, TLR8, TNF, TOP1, TPBG, Trop-2, VEGF, ZIP6, cytokines, tubulins and combinations thereof.

In a particular embodiment of the combination of the invention, the target is the epidermal growth factor receptor variant III (EGFRvlll).

In a more particular embodiment of the combination of the invention, the target of the TCE or of the CAR-T is EGFRvIII.

In an embodiment of the combination of the invention, the solid tumor us selected from the group consisting of glioblastoma, bone cancer, liver cancer, brain cancer, lung cancer, breast cancer, ovarian cancer, uterine cancer, pancreatic cancer, prostate cancer, hepatobiliary cancer, kidney cancer, renal cancer, head and neck cancer, tongue cancer, colorectal cancer, adenocarcinoma, carcinoma, melanoma, epithelial cancer, oral cancer, salivary gland cancer, esophageal cancer, thyroid cancer, endometrial cancer, skin cancer, gall bladder cancer, sarcoma, bladder cancer, urethral cancer, gastric cancer, stomach cancer, testicular cancer, cervical cancer, a glial-derived tumor, a neural crest-derived tumor, adrenal cancer, carcinoid tumors, nervous system tumors, glioma, neuroblastoma, lymphoma and metastasis thereof.

The term "cancer", "metastasis" and their particulars have been described in detail the context of the previous aspects of the invention and apply equally to this aspect of the invention and its embodiments.

In an embodiment of the combination of the invention, the cancer is glioblastoma, bone metastasis, liver metastasis and/or brain metastasis.

The term "glioblastoma", "bone metastasis", "liver metastasis", "brain metastasis" and their particulars have been described in detail the context of the previous aspects of the invention and apply equally to this aspect of the invention and its embodiments.

In a preferred embodiment of the combination of the invention, the cancer is a primary tumor and/or a secondary or metastatic tumor.

In a more preferred embodiment of the combination of the invention, the secondary or metastatic tumor is bone metastasis, brain metastasis or liver metastasis. In a yet more preferred embodiment, the secondary or metastatic tumor is bone metastasis.

In a more particular embodiment of the combination of the invention, the TCE or the CAR-T targets EGFRvIII expressed in glioblastoma.

All the terms and embodiments described in any of the previous aspects of the invention are equally applicable to the present aspects of the invention and their embodiments.

### Other methods of the invention

The present invention provides methods for increasing the therapeutic efficacy of therapies in combination with a neutralizing antibody specifically binding to gp130.

Thus, a twelfth aspect of the present invention relates to a method, hereinafter referred to as "the second method of the invention", for increasing the therapeutic efficacy of at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC),
the method comprising the administration of a combination of said therapy and a neutralizing antibody, antigen-binding fragment thereof or antibody construct comprising an antigen-binding fragment thereof specifically binding to gp130, to a subject in need thereof.

The terms and expressions "antibody", "antigen-binding fragment", "antibody construct", "neutralizing", "specifically binding", "gp130", "subject", "adoptive T cell therapy (ACT)", "immune checkpoint inhibitor (ICI)", "agent which avoids and/or prevents bone degradation", "antibody-drug conjugate (ADC)" and their particulars have been described in detail the context of the previous aspects of the invention and apply equally to this aspect of the invention and its embodiments.

The term "increasing the therapeutic efficacy", as used herein, refers to the capacity of the antibody, antigen-binding fragment thereof or antibody construct to increase ability of a therapy (e.g., ACT, ICI, an agent which avoids and/or prevents bone degradation and/or ADC) to control a subject's disease state or to produce a beneficial result in such disease state.

The present invention also provides methods for reducing the immunosuppressive effect of tumor-associated macrophages (TAMs) in the tumor of a subject by the administration of a neutralizing antibody specifically binding to gp130.

Therefore, a thirteenth aspect of the present invention relates to a method for reducing the immunosuppressive effect of tumor-associated macrophages (TAMs) in the tumor of a subject suffering from said tumor, hereinafter referred to as "the third method of the invention", the method comprising the administration of a neutralizing antibody, antigen-binding fragment thereof or antibody construct comprising an antigen-binding fragment thereof specifically binding to gp130, to a subject in need thereof.

The terms and expressions "antibody", "antigen-binding fragment", "antibody construct", "neutralizing", "specifically binding", "gp130", "subject", "combination", "tumor associates macrophages (TAMs)", "tumor" and their particulars have been described in detail the context of the previous aspects of the invention and apply equally to this aspect of the invention and its embodiments.

The term "reducing the immunosuppressive effect", as used herein, refers to the capacity of the antibody, antigen-binding fragment thereof or antibody construct to reduce or reverse the immunosuppressive features of the TAMs within the tumor microenvironment (TME) enabling the tumor immune evasion. This reduction of the immunosuppressive effect of the TAMs in the tumor generates an anti-tumor response.

In an embodiment of the third method of the invention, the subject further receives at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC).

The terms and expressions "adoptive T cell therapy (ACT)", "immune checkpoint inhibitor (ICI)", "agent which avoids and/or prevents bone degradation", "antibody-drug conjugate (ADC)" and their particulars have been described in detail the context of the previous aspects of the invention and apply equally to this aspect of the invention and its embodiments.

In another embodiment of the second and of the third methods of the invention, the neutralizing antibody is the antibody according to the first aspect of the invention.

In another embodiment of the second and of the third methods of the invention, the adoptive T cell therapy (ATC) is a therapy using a CAR T-cell, a T-cell engager (TCE), an engineered T cell, a tumor infiltrating lymphocyte (TIL), a CAR natural killer cell (NK) and/or an exogenous T cell receptor (TCR).

In another embodiment of the second and of the third methods of the invention, the adoptive T cell therapy (ATC) is a therapy using a CAR T-cell and/or a T-cell engager (TCE).

In yet another embodiment of the second and of the third methods of the invention, the target of the CAR T-cell, of the T-cell engager (TCE) or of the antibody-drug conjugate (ADC) is any target commonly expressed in a solid tumor.

The terms and expressions "CAR-T", "T-cell engager", "target", "solid tumor" and their particulars have been described in detail the context of the previous aspects of the invention and apply equally to this aspect of the invention and its embodiments.

In some embodiments, the target of the antibody-drug conjugate is selected from the group consisting of BCMA, CD79B, c-Met, GPNMB, IL2RA, LY6E, CD1, CD1a, CD2, CD3, CD4, CD5, CD8, CD11A, CD14, CD15, CD16, CD171, CD18, CD19, CD20, CD21, CD22, CD23, CD25, CD29, CD30, CD32, CD32b, CD33, CD37, CD38, CD40, CD40L, CD44, CD45, CD46, CD52, CD54, CD55, CD59, CD64, CD67, CD70, CD74, CD79a, CD79b, CD80, CD83, CD95, CD126, CD133, CD138, CD147, CD154, CD166, CD276, CSPG4, HER1, HER2, HER3, MUC1, PTK7, STEAP1, VTCN1, AXL, BCMA, CA9, CASP, CASP3, CDH3, CDKs, CEACAM5, CLDN18, c-Met, Cripto-1, CTL4, DLL3, EF2, EFNA4, EGFR, EGFRvIII, ENPP3, EphA2, ETBR, FGFR2, FGFR3, FOLR1, FOLR1, Ganglioside, GCPII, HER2, HER3, HGFR, HLA-DR, IGF1R, IL3RA, IL13RA, ITGAV, ITGB3, KIT, LAMP1, Lewis-Y, LRRC15, LY75, LYPD3, MCP, MELTF, MSLN, MUC1, MUC16, NaPi-2b, NCAM1, NECTIN4, NOTCH3, Prolactin receptor, RNA polymerase II, ROR1, SDC1, SGLT2, SLAMF6, SLAMF7, SLITRK6, STAR, STING, TfR, TIM1, TLR8, TNF, TOP1, TPBG, Trop-2, VEGF, ZIP6, cytokines, tubulins and combinations thereof.

In a particular embodiment of the second and of the third methods of the invention, the target is the epidermal growth factor receptor variant III (EGFRvlll).

In a more particular embodiment of the second and of the third methods of the invention, the target of the TCE or of the CAR-T is EGFRvlll.ln an embodiment of the second and of the third methods of the invention, the agent which avoids and/or prevents bone degradation is a RANKL inhibitor. In a yet more preferred embodiment, the RANKL inhibitor is denosumab.

The term "RANKL inhibitor" and its particulars have been described in detail the context of the previous aspect of the invention and apply equally to this aspect of the invention and its embodiments.

In an embodiment of the second and of the third methods of the invention, the solid tumor us selected from the group consisting of glioblastoma, bone cancer, liver cancer, brain cancer, lung cancer, breast cancer, ovarian cancer, uterine cancer, pancreatic cancer, prostate cancer, hepatobiliary cancer, kidney cancer, renal cancer, head and neck cancer, tongue cancer, colorectal cancer, adenocarcinoma, carcinoma, melanoma, epithelial cancer, oral cancer, salivary gland cancer, esophageal cancer, thyroid cancer, endometrial cancer, skin cancer, gall bladder cancer, sarcoma, bladder cancer, urethral cancer, gastric cancer, stomach cancer, testicular cancer, cervical cancer, a glial-derived tumor, a neural crest-derived tumor, adrenal cancer, carcinoid tumors, nervous system tumors, glioma, neuroblastoma, lymphoma and metastasis thereof.

The term "cancer", "metastasis" and their particulars have been described in detail the context of the previous aspects of the invention and apply equally to this aspect of the invention and its embodiments.

In an embodiment of the second and of the third methods of the invention, the cancer is glioblastoma, bone metastasis, liver metastasis and/or brain metastasis.

The term "glioblastoma", "bone metastasis", "liver metastasis", "brain metastasis" and their particulars have been described in detail the context of the previous aspects of the invention and apply equally to these aspects of the invention and their embodiments.

In a preferred embodiment of the second and of the third methods of the invention, the cancer is a primary tumor and/or a secondary or metastatic tumor.

In a more preferred embodiment of the second and of the third methods of the invention, the secondary or metastatic tumor is bone metastasis, brain metastasis or liver metastasis. In a yet more preferred embodiment, the secondary or metastatic tumor is bone metastasis.

In a more particular embodiment of the combination of the invention, the TCE or the CAR-T targets EGFRvIII expressed in glioblastoma.

In another particular embodiment of the second and of the third methods of the invention, the antibody of the selected therapy or the antibody-drug conjugate targets RANKL expressed in bone metastasis.

All the terms and embodiments described in any of the previous aspects of the invention are equally applicable to the present aspects of the invention and their embodiments.

The invention is defined below by means of the following aspects:
1. A neutralizing antibody, an antigen-binding fragment thereof or an antibody construct comprising an antigen-binding fragment thereof capable of specifically binding to and neutralizing gp130, wherein said antibody, antigen-binding fragment thereof or antibody construct comprises:
   (a) within the heavy chain of the variable region, a CDR comprising the amino acid sequence set forth in SEQ ID NO: 1 [CDR-H1], a CDR comprising the amino acid sequence set forth in SEQ ID NO: 2 [CDR-H2], and a CDR comprising the amino acid sequence set forth in SEQ ID NO: 3 [CDR-H3], or a functionally equivalent variant of said CDRs; and
   (b) within the light chain of the variable region, a CDR comprising the amino acid sequence set forth in SEQ ID NO: 4 [CDR-L1], a CDR comprising the amino acid sequence set forth in SEQ ID NO: 5 [CDR-L2] (SAN), and a CDR comprising the amino acid sequence set forth in SEQ ID NO: 6 [CDR-L3], or a functionally equivalent variant of said CDRs.
2. The antibody, antigen-binding fragment thereof or antibody construct according to aspect 1, wherein said antibody, antigen-binding fragment thereof or antibody construct comprises:
   (a) within the heavy chain of the variable region, a FR region comprising the amino acid sequence set forth in SEQ ID NO: 7 [FR-H1], a FR region comprising the amino acid sequence set forth in SEQ ID NO: 8 [FR-H2], a FR region comprising the amino acid sequence set forth in SEQ ID NO: 9 [FR-H3], and a FR region comprising the amino acid sequence set forth in SEQ ID NO: 10 [FR-H4], or a functionally equivalent variant of said FR regions; and
   (b) within the light chain of the variable region, a FR region comprising the amino acid sequence set forth in SEQ ID NO: 11 [FR-L1], a FR region comprising the amino acid sequence set forth in SEQ ID NO: 12 [FR-L2], a FR region comprising the amino acid sequence set forth in SEQ ID NO: 13 [FR-L3], and a FR region comprising the amino acid sequence set forth in SEQ ID NO: 14 [FR-L4], or a functionally equivalent variant of said FR regions.
3. The antibody, antigen-binding fragment thereof or antibody construct according to any one of aspects 1 or 2, wherein said antibody, antigen-binding thereof or antibody construct comprises:
   (a) at least a heavy chain of the variable region comprising the sequence set forth in SEQ ID NO: 15 [VH], or a functionally equivalent variant thereof; and
   (b) at least a light chain of the variable region comprising the sequence set forth in SEQ ID NO: 16 [VL], or a functionally equivalent variant thereof.
4. The antibody, antigen-binding fragment thereof or antibody construct according to any one of aspects 1 to 3, wherein said antibody, antigen-binding fragment thereof or antibody construct is an animal antibody, a chimeric antibody, a human antibody, or a humanized antibody.
5. The antibody, antigen-binding fragment thereof or antibody construct according to any one of aspects 1 to 4, wherein said antigen-binding fragment is selected from the group consisting of Fv, Fab, F(ab')₂ and Fab'; and wherein said antibody construct is selected from the group consisting of scFv, (scFv)₂, scFv-Fc, minibody, nanobody, diabody and bispecific antibody.
6. A nucleic acid encoding the antibody, antigen-binding fragment thereof or antibody construct according to any one of aspects 1 to 5.
7. A gene construct, an expression cassette or a vector comprising the nucleic acid according to aspect 6.
8. A cell comprising the nucleic acid according to aspect 6, or the gene construct, the expression cassette, or the vector according to aspect 7.
9. A conjugate selected from the group consisting of:
   (i) an antibody-drug conjugate (ADC) comprising a therapeutic agent and the antibody, antigen-binding fragment thereof or antibody construct according to any one of aspects 1 to 5; and
   (ii) a detectable label and the antibody, antigen-binding fragment thereof or antibody construct according to any one of aspects 1 to 5.
10. An *in vitro* method for detecting and/or quantifying the presence of gp130 in a sample, comprising:
   (a) contacting the antibody, antigen-binding fragment thereof or antibody construct according to any one of aspects 1 to 5, or the conjugate according to aspect 9(ii) with the sample; and
   (b) detecting and/or quantifying the level of gp130 in the sample by measuring the formation of immune complexes formed between the gp130 in the sample and the antibody, antigen-binding fragment thereof, antibody construct, or the conjugate.
11. A pharmaceutical composition comprising the antibody, antigen-binding fragment thereof or antibody construct according to any one of aspects 1 to 5, or the ADC according to aspect 9(i), and a pharmaceutically acceptable excipient and/or carrier.
12. The antibody, antigen-binding fragment thereof or antibody construct according to any one of aspects 1 to 5, the ADC according to aspect 9(i), or the pharmaceutical composition according to aspect 11, for use in medicine.
13. The antibody, antigen-binding fragment thereof or antibody construct according to any one of aspects 1 to 5, the ADC according to aspect 9(i), or the pharmaceutical composition according to aspect 11, for use in the treatment and/or prevention of cancer and/or an inflammatory disease.
14. The antibody, antigen-binding fragment thereof or antibody construct, the ADC, or the pharmaceutical composition for use according to aspect 13 , wherein:
   - the cancer is characterized by the presence of tumor-associated macrophages (TAMs) with increased gp130 signaling; and/or
   - the inflammatory disease is characterized by increased gp130 signaling.
15. The antibody, antigen-binding fragment thereof or antibody construct, the ADC, or the pharmaceutical composition for use according to any one of aspect 13 or 14, wherein:
   - the cancer is a solid tumor; and/or
   - the inflammatory disease is selected from the group consisting of arthritis, rheumatoid arthritis, fibrosis, inflammatory bowel disease, Crohn's disease, ulcerative colitis, asthma, pulmonary hypertension, chronic obstructive pulmonary disease, bronchopulmonary displasia, encephalomyelitis, diabetes, lupus, psoriasis, rosacea, vasculitis, hepatitis, systemic sclerosis, gout, myositis and scleroderma.
16. The antibody, antigen-binding fragment thereof or antibody construct, the ADC, or the pharmaceutical composition for use according to any one of aspects 13 to 15, wherein the cancer is selected from the group consisting of glioblastoma, bone cancer, liver cancer, brain cancer, lung cancer, breast cancer, ovarian cancer, uterine cancer, pancreatic cancer, prostate cancer, hepatobiliary cancer, kidney cancer, renal cancer, head and neck cancer, tongue cancer, colorectal cancer, adenocarcinoma, carcinoma, melanoma, epithelial cancer, oral cancer, salivary gland cancer, esophageal cancer, thyroid cancer, endometrial cancer, skin cancer, gall bladder cancer, sarcoma, bladder cancer, urethral cancer, gastric cancer, stomach cancer, testicular cancer, cervical cancer, a glial-derived tumor, a neural crest-derived tumor, adrenal cancer, carcinoid tumors, nervous system tumors, glioma and neuroblastoma, lymphoma and metastasis thereof.
17. The antibody, antigen-binding fragment thereof or antibody construct, the ADC, or the pharmaceutical composition for use according to any one of aspects 13 to 16, wherein:
   - the cancer is glioblastoma, bone metastasis, liver metastasis and/or brain metastasis; and/or
   - the inflammatory disease is rheumatoid arthritis and/or fibrosis.
18. Use of the antibody, antigen-binding fragment thereof or antibody construct according to any one of aspects 1 to 5, the ADC according to aspect 9(i), the conjugate according to aspect 9(ii), or the pharmaceutical composition according to aspect 11:
   - for targeting a therapeutic agent to a specific site wherein said specific site is characterized by the presence of tumor-associated macrophages (TAMs) with increased gp130 signaling or said specific site is characterized by increased gp130 signaling; and/or
   - for detecting and/or quantifying the presence of gp130 in a sample.
19. A combination therapy comprising a neutralizing antibody, antigen-binding fragment thereof or antibody construct comprising an antigen-binding fragment thereof specifically binding to gp130 and at least a therapy selected from the group consisting of:
   (i) an adoptive T cell therapy (ACT),
   (ii) an immune checkpoint inhibitor (ICI),
   (iii) an agent which avoids and/or prevents bone degradation; and
   (iv) an antibody-drug conjugate (ADC).
20. The combination according to aspect 19, wherein the antibody, antigen-binding fragment thereof or antibody construct is as defined in any one of aspects 1 to 5.
21. The combination according to any one of aspects 19 or 20, wherein the adoptive T cell therapy (ACT) is a therapy using a CAR T-cell, a T-cell engager (TCE), an engineered T cell, a tumor infiltrating lymphocyte (TIL), a CAR natural killer cell (NK) and/or an exogenous T cell receptor (TCR).
22. The combination according to aspect 21, wherein the adoptive T cell therapy is a therapy using a CAR T-cell and/or a T-cell engager (TCE).
23. The combination according to any one of aspects 19 to 22, wherein the target of the CAR T-cell, of the T-cell engager (TCE) or of the antibody-drug conjugate (ADC) is any target commonly expressed in a solid tumor.
24. The combination according to aspect 23, wherein the solid tumor is selected from the group consisting of glioblastoma, bone cancer, liver cancer, brain cancer, lung cancer, breast cancer, ovarian cancer, uterine cancer, pancreatic cancer, prostate cancer, hepatobiliary cancer, kidney cancer, renal cancer, head and neck cancer, tongue cancer, colorectal cancer, adenocarcinoma, carcinoma, melanoma, epithelial cancer, oral cancer, salivary gland cancer, esophageal cancer, thyroid cancer, endometrial cancer, skin cancer, gall bladder cancer, sarcoma, bladder cancer, urethral cancer, gastric cancer, stomach cancer, testicular cancer, cervical cancer, a glial-derived tumor, a neural crest-derived tumor, adrenal cancer, carcinoid tumors, nervous system tumors, glioma, neuroblastoma, lymphoma and metastasis thereof.
25. A method for increasing the therapeutic efficacy of at least a therapy selected from the group consisting of:
   (i) an adoptive T cell therapy (ACT),
   (ii) an immune checkpoint inhibitor (ICI),
   (iii) an agent which avoids and/or prevents bone degradation; and
   (iv) an antibody-drug conjugate (ADC),
   the method comprising the administration of a combination of said therapy and a neutralizing antibody, antigen-binding fragment thereof or antibody construct comprising an antigen-binding fragment thereof specifically binding to gp130, to a subject in need thereof.
26. A method for reducing the immunosuppressive effect of tumor-associated macrophages (TAMs) in the tumor of a subject suffering from said tumor, the method comprising the administration of a neutralizing antibody, antigen-binding fragment thereof or antibody construct comprising an antigen-binding fragment thereof specifically binding to gp130 to a subject in need thereof.
27. The method according to aspect 26, wherein the subject further receives at least a therapy selected from the group consisting of:
   (i) an adoptive T cell therapy (ACT),
   (ii) an immune checkpoint inhibitor (ICI),
   (iii) an agent which avoids and/or prevents bone degradation; and
   (iv) an antibody-drug conjugate (ADC).
28. The method according to any of aspects 25 to 27 wherein the antibody, antigen-binding fragment thereof or antibody construct is as defined in any one of aspects 1 to 5.
29. The method according to any one of aspects 25 to 28, wherein the adoptive T cell therapy (ACT) is a therapy using a CAR T-cell, a T-cell engager (TCE), an engineered T cell, a tumor infiltrating lymphocyte (TIL), a CAR natural killer cell (NK) and/or an exogenous T cell receptor (TCR).
30. The method according to aspect 29, wherein the adoptive T cell therapy is a therapy using a CAR T-cell and/or a T-cell engager (TCE).
31. The method according to any one of aspects 26 to 30, wherein the target of the CAR T-cell, of the T-cell engager (TCE) or of the antibody-drug conjugate (ADC) is any target commonly expressed in a solid tumor.

The invention will be described by way of the following examples which are to be considered as merely illustrative and not limitative of the scope of the invention.

### EXAMPLES

We have generated 24 hybridomas producing antibodies targeting mouse GP130 and have screened them based on their ability to inhibit the GP130 downstream signal, namely inhibit the phosphorylation of STAT3 at Y705 (Figure 1A).

Selected antibodies were tested to assess their capacity to inhibit two GP130 cytokines, LIF and OSM in two cell lines (RM1 and GL261N) (Figure 1B).

The anti-GP130 antibody clone 4B3 inhibited the induction of p-STAT3 by several GP130 cytokines (LIF, OSM, IL11, IL6, CLCF1, CNTF, CT1) but not IL27 in the colorectal cancer cell line MC-38 and bone marrow-derived macrophages (BMDM) (Figure 2). This is relevant since all GP130 family members are oncogenic except for IL27 that has been described to potentiate the anti-tumor immune response (Breart et al., Nature 2025, doi: 10.1038/s41586-024-08510-w; Salcedo et al., J Immunol 2004, 173(12):7170-82; Hisada et al., Cancer Research 2004, 64(3):1152-6; Chiyo et al., Int. J. Cancer 2005, 115(3):437-42; Rolvering et al., Biochim Biophys Acta Mol Cell Res 2017, 1864(3):516-526; Zhu et al., JCI Insight 2018, 3(7):e98745).

We generated 17 hybridomas producing antibodies targeting human GP130 and screened them based on their ability to inhibit the GP130 downstream signal, namely inhibit the phosphorylation of STAT3 at Y705 (Figure 3A, B).

We generated a humanized version of the anti-human GP130 antibody clone 2G3, the antibody 2G3.V03. The antibody V03 inhibited the induction of p-STAT3 by several GP130 cytokines (LIF, OSM, IL6, IL11, CT-1) but not IL27 in the human cell lines 293T and A549 (Figure 4).

The anti-GP130 antibody transformed immunosuppressive macrophages into immune activator macrophages. The conditioned medium from lung cancer cells induced an immunosuppressive phenotype in bone marrow-derived macrophages (BMDM). The anti-GP130 antibody inhibited the immunosuppressive phenotype and restored the IFNγ response, a sign of immune activity (Figure 5).

The anti-GP130 antibody inhibited tumor growth in glioblastoma (Figure 6A), lung cancer brain metastasis (Figure 6B), pancreatic (PDCA) cancer liver metastasis (Figure 6C), breast cancer bone metastasis (Figure 6D), prostate cancer bone metastasis (Figure 6E), and cooperated with the anti-RANKL treatment in bone metastasis (Figure 6F).

The 2G3 anti-GP130 potentiated the cytotoxic activity (measured by the secretion of GZMB) of T cell engagers (TCE) in patient-derived models. Patient-derived tumor tissue cultures (PDTTCs) were generated by sectioning tumor fragments into 350□m thick slides. These slides were co-cultured with autologous leucocytes and the EGFRvIII T cell engager (TCE) antibodies or EGFRvIII CAR-T cells (Figure 7A). The blockade of GP130 with our humanized anti-GP130 antibodies (V03) in PDTTCs promoted an increase of GZMB and perforin secretion in cultures treated with EGFRvIII TCE cells when compared to cultures in the absence of V03 (Figure 7B). The blockade of GP130 with our humanized anti-GP130 antibodies (V03) in PDTTCs promoted an increase in GZMB secretion in cultures treated with EGFRvIII CAR-T cells when compared to cultures in the absence of V03 (Figure 7C).

## Claims

1. A neutralizing antibody, an antigen-binding fragment thereof or an antibody construct comprising an antigen-binding fragment thereof capable of specifically binding to and neutralizing gp130, wherein said antibody, antigen-binding fragment thereof or antibody construct comprises:
(a) within the heavy chain of the variable region, a CDR comprising the amino acid sequence set forth in SEQ ID NO: 1 [CDR-H1], a CDR comprising the amino acid sequence set forth in SEQ ID NO: 2 [CDR-H2], and a CDR comprising the amino acid sequence set forth in SEQ ID NO: 3 [CDR-H3], or a functionally equivalent variant of said CDRs; and
(b) within the light chain of the variable region, a CDR comprising the amino acid sequence set forth in SEQ ID NO: 4 [CDR-L1], a CDR comprising the amino acid sequence set forth in SEQ ID NO: 5 [CDR-L2] (SAN), and a CDR comprising the amino acid sequence set forth in SEQ ID NO: 6 [CDR-L3], or a functionally equivalent variant of said CDRs.

2. A nucleic acid encoding the antibody, antigen-binding fragment thereof or antibody construct according to claim 1.

3. A cell comprising the nucleic acid according to claim 2.

4. A conjugate selected from the group consisting of:
(i) an antibody-drug conjugate (ADC) comprising a therapeutic agent and the antibody, antigen-binding fragment thereof or antibody construct according to claim 1; and
(ii) a detectable label and the antibody, antigen-binding fragment thereof or antibody construct according to claim 1.

5. An *in vitro* method for detecting and/or quantifying the presence of gp130 in a sample, comprising:
(a) contacting the antibody, antigen-binding fragment thereof or antibody construct according to claim 1, or the conjugate according to claim 4(ii) with the sample; and
(b) detecting and/or quantifying the level of gp130 in the sample by measuring the formation of immune complexes formed between the gp130 in the sample and the antibody, antigen-binding fragment thereof, antibody construct, or the conjugate.

6. A pharmaceutical composition comprising the antibody, antigen-binding fragment thereof or antibody construct according to claim 1, or the ADC according to claim 4(i), and a pharmaceutically acceptable excipient and/or carrier.

7. The antibody, antigen-binding fragment thereof or antibody construct according to claim 1, the ADC according to claim 4(i), or the pharmaceutical composition according to claim 6, for use in medicine.

8. The antibody, antigen-binding fragment thereof or antibody construct according to claim 1, the ADC according to claim 4(i), or the pharmaceutical composition according to claim 6, for use in the treatment and/or prevention of cancer and/or an inflammatory disease.

9. The antibody, antigen-binding fragment thereof or antibody construct, the ADC, or the pharmaceutical composition for use according to claim 8 , wherein:
- the cancer is **characterized by** the presence of tumor-associated macrophages (TAMs) with increased gp130 signaling; and/or
- the inflammatory disease is **characterized by** increased gp130 signaling.

10. The antibody, antigen-binding fragment thereof or antibody construct, the ADC, or the pharmaceutical composition for use according to any one of claims 8 or 9, wherein:
- the cancer is a solid tumor; and/or
- the inflammatory disease is selected from the group consisting of arthritis, rheumatoid arthritis, fibrosis, inflammatory bowel disease, Crohn's disease, ulcerative colitis, asthma, pulmonary hypertension, chronic obstructive pulmonary disease, bronchopulmonary displasia, encephalomyelitis, diabetes, lupus, psoriasis, rosacea, vasculitis, hepatitis, systemic sclerosis, gout, myositis and scleroderma.

11. Use of the antibody, antigen-binding fragment thereof or antibody construct according to claim 1, the ADC according to claim 4(i), the conjugate according to claim 4(ii), or the pharmaceutical composition according to claim 6:
- for targeting a therapeutic agent to a specific site wherein said specific site is **characterized by** the presence of tumor-associated macrophages (TAMs) with increased gp130 signaling or said specific site is **characterized by** increased gp130 signaling; and/or
- for detecting and/or quantifying the presence of gp130 in a sample.

12. A combination therapy comprising a neutralizing antibody, antigen-binding fragment thereof or antibody construct comprising an antigen-binding fragment thereof specifically binding to gp130 and at least a therapy selected from the group consisting of:
(i) an adoptive T cell therapy (ACT),
(ii) an immune checkpoint inhibitor (ICI),
(iii) an agent which avoids and/or prevents bone degradation; and
(iv) an antibody-drug conjugate (ADC).

13. The combination according to claim 12, wherein the antibody, antigen-binding fragment thereof or antibody construct is as defined in claim 1.

14. The combination according to any one of claims 12 or 13, wherein the adoptive T cell therapy (ACT) is a therapy using a CAR T-cell, a T-cell engager (TCE), an engineered T cell, a tumor infiltrating lymphocyte (TIL), a CAR natural killer cell (NK) and/or an exogenous T cell receptor (TCR).

15. The combination according to any one of claims 12 to 14, wherein the target of the CAR T-cell, the T-cell engager (TCE) or the antibody-drug conjugate (ADC) is any target commonly expressed in a solid tumor.
